# EUROPEAN PATENT APPLICATION

(11) **EP 1 767 634 A1**
(43) Date of publication of application: **28.03.2007**
(21) Application number: 05741267.8
(22) Date of filing: 13.05.2005
(51) Int. Cl.: C12N 15/12, A61K 38/17, A61P 7/04, A61P 29/00, A61P 35/00, A61P 37/02, A61P 43/00, C07K 14/47, C12N 1/15, C12N 1/19, C12N 1/21, C12N 5/10

(54) **NOVEL GALECTIN 8 MODIFICATION PROTEIN AND USE THEREOF**

(30) Priority: 14.06.2004 JP 2004175086
(71) Applicant: Galpharma Co., Ltd., Takamatsu-shi, Kagawa 761-0301 (JP)
(72) Inventor: NISHI, Nozomu, Kita-gun, Kagawa 761-0703 (JP); HIRASHIMA, Mitsuomi, Takamatsu-shi, Kagawa 761-8071 (JP); YAMAUCHI, Akira, Takamatsu-shi, Kagawa 761-0113 (JP); ITO, Aiko, Kita-gun, Kagawa 761-0122 (JP)
(74) Representative: von Kreisler Selting Werner
(86) International application number: PCT/JP2005/009211
(87) International publication number: WO 2005/121340

(57) **Abstract**

Recombinant galectin 8 (rGal 8), produced in host Escherichia coli, exerts hemagglutinating activity, neutrophil adhesion inducing activity, integrin α_{M}-binding activity, proMMP-9 binding activity, active form MMP-9 production promoting activity, superoxide production promoting activity, apoptosis inducing activity for a particular cell, suppressive or inhibitory activity against the metastasis/invasion of tumor cells, etc. In the rGal 8, however, a link domain linking two CRDs is highly susceptible to protease and, therefore, is very easily digestible with the enzyme, thereby losing the above activities. Thus, there is a need for a more stabilized molecule in view of further studies. Modification of the link domain linking two CRDs in galectin 8 provides a modified molecule having an elevated activity without any undesirable effects on the above activities.

## Description

### FIELD OF THE INVENTION

The present invention relates to novel modified galectin 8 proteins (galectin-8 muteins) and applications thereof. Particularly, the present invention relates to functional mutant galectin 8 proteins wherein each of said functional mutant galectin 8 proteins has a modified link peptide region, and their practical applications in biochemistry, medical diagnostics, therapy and pharmacology.

### BACKGROUND OF THE INVENTION

Evidence indicating the following fact has been found: specific saccharide chains and proteins that bind to the same play a lot of various roles and functions in physiological phenomena, events associated with development/growth, and a variety of diseases in mammal's living bodies. It has been found that there are animal lectins, in living bodies, which specifically recognize saccharide chains with β-galactoside structure. Until now at least 14 types of genes have been identified for galectins which belong to the group of such lectins. Although the galectin family is classified, based on their structure, into three subgroups, i.e., prototype, chimera, and tandem repeat groups, the in vivo functions have scarcely been disclosed. Particularly, a study on tandem repeat type galectins retaining two carbohydrate recognition domains has only a short history. Since in vivo saccharide chains (receptors) to be targeted are never yet revealed, the functions are not yet clarified. From details including how galectins were discovered while a search was made for proteins which recognize complicated sugar chains on the surface of cells, it is forecasted that they must have functions such as involvement in cell adhesion, cell-to-cell communication, cell activation, etc. Therefore, galectins attract attention. In addition, research results anticipating the following are being obtained: the galectins retain, besides such functions, a variety of other important functions.

Galectin 8 (Gal-8), one of tandem repeat type galectins, was discovered during the screening of ZAP expression libraries with antibodies against substrates for the insulin receptor (IR), cloned and isolated (Non-Patent Document 1). Although Gal-8 was first cloned from the rat liver cDNA library, human galectin-8 coding cDNA was isolated from human hippocampus cDNA libraries with rat full-length cDNA as a probe (Non-Patent Document 2: GenBank/EMBL accession no. X91790). Subsequent investigations have revealed that Gal-8 ranges more widely among mammal tissue types (for example, liver, heart, muscle, kidney, brain and others) than other galectins such as Galectin-4, -6, -9 and -12. Gal-8 exerts potent hemagglutinating activity and binds to a lactosyl-agarose chain. The N-terminal CRD of Gal-8 is approximately 40% identical with the C-terminal CRD of Gal-8 at an amino acid level. For the biological functions, it has been disclosed that Gal-8 inhibits the adhesion of human carcinoma cells to an integrin-coated plate, and induces apoptosis of said carcinoma cells (Non-Patent Document 3). It has also been revealed that Gal-8 affects migration of colon carcinoma (Non-Patent Document 4). It has been reported that Gal-8 mRNA is intensely expressed in lung, and expressed in a variety of tissue types, such as liver, kidney, spleen, hind legs, and cardiac muscle though the expression levels are decreased. In addition, there is a report that it is highly homologous to human prostate carcinoma tumor antigen, PCTA-1, at the protein level.

Inflammation is a response driving extravasation of white blood cells, or leukocytes, and serum molecule groups into foci of infection and tissue sites of injury. Inflammatory responses include three key elements:
(1) increased blood flow at inflammatory foci,
(2) increased capillary permeability due to endothelial cell constriction (whereby abnormal macromolecules extravasate from blood vessels and soluble immunoreactive molecules migrate into inflamed local sites), and
(3) leukocyte extravasation from the blood vessel and leukocyte transmigration into the surrounding tissue (polymorphonuclear leukocytes [predominantly neutrophils] abound most at the early stage of inflammation, and mononuclear cells and lymphocytes migrate to the inflamed local site in the latter half stage). The transmigration of neutrophils into the inflamed site progresses through several independent steps. Selectins are involved in the first step wherein the neutrophil adheres weakly to the venular endothelium and then rolls across the surface of the venular endothelium. In the second step, the actions of chemoattractants and integrins lead to the formation of firm adhesion between the neutrophil and the endothelial cell, thereby bringing the neutrophil to a halt on the endothelial cell. The neutrophil will then travel through the interstitium of the endothelial cell lining into the tissue. Chemoattractants and integrins are also involved in this process.

The firm adhesion between the neutrophil and the endothelial cell is triggered by interactions between integrins, such as integrin *α*_{L}*β*₂ (lymphocyte function-associated antigen-1; LFA-1), integrin *α*_{M}*β*₂ (macrophage receptor 1; Mac-1), and integrin *α*ₓ*β*₂ (p150/95) (principally LFA-1 and Mac-1), and molecules (ligand/receptor molecules) existing on the surface of the endothelial cell. It is known that LFA-1 interacts with ICAM-1 and ICAM-2 while Mac-1 does with ICAM-1. Integrins generally exist in an inactive conformation with no affinity or low affinity for ligands, and the transformation to a high affinity active form takes place with intracellular and extracellular stimuli (reversible activation due to stereo structural alterations). Although a monoclonal antibody capable of inducing said activation has been known among those against the extracellular domain of the integrin, an in vivo activation mechanism is still unknown.

Neutrophils primarily play important roles in body defense. Neutrophils phagocytically destroy invading microorganisms, repair inflamed sites and release potently disinfectant acids, oxygen, active oxygen species, and others. However, these cytotoxic factors (injurious factors) lack target specificity, unlike antibodies, and have some possibility of damaging even normal tissue sites in the vicinity of affected sites. Accordingly, the living body has a mechanism for localizing such reactions. When said control mechanism is disturbed, cytotoxic factors produced by neutrophils will cause the damage of the tissue. Known diseases in which neutrophils are involved include various chronic inflammatory diseases (Behçet's disease, Crohn's disease, etc.), ischemic-reperfusion injury, neutrophilic dermatosis (Sweet's syndrome, etc.), systemic inflammatory response syndrome (SIRS) and others.

When it is considered that the first step where such neutrophils transmigrate to the inflamed site is the interaction with the endothelial cell, modulating or blocking this stage would promise the inhibition of neutrophil hyperfunction, etc. Particularly, in case where the occurrence of injury caused by neutrophils upon the therapy of cardiac infarction and others can be anticipated, the development of prophylactically utilizable drugs or agents will be expectable. There is some possibility that abnormality in steps where the neutrophil interacts with the endothelial cell (+ cytotoxic factor release) may be a cause of neutrophil hyperfunction. In addition, if it is possible to specify such a cause of neutrophil hyperfunction, it may be contributed to accurate diagnosis and effective therapy. It is important to reveal a mechanism for regulating the acquired ability of the neutrophil to adhere to the endothelial cell in view of comprehending not only inflammation but also a variety of physiological phenomena and controlling pathological conditions associated with inflammation and others. Since controlling the adhesive property of the neutrophil is useful in the development of pharmaceuticals, diagnostics, screening assays, diagnostic methods and others, there is a great demand for elucidating such.

Recently, the present inventor group has succeeded in finding that human galectin-8 (hGal-8) has potent neutrophil adhesion inducing (neutrophil chemoattractant) activity, and the C-terminal CRD of Gal-8 binds to integrin α_{M} while the N-terminal CRD of Gal-8 does to proMMP-9, that Gal-8 further activates proMMP-9, and promotes the production of active form MMP-9, and that the C-terminal CRD of Gal-8 has the ability to promote superoxide production, for example, in neutrophils, said ability being inhibitible with sugar analogs such as lactose. As a result, the present inventor group has succeeded in providing techniques for various reagents, methods and applications for the purpose of studying, analyzing, assaying, diagnosing, preventing and treating responses, symptoms, disorders and diseases related to, for example, interactions between galectin-8 and neutrophils (including interactions between galectin-8 and integrin α_{M}, interactions between galectin-8 and proMMP-9, and proMMP-9 activation) (Patent Document 1).
**[**Patent Document 1**]** JP, A, 2003-246749
**[**Non-Patent Document 1**]** Hadari et al., J. Biol. Chem., 270: 3447-3453 (1995)
[Non-Patent Document 2] Hadari et al., Trends in Glycoscience and Glycotechnology, Vol.9, No.45, pp. 103 to 112 (1997)
**[**Non-Patent Document 3] Hadari et al., J. Cell Sci., 113: 2385 to 2397 (2000)
[Non-Patent Document 4] N. Nagym et al., Gut, 50: 392 to 401 (2002)

### SUMMARY OF THE INVENTION

Utilization of such versatile properties possessed by galectin 8 is expected to reveal various biofunctions and bioactions for cancer therapy, neutrophil-associated disorders, physiological actions and biologically active actions, cell adhesion that occurs due to saccharide chain recognition via the saccharide chain binding property, cell-to-cell communication, cell activation, and others, including involvement in metastasis/invasion of tumor cells, thereby promising therapeutic techniques for a variety of diseases, disorders and pathological or abnormal conditions. However, recombinant galectin 8 (rGal 8) has a link area susceptible to protease wherein said link area connects two CRDs together, and is therefore readily digestible with proteolytic enzymes. The proteolytic cleavage of rGal 8 will result in loss of the aforementioned activity.

The present inventors have conducted an extensive research on various molecules in order to solve the above problems. As a result, the present inventors have succeeded in producing novel molecules having a more stable molecular structure against the action of protease while the carbohydrate recognizing activity of wild type galectin 8 is retained. The present inventors have succeeded in constructing highly stabilized modified molecules without adversely affecting the aforementioned activity wherein said molecule has an altered Gal 8 link area that links two CRDs of Gal 8 together. Therefore, the present invention has been achieved.

The present invention provides the following:
(1) A protein, or a salt thereof, comprising a functional mutant galectin 8 protein with an amino acid sequence that differs from an amino acid sequence of wild type galectin 8 or a protein with substantially equivalent galectin 8 activity wherein said functional mutant galectin 8 protein has a modified link peptide or a modified site or region in the vicinity of the galectin 8 link peptide.
(2) The protein, or a salt thereof, according to the above (1), wherein said functional mutant galectin 8 protein has not only a modified sequence that differs from an amino acid sequence of wild type galectin 8 or a protein with substantially equivalent galectin 8 activity by the deletion, substitution or addition of at least one or more amino acid residues at a link peptide or a site or region in the vicinity of the galectin 8 link peptide but also altered susceptibility to degradation of said galectin 8 link peptide as compared to wild type galectin 8.
(3) The protein, or a salt thereof, according to the above (1) or (2), wherein said protein with substantially equivalent galectin 8 activity is at least 70% or more homologous to wild type galectin 8 at an amino acid level.
(4) The protein, or a salt thereof, according to any of the above (1) to (3), wherein
   [1] the N-terminal carbohydrate recognition domain (NCRD) of wild type galectin 8 or a polypeptide with substantially equivalent galectin 8 NCRD activity is coupled with
   [2] the C-terminal carbohydrate recognition domain (CCRD) of wild type galectin 8 or a polypeptide with substantially equivalent galectin 8 CCRD activity via
   [3] a modified link peptide with an amino acid sequence that differs from an amino acid sequence of wild type galectin 8 link peptide by the deletion, substitution or addition of at least one or more amino acid residues at a galectin 8 link peptide region.
(5) The protein, or a salt thereof, according to any of the above (1) to (4), wherein
   [1] a member selected from the group consisting of a polypeptide having an amino acid sequence of SEQ ID NO: 3, a polypeptide having not only substantially equivalent SEQ ID NO: 3 polypeptide activity but also an amino acid sequence at least 70% homologous to SEQ ID NO: 3, and a polypeptide having a mutant amino acid sequence that differs from an amino acid sequence of SEQ ID NO: 3 by the deletion, substitution or addition of at least 1 to 8 amino acid residues on the SEQ ID NO: 3 amino acid sequence is coupled with
   [2] a member selected from the group consisting of a polypeptide having an amino acid sequence of SEQ ID NO: 4, a polypeptide having not only substantially equivalent SEQ ID NO: 4 polypeptide activity but also an amino acid sequence at least 70% homologous to SEQ ID NO: 4, and a polypeptide having a mutant amino acid sequence that differs from an amino acid sequence of SEQ ID NO: 4 by the deletion, substitution or addition of at least 1 to 21 amino acid residues on the SEQ ID NO: 4 amino acid sequence via
   [3] a modified link peptide with an amino acid sequence that differs from an amino acid sequence of a member selected from the group consisting of SEQ ID NOs 9 and 10 by the deletion, substitution or addition of at least one or more amino acid residues on any amino acid sequence of SEQ ID NOs 9 to 10, provided that the deletion of residues 29 to 70 on SEQ ID NO: 10 is excluded.
(6) A nucleic acid molecule comprising a nucleotide sequence encoding the protein according to any of the above (1) to (5).
(7) The nucleic acid molecule according to the above (6), wherein said molecule is a polynucleotide.
(8) The nucleic acid molecule according to the above (6) or (7), wherein said molecule is DNA or RNA.
(9) A recombinant vector comprising the nucleic acid molecule according to any of the above (6) to (8).
(10) The recombinant vector according to the above (9) wherein said vector comprises a nucleotide sequence coding for a protein marker and/or a peptide marker in combination with the nucleic acid molecule according to any of the above (6) to (8).
(11) A transformed or transfected cell carrying the nucleic acid molecule according to any of the above (6) to (8) or the recombinant vector according to the above (9) or (10).
(12) The transformed or transfected cell according to the above (11), wherein said host cell is procaryotic or eucaryotic.
(13) A pharmaceutical drug comprising an effective amount of at least one member selected from the group consisting of the protein according to any of the above (1) to (5), the nucleic acid molecule according to any of the above (6) to (8), the recombinant vector according to the above (9) or (10), and the transformed or transfected cell according to the above (11) or (12).
(14) The pharmaceutical drug according to the above (13) which is an immunoregulator, immunomodulator, anti-inflammatory agent, or depressant against endotoxin shock.
(15) The pharmaceutical drug according to the above (13) which is an antineoplastic, antitumor agent, or anti-metastatic agent.
(16) The pharmaceutical drug according to the above (13) which is a therapeutic or prophylactic agent for a pathological condition, disease or disorder wherein said agent utilizes at least one Gal-8 mutein activity selected form the group consisting of (1) hemagglutination, (2) induction of apoptosis, (3) induction of cell adhesion, (4) integrin α_{M}-binding, (5) proMMP-9 binding, proMMP-9 activation, or promotion of active form MMP-9 production, (6) promotion of superoxide production, (7) suppression of LPS-induced inflammation, (8) suppression of LPS-induced TNF-α, IL-12, and/or IFN-γ production, and (9) inhibition of endotoxin shock.
(17) An assay or test reagent or kit comprising an effective amount of at least one member selected from the group consisting of the protein according to any of the above (1) to (5), the nucleic acid molecule according to any of the above (6) to (8), the recombinant vector according to the above (9) or (10), and the transformed or transfected cell according to the above (11) or (12).
(18) An inducer for neutrophil adhesion, comprising an effective amount of at least one member selected from the group consisting of the protein according to any of the above (1) to (5), the nucleic acid molecule according to any of the above (6) to (8), the recombinant vector according to the above (9) or (10), and the transformed or transfected cell according to the above (11) or (12).
(19) The inducer according to the above (18), for inducing attachment of neutrophils to at least one member selected from the group consisting of vascular endothelial cells, interstitial cells, epithelial cells and artificial substrates.
(20) A method for screening for a neutrophil adhesion inhibitor, which comprises contacting a test sample with neutrophil in the presence of at least one protein according to any of the above (1) to (5) and assaying for neutrophil attachment.
(21) A method for screening for a proMMP-9 activation inhibitor, which comprises contacting a test sample with proMMP-9 in the presence of at least one protein according to any of the above (1) to (5) and assaying for MMP-9.
(22) A method for screening for a neutrophil superoxide production inhibitor, which comprises contacting a test sample with neutrophil in the presence of at least one protein according to any of the above (1) to (5) and assaying the neutrophil for the superoxide producing property.
(23) A method for screening for a galectin 8 inhibitor, which comprises contacting a test sample with integrin α_{M} in the presence of at least one protein according to any of the above (1) to (5) and assaying for bioactivities attributable to interactions between said protein and integrin α_{M}.
(24) A method for separating and/or detecting a substance, which comprises an application of its binding affinity to at least one protein according to any of the above (1) to (5).
(25) The method according to the above (24), wherein the substance is selected from the group consisting of sugar chain-containing compounds, integrin α_{M}, proMMP-9 and neutrophils.

### ADVANTAGEOUS PROFILES OF THE INVENTION

Modified galectin 8 molecules, which are designed on the basis of galectin 8, are more stabilized against proteases as compared to wild type galectin 8. Therefore, the modified Gal 8 molecules can be expected to be useful in eliciting and revealing the in vivo functions of Gal 8. Said modified Gal 8 molecules are also applicable to studies on functions and actions of galectin 8 that may contribute to the regulation and control of various bioreactions including the regulation of tumorized cells, immunoregulation, and the control of allergy and inflammation, the inhibition of LPS-induced inflammation, and the protection of endotoxin shock (including endotoxin lethality). Further, said modified Gal 8 molecules and related substances thereof have bright prospects for reagents and agents in clinical, molecular biological biochemical and medical applications.

The above objects and other objects, features, advantages, and aspects of the present invention are readily apparent to those skilled in the art from the following disclosures. It should be understood, however, that the disclosures in the specification including the following best modes of carrying out the invention, examples, and others are illustrating preferred embodiments of the present invention and given for purposes of illustration only. It will become apparent to the skilled in the art that a great number of variations and/or alterations (or modifications) of this invention may be made based on knowledge from the disclosure in the following parts and other parts of the specification without departing from the spirit and scope thereof as disclosed herein. All of the patent publications and reference documents cited herein for illustrative purposes are hereby incorporated by reference into the present disclosure.

### BRIEF DESCRIPTION OF THE DRAWINGS

FIG. 1 is a scheme illustrating steps for construction of modified galectin 8 mutein (G8NC(null)) expression vector.
FIG. 2 shows an amino acid sequence of modified galectin 8 protein, G8NC(null).
FIG. 3 is a photo showing electrophoretic patterns for an expressed modified galectin 8 protein (G8NC(null)) product and a purified expressed G8NC(null) product.
FIG. 4 is a photo showing electrophoretic patterns resulting from comparison for resistance against proteases between wild type galectin 8 (G8(M)) and modified galectin 8 protein (G8NC(null)). The left drawing shows test results for resistance against elastase, and the right drawing for resistance against trypsin.
FIG. 5 is a graph showing comparison results for bioactivity between wild type galectin 8 (G8(M)) and modified galectin 8 protein (G8NC(null)). The activity of inducing peripheral blood neutrophil adhesion was assayed.
FIG. 6 is a graph showing assay results for the efficacy of galectin 8 mutein (G8= G8NC(null)) on endotoxin-administered mice (serum TNF-α levels).
FIG. 7 is a graph showing assay results for the efficacy of galectin 8 mutein (G8= G8NC(null)) on endotoxin-administered mice (serum IL-12(p70) levels).
FIG. 8 is a graph showing assay results for the efficacy of galectin 8 mutein (G8= G8NC(null)) on endotoxin-administered mice (serum IFN-γ levels).

### BEST MODES OF CARRYING OUT THE INVENTION

The invention described herein draws on previously published work and pending patent applications, for example, Japan Patent Application No. 2002-46478 (JP, A, 2003-246749), and Japan Patent Application No. 2003-9173. All such published work and pending patent applications are hereby incorporated by reference in full.

The "modified galectin 8 mutein", "modified galectin 8 variant", "modified galectin 8", or "modified galectin 8 protein" refers to a substance provided with an activity to specifically bind to a specific saccharide chain wherein said activity is retained by the carbohydrate recognition domain of galectin 8, or its analogous activity (including qualitative or/and quantitative). It is noted that galectin 8 has hemagglutinating activity, activity to induce cell adhesion such as neutrophil adhesion, integrin α_{M}-binding activity, proMMP-9-binding activity, activity to accelerate active form MMP-9 production, activity to increase superoxide production, or/and activity to induce apoptosis of a specific cell. The modified galectin 8 protein (Gal-8 mutein) may be a substance having at least one of said activities, owned by wild type galectin 8, or an analogous activity thereof, and a substance wherein the bioactivity retained by wild type galectin 8 is altered or modified, which is preferable in some cases. The particularly preferred modified galectin 8 mutein herein is a molecule retaining a more desirable property (in order to serve as a biologically active reagent in diagnostic, analytic, medical, or pharmaceutical applications) over wild type galectin 8.

The modified galectin 8 mutein may be, for example, a mutant galectin 8 protein, or a salt thereof, wherein the link peptide of wild type (native) galectin 8 or a protein with substantially equivalent galectin 8 activity is modified, or the site or region in the vicinity of said link peptide is modified; a modified galectin 8 protein, or a salt thereof, having not only a modified sequence that differs from an amino acid sequence of wild type galectin 8 or a protein with substantially equivalent galectin 8 activity by at least one deletion, substitution or addition of one or more amino acid residues at a link peptide or a site or region in the vicinity of the galectin 8 link peptide but also altered susceptibility to degradation of said galectin 8 link peptide as compared to wild type galectin 8; a protein, or a salt thereof, not only retaining substantially equivalent galectin 8 activity but also being at least 70%, still at least 75%, yet at least 80%, also at least 85%, at least 90%, or at least 95% (or higher) homologous to the amino acid sequence of wild type galectin 8; a protein, or a salt thereof, having the formula:
NCRD Peptide (1)-Link Peptide (3)-CCRD Peptide (2)
in which (i) the NCRD Peptide (1) is selected from the group consisting of the N-terminal carbohydrate recognition domain (NCRD) of wild type galectin 8 and polypeptides with substantially equivalent Gal-8 NCRD activity, (ii) the CCRD Peptide (2) is selected from the group consisting of the C-terminal carbohydrate recognition domain (CCRD) of wild type galectin 8 and polypeptides with substantially equivalent Gal-8 CCRD activity, (iii) the Link Peptide (3) is a modified link peptide that differs from the link peptide amino acid sequence of wild type galectin 8 by at least one deletion, substitution or addition of one or more amino acid residues in the galectin 8 link peptide amino acid sequence, and the NCRD Peptide (1) is linked to the CCRD Peptide (2) via the Link Peptide (3); etc.

The nucleotide and amino acid sequences of galectin 8 are disclosed in, for example, NP_963837. galectin 8 isofor...[gi:42544187] (Search "Protein" for "42544187" at NCBI Internet Web Page); NM_201543. Homo sapiens lect...[gi:42544186] (Search "CoreNucleotide" for "42544186" at the NCBI); NP_963838. galectin 8 isofor...[gi:42544191] (Search "Protein" for "42544191" at the NCBI); NM_201544. Homo sapiens lect...[gi:42544190] (Search "CoreNucleotide" for "42544190" at the NCBI); NP 963839. galectin 8 isofor...[gi:42544193] (Search "Protein" for "42544193" at the NCBI); NM_201545. Homo sapiens lect...[gi:42544192] (Search "CoreNucleotide" for "42544192" at the NCBI), on databases at NCBI. It has been found that galectin 8 has mutations on the nucleotide sequence for the coding region, i.e., 8 nucleotide mutations have been reported and 4 positions among them are known to result in amino acid mutations (C. Maier et al., European Urology, 42, pp. 301 to 307 (2002)). It has been reported that, for SEQ ID NO: 5, nucleotides may be mutated at positions 56 (a → t), 72 (c → t), 106 (t → c), 165 (t → c), 166 (g → a), 330 (a → g), 552 (c → g), and 816 (c → t), respectively, in the DNA sequence and amino acid mutations take place at positions 19 (Tyr → Phe), 36 (Cys → Arg) , 56 (Val → Met), and 184 (Ser → Arg), respectively, in the amino acid sequence. Human galectin-8 coding genes which are known as aforementioned can serve preferably as templates for constructing the inventive mutants (muteins). Alternatively, it is possible to use each gene-isolate cloned from said gene-expressing sources such as human epidermoid carcinoma cell lines (A431). Typically, it is possible to obtain and use the target coding gene portion via utilizing suitable nucleotide sequences as primers.

The mutant polynucleotides (or mutant nucleic acid molecules) are any as long as they are typically those in which an initiation codon (codon coding for Met) and a termination codon (stop codon such as, for example, TGA, TAA and TAG) are existing or added to the coding sequence lacking such a codon, and those coding for a peptide having not only an amino acid sequence with at least 80% homology to the protein encoded by said nucleotide sequence wherein said amino acid sequence, but also either a sugar chain binding activity or a substantially equivalent biological activity such as an equivalent antigenicity, that is, those containing a nucleotide sequence with the same efficacy. The polynucleotides (or nucleic acids) are single- and double-stranded DNA, RNA, DNA:RNA hybrids, synthetic DNA, and others. They may be any of human genome DNA, human genomic DNA libraries, human tissue/cell-derived cDNA, and synthetic DNA. The polynucleotide (or nucleic acid) sequences can be modified (by addition, deletion, substitution, etc.), and those thus modified may be encompassed herein. Further, as discussed herein below, the polynucleotides (or nucleic acid molecules) are those encoding any of peptides as disclosed herein, particularly mutant (mutein or modified protein) peptides or fragments thereof, and are preferably DNA. They may include those derived from a mammal such as human, chimpanzee, monkey, mouse, rat, cattle, pig, goat, sheep, dog, cat, and rabbit. The terms "nucleotide sequence with the same efficacy", "equivalently effective nucleotide sequence" and "equivalent nucleotide sequence" refer to those which hybridize with a nucleotide sequence with 5 or more contiguous nucleotide residues, preferably 10 or more contiguous nucleotide residues, more preferably 15 or more contiguous nucleotide residues, and still more preferably 20 or more contiguous nucleotide residues, in the nucleotide sequence encoding the CRD, under stringent conditions, and encode a substantially equivalent amino acid sequence to human galectin 8, their complementary strands, etc. in view of sugar chain binding activity.

In preferred embodiments, the modified galectin 8 mutein includes, for example, molecules wherein
(1) the NCRD of galectin 8 is selected from the group consisting of the amino acid sequence of SEQ ID NO: 3, a mutant amino acid that differs from the amino acid sequence of SEQ ID NO: 3 by at least one deletion, substitution, or addition of one or more amino acid residues in the SEQ ID NO: 3 amino acid sequence, and an amino acid sequence which not only is at least 70%, still at least 75%, yet at least 80%, also at least 85%, at least 90%, or at least 95% (or higher) homologous to the amino acid sequence of SEQ ID NO: 3, but also retains lactose binding activity;
(2) the CCRD of galectin 8 is selected from the group consisting of the amino acid sequence of SEQ ID NO: 4, a mutant amino acid that differs from the amino acid sequence of SEQ ID NO: 4 by at least one deletion, substitution, or addition of one or more amino acid residues in the SEQ ID NO: 4 amino acid sequence, and an amino acid sequence which not only is at least 70%, still at least 75%, yet at least 80%, also at least 85%, at least 90%, or at least 95% (or higher) homologous to the amino acid sequence of SEQ ID NO: 4, but also retains lactose binding activity; and
(3) the link region that is a link between the above (1) and (2) is selected from the group consisting of the amino acid sequence of SEQ ID NO: 9, and a mutant amino acid that differs from the amino acid sequence of SEQ ID NO: 9 by at least one deletion, substitution, or addition of one or more amino acid residues in the SEQ ID NO: 9 amino acid sequence; preferably those which are more stabilized against proteolytic enzymes, such as matrix metalloproteinases, than native galectin 8 (wild type galectin 8). Said link peptide region (3) includes deletion analogues with at least one amino acid deletion of one or more (for example, from 1 to 2, preferably from 3 to 4, still preferably from 5 to 6, more preferably from 7 to 8, and inter alia from 1 to 9 or not less than 9) amino acid residues in the amino acid sequence of SEQ ID NO: 9; substitution analogues where one or more (for example, from 1 to 9, preferably from 1 to 8, still preferably from 1 to 6, more preferably from 1 to 4, and inter alia from 1 to 2 or not less than 9) amino acid residues in said amino acid sequence are substituted with other residues; and addition analogues with at least one amino acid addition (or insertion) of one or more (for example, from 1 to 28, preferably from 1 to 20, still preferably from 1 to 15, more preferably from 1 to 10, and inter alia from 1 to 5) amino acid residues, provided that residual portions derived by removing SEQ ID NO: 9 from SEQ ID NO: 10 are excluded. In representative embodiments, said link region (3) includes those having a deleted amino acid sequence that differs from the amino acid sequence of SEQ ID NO: 9 by amino acid substitution with HM, RIP, or any of sequences consisting of 2 amino acids. The substitution, deletion or insertion (addition) of amino acids may or may not cause a great alteration in physiological or chemical properties of a polypeptide. In some cases, a desirable modification will be provided. Substituents of amino acids in the amino acid sequence can be selected from other amino acids in the class to which the amino acid belongs. For instance, non-polar (hydrophobic) amino acids include alanine, phenylalanine, leucine, isoleucine, valine, proline, tryptophan, methionine and the like; polar (neutral) amino acids include glycine, serine, threonine, cysteine, tyrosine, asparagine, glutamine and the like; amino acids having a positive charge (basic amino acids) include arginine, lysine, histidine and the like; and amino acids having a negative charge (acidic amino acids) include aspartic acid, glutamic acid and the like.

Further, the link region (3) includes those having a substituted amino acid sequence that differs from the sequence of SEQ ID NO: 9 or 10 by replacement with HM, RIP, or any of sequences consisting of 2 amino acids, provided that the amino acid residue positions 29 to 70 of SEQ ID NO: 10 are excluded for this replacement; those having a deleted amino acid sequence that differs from the sequence of SEQ ID NO: 9 or 10 by retention of 6 amino acid residues and deletion of all the residual amino acid residues, excluding the amino acid residue positions 29 to 70 of SEQ ID NO: 10; and others. The link region (3) also includes deletion analogues with amino acid deletions of one or more (for example, from 1 to 5, preferably from 3 to 10, still preferably from 5 to 15, more preferably from 7 to 20, and inter alia from 1 to 28) amino acid residues in the amino acid sequence of SEQ ID NO: 9 or 10, excluding the amino acid residue positions 29 to 70 of SEQ ID NO: 10; substitution analogues where one or more (for example, from 1 to 9, preferably from 1 to 8, still preferably from 1 to 6, more preferably from 1 to 4, and inter alia from 1 to 2 or not less than 9) amino acid residues in said amino acid sequence are substituted with other residues; and addition analogues with amino acid additions (or insertions) of one or more (for example, from 1 to 60, preferably from 1 to 40, still preferably from 1 to 20, more preferably from 1 to 10, and inter alia from 1 to 5) amino acid residues, provided that the amino acid residue positions 29 to 70 of SEQ ID NO: 10 are excluded.

The mutants as aforementioned are all included in the present invention as long as they retain the domain structure or active carbohydrate binding structure characteristic of native human galectin 8 (or wild type human galectin 8). Also, it is thought that the peptides or polypeptides of the present invention may include those having all or part of substantially equivalent primary structure conformations to those of native human galectin 8 proteins. Furthermore, it is also thought that the inventive peptides or polypeptides may include those having substantially equivalent biological activity as compared to said native human galectin 8 proteins. Moreover, each of the N-terminal and C-terminal CRDs thereof can be, for example, one derived from the mutants which naturally occur. The human-derived proteins (or peptides or polypeptides) according to the present invention include, for example, those having each domain with an amino acid sequence which is at least 60% or more, and in some cases at least 70% or more, homologous to at least one sequence selected from SEQ ID NOs: 5 to 8 in the Sequence Listing, inter alia the N-terminal CRD or C-terminal CRD, at an amino acid level, and more preferably those having each domain with an 80, or 90%, or more homologous amino acid sequence to any amino acid sequence of said SEQ ID NOs: 5 to 8, inter alia the NCRD or CCRD. The peptide fragments (partial peptides) derived from the inventive human-derived protein may be any as long as they are part of said human-derived proteins (that is, partial peptides or fragmented peptides of said proteins) and have substantially equivalent activity to the inventive galectin 8 protein. For example, the partial peptides (or peptide fragments) of the protein according to the present invention include peptides having a sequence with at least 5 or more, preferably 20 or more, still preferably 50 or more, more preferably 70 or more, still more preferably 100 or more, and, in some cases, 200 or more amino acid residues contained in the modified Gal-8 variant-constituent amino acid sequence, preferably wherein said amino acid residues are contiguous. Preferable examples thereof are those having the same homology as aforementioned, with respect to homology to the region corresponding to any amino acid sequence of SEQ ID NOs: 5 to 8 in the Sequence Listing.

The term "substantially equivalent" used herein means that proteins of interest are substantially equivalent or equal one another in view of activity, for example, hemagglutinating, neutrophil adhesion-inducing, integrin α_{M}-binding, proMMP-9-binding, active form MMP-9 production-accelerating, superoxide production-accelerating, cytotoxic, apoptosis-inducing, anti-inflammatory, LPS-induced inflammation-inhibitory, anti-allergic, immunoregulatory (or immunomodulatory), LPS-induced TNF-α, IL-12, and/or IFN-γ production-inhibitory, endotoxin shock-depressant, saccharide chain-binding, physiological or biological activity. Further, the meanings of that term may include a case having the substantially same quality of activity. The substantially same quality of activity can include, for example, hemagglutination, induction of neutrophil adhesion, integrin-binding activity, cytotoxity, apoptosis-inducing activity, depression of endotoxin shock, etc. The substantially same quality of activity indicates that these activities are qualitatively homogeneous; for example, they are physiologically, pharmacologically or biologically homogeneous. For instance, it is preferable that the activities including the hemagglutination, the induction of cell adhesion such as neutrophil adhesion, the binding activity such as the integrin-binding activity, the cytotoxity, the apoptosis-inducing activity, and the depression of endotoxin shock, are equivalent (for example, from about 0.001 to 1000 fold, preferably from about 0.01 to 100 fold, more preferably from about 0.1 to 20 fold, and still preferably from about 0.5 to 2 fold), but quantitative elements such as the extents of these activities, molecular weights of the proteins etc. may be different.

The "modified galectin 8 polypeptide", "modified Gal-8 variant polypeptide" or "modified Gal-8 mutein polypeptide" embodies modified variants, derivatives, analogues, fragments, chimeras and mutants of the native sequence of wild type galectin 8. The polypeptides are encoded by recombinantly produced polynucleotides sequences designed to encode the specific modified galectin 8 polypeptide intended for expression in a host cell.

The "modified galectin 8 variant therapeutic agent" includes molecules derived from modified Gal-8 variant-coding polynucleotide (modified galectin 8 mutein polynucleotide) or modified Gal-8 polypeptide sequence, and variants, mutants, analogues, chimeras, and fragments of such modified Gal-8 polynucleotide or polypeptide. Polynucleotide modified galectin 8 mutein therapeutic agents are generally sequences encoding a modified galectin 8 polypeptide that can be recombinantly expressed in a host cell. Additionally, a modified galectin 8 mutein therapeutic agent can be a small molecule agonist of galectin 8 activity. Other modified Gal-8 mutein therapeutic agents may include substances providing a modified Gal 8 mutein, and modulators of galectin 8 activity that have modified galectin 8 mutein activity and cause a prophylactic and/or therapeutic effect on disorders, diseases, and abnormal conditions associated with the insufficiency or absence of galectin 8 activity. A modulator of galectin 8 activity can be, for example, a polynucleotide, a polypeptide, or a small molecule.

The term "diagnostic agent" as used herein refers to any agent that contributes to one or more diagnostic actions used in diagnostic applications of the invention. These diagnostic applications include methods for determining the presence of galectin 8-producing cells, or methods for determining the presence of galectin 8-binding substance presenting cells. The diagnostic agents include the following: DNA encoding a modified galectin 8 protein (galectin 8 mutein), a stabilized galectin 8 variant (mutein), and cells or cell homogenates having the stabilized galectin 8 variant (mutein).

The term "therapeutic agent" as used herein can be any agent that accomplishes or contributes to the accomplishment of one or more therapeutic actions or elements used in therapeutic applications of the invention. For example, where the therapeutic agent is a polynucleotide designed to express a modified galectin 8 mutein polypeptide, that agent is a polynucleotide that can be administered to and expressed in a cell in the mammal. Thus, the active form of the agent will initially be the expressed polypeptide.

The modified galectin 8 variant therapeutic agent is a therapeutic agent with the bioactivity of galectin 8, or a therapeutic agent derived from modified galectin 8, such as a polypeptide capable of binding on a certain saccharide chain longer than native galectin 8 or a polynucleotide encoding a modified galectin 8 mutein polypeptide that is more stabilized against proteolytic enzymes, such as elastase and trypsin, than native galectin 8. The therapeutic agent achieves a therapeutic goal, alone or in combination with other agents (for example, an agent used in other known treatments for a particular tumor or autoimmunity in conjunction with administration of modified galectin 8 mutein, or a gene delivery vehicle capable of facilitating expression of modified galectin 8 mutein in the mammal). The therapeutic agents may include for example modified galectin 8 variant-containing drugs developed for other purposes, agonists of galectin 8, and further drugs that modulate or regulate galectin 8 activity. The therapeutic agent can be, for example, a small organic molecule, a peptide, a peptoid or peptidic compound, a polynucleotide encoding a modified galectin 8 mutein polypeptide, a modified galectin 8 variant polypeptide, or a transformed or transfected cell expressing a chimera or mutant of the modified galectin 8 mutein that is stabilized toward protease more than native galectin 8 (wild type galectin 8).

The "combination therapeutic agent" is a therapeutic composition having several components or agents that produce their separate effects when administered together, and may produce a synergistic effect when administered together to treat a disease. Preferably, the separate effects of the combination therapeutic agent combine to result in a larger therapeutic effect, for example elimination or reduction of tumors, normalization of tumor cells or tissues, recovery from an autoimmune disease and long term survival. An example of separate effects resulting from administration of a combination therapeutic agent is the combination of such effects as short-term, or long-term remission, or decrease of an autoimmune response to a particular type of cell in the patient. An example of the combination therapeutic agent according to the present invention would be administration of a gene delivery vehicle including a polynucleotide encoding a modified galectin 8 protein (Gal-8 mutein) in combination with a polynucleotide encoding at least one member selected from the group consisting of IFNs, IL-2, and other cytokines. Alternatively, two gene delivery vectors can be used, one expressing modified galectin 8 protein (Gal-8 mutein) and one encoding at least one of cytokines. Also IFNs, IL-2, and others, or a gene delivery vehicle expressing at least one member selected from the group consisting of IFNs, such as IFN-γ, IL-2, and other cytokines, can be administered to upregulate modified galectin 8 protein (Gal-8 mutein) expression in anticipation of an administration of modified galectin 8 protein (Gal-8 mutein) for inducing apoptosis in target cells. The various therapeutic agents can be administered in the same pharmaceutically acceptable carrier at the same time, followed, for example, by repeated administration of one or all of the individual agents as needed to make the therapy efficacious.

The term "gene delivery vehicle" refers to a component that facilitates delivery to a cell of a coding sequence for expression of a polypeptide in the cell. The cell can be inside the mammal, as in in vivo gene therapy, or can be removed from the mammal for transfection or transformation and returned to the mammal for expression of the polypeptide as in ex vivo gene therapy. The gene delivery vehicle can be any component or vehicle capable of accomplishing the delivery of a gene to a cell, for example, a liposome, a particle, or a vector. The gene delivery vehicle includes a recombinant vehicle, such as a recombinant viral vector, a nucleic acid vector (such as plasmid), a naked nucleic acid molecule such as genes, a nucleic acid molecule complexed to a polycationic molecule capable of neutralizing the negative charge on the nucleic acid molecule and condensing the nucleic acid molecule into a compact molecule, a nucleic acid associated with a liposome (U.S. Patent Nos. 5,166,320; 5,547,932; Wang et al., Proc. Natl. Acad. Sci. USA, 84:7851, 1987), and others. Said gene delivery vehicles include certain eukaryotic cells (e.g., a producer cell), that are capable of delivering a nucleic acid molecule biologically having one or more desirable properties to host cells. As discussed further below, the desirable properties include the ability to express a desired substance, such as, for example, a protein, enzyme, or antibody, and/or the ability to provide a biological activity, which is where the nucleic acid molecule carried by the gene delivery vehicle is itself the active agent without requiring the expression of a desired substance. One example of such biological activity is found in gene therapy where the delivered nucleic acid molecule incorporates into a specified gene so as to inactivate the gene and "turn off" the product formation directed by the gene, thereby allowing the specific expression of said delivered nucleic acid molecule. Gene delivery vehicle refers to an assembly which is capable of directing the expression of one or plural sequences or genes of interest.

The gene delivery vehicle generally includes promoter elements and may include a signal that directs polyadenylation. In addition, the gene delivery vehicle includes a sequence which, when transcribed, is operably linked to one or plural sequences or genes of interest and acts as a translation initiation sequence. The gene delivery vehicle may also include a selectable marker such as Neo, SV²Neo, TK, hygromycin, bleomycin (phleomycin), puromicin, histidinol, or DHFR, as well as one or more restriction sites and a translation termination sequence. Gene delivery vehicles as used within the present invention refers to recombinant vehicles, such as viral vectors (Jolly, Cancer Gen. Therapy, 1: 51 to 64, 1994), nucleic acid vectors, naked DNA, liposomal DNA, cosmids, bacteria, and certain eukaryotic cells (including producer cells; see U.S. Patent No. 6,333,195).

The term "biologically active" refers to a molecule that retains a specific activity. A biologically active modified galectin 8 polypeptide (galectin 8 mutein, or modified Gal-8 variant), for example, retains not only the ability to bind specifically a certain saccharide chain on the carbohydrate recognition domain, as possessed by the CRD of galectin 8, or a substantially equivalent property thereto, but also qualitatively or quantitatively the more stable property against digestion with proteolytic enzymes such as elastase and trypsin, as compared to native galectin 8 (wild type galectin 8). For example, said biologically active modified galectin 8 polypeptide has hemagglutinating activity, the ability to induce cell adhesion such as neutrophil adhesion, integrin α_{M}-binding activity, proMMP-9 binding activity, the ability to accelerate production of active form MMP-9, the ability to promote superoxide production, the ability to suppress inflammation induced with LPS, the ability to suppress LPS-induced TNF-α, IL-12, and/or IFN-γ production, the ability to suppress endotoxin shock, antitumor activity or the ability to activate the apoptotic pathway leading to apoptosis, as owned by native galectin 8.

The "nucleic acid molecule" or "polynucleotide," as used herein, refers to RNA or DNA molecules, or DNA:RNA hybrids that encode a specific amino acid sequence or its complementary strand. The "coding sequence" as used herein refers to any of RNA, DNA, and DNA:RNA hybrids that encode a specific amino acid sequence or its complementary strand. The polynucleotide may include, for example, an antisense oligonucleotide, or a ribozyme, and may also include such items as a 3'- or 5'-untranslated region of a gene, or an intron of a gene, or other region of a gene that does not make up the coding region of the gene. The DNA or RNA may be single stranded or double stranded. Synthetic nucleic acids or synthetic polynucleotides can be chemically synthesized nucleic acid sequences, and may also be modified with chemical moieties to render the molecule resistant to degradation. The polynucleotide can be generated, for example, by polymerase chain reaction (PCR) amplification, or recombinant expression of complementary DNA or RNA, or by chemical synthesis.

The term "expression control sequence" or "regulatory sequence" refers to a sequence that is conventionally used to effect expression of a gene that encodes a polypeptide and include one or more components, elements, or factors that affect expression, including transcription and translation signals. The expression control sequence that is appropriate for expression of the present polypeptides differs depending upon the host system in which the polypeptide is to be expressed.

The "polypeptide" of the invention is any one comprising any part of the modified galectin 8 protein (Gal-8 mutein) including the mature protein, as long as it includes a modified galectin 8 variant polypeptide or a fragment thereof, and may further include truncations, variants, alleles, analogs and derivatives thereof. The variants can be spliced variants expressed from the same gene as the related protein. Unless specifically mentioned otherwise, such a polypeptide possesses one or more of the bioactivities of the galectin 8 protein, including for example specific binding affinity for a specific saccharide chain or binding activity to a specific partner. This term "polypeptide" is not limited to a specific length of the product of the gene. Thus, polypeptides that are identical or contain at least 60%, preferably 70%, still preferably 80%, more preferably 90%, and most preferably 95% homology to the target protein or the mature protein with regard to the N-terminal carbohydrate recognition domain (NCRD) and C-terminal carbohydrate recognition domain (CCRD) of galectin 8, wherever derived, from human or nonhuman sources are included within this definition of the polypeptide. Also included, therefore, are alleles and variants of the product of the gene that contain amino acid substitutions, deletions, or insertions. The amino acid substitutions can be conservative amino acid substitutions or substitutions to eliminate non-essential amino acid residues, such as to alter a glycosylation site, a phosphorylation site, an acetylation site, or to alter the folding pattern by altering the position of the cysteine residue that is not necessary for function. Conservative amino acid substitutions are those that preserve the general charge, hydrophobicity/hydrophilicity and/or steric size (bulk) of the amino acid substituted, for example, substitutions between the members of the following groups are conservative substitutions: Gly/Ala, Val/Ile/Leu, Asp/Glu, Lys/Arg, Asn/Gln, Ser/Cys/Thr and Phe/Trp/Tyr.

Analogues include peptides having one or more peptide mimics, also known as peptoids, that possess the target protein-like activity. Included within the definition as set forth herein are, for example, polypeptides containing one or more analogues of an amino acid (including, for example, unnatural amino acids, etc.), polypeptides with substituted linkages, as well as other mutations/modifications known in the art, both naturally occurring and non-naturally occurring. The term "polypeptide" also does not exclude post-translational modifications of the polypeptide, for example, glycosylations, acetylations, phosphorylations, myristoylations and the like.

The term "naked DNA" as used herein refers to polynucleotide DNA for administration to a mammal for expression in the mammal. The polynucleotide can be, for example, a coding sequence, and the polynucleotide DNA can be directly or indirectly connected to an expression control sequence that can facilitate the expression of the coding sequence once the DNA is inside a cell. The indirect connection is equivalent from the perspective of facilitating the expression of the DNA in the mammalian cells, and merely allows the possibility of the inclusion of other sequences between the regulatory region and the coding sequence that may facilitate the expression further, or may merely act as a linker or spacer to facilitate connecting the two polynucleotide regions together.

The "vector" used herein refers to an assembly which is capable of directing the expression of one or more sequences of interest, or one or more genes of interest. The vector must include transcriptional promoter/enhancer or one or more locus defining elements, or other elements which control gene expression by other means, such as alternate splicing, nuclear RNA export, post-translational modification of messenger, or post-transcriptional modification of protein. In addition, the vector must include a sequence which, when transcribed, is operably linked to one or more sequences or genes of interest and acts as a translation initiation sequence. Optionally, the vector may also include a signal which directs polyadenylation, a selectable marker such as Neo, TK, hygromycin, bleomycin (phleomycin), histidinol, or DHFR, as well as one or more restriction sites and a translation termination sequence. Further, if the vector is placed into a retrovirus, the vector must include a packaging signal, long terminal repeats (LTRs), and positive and negative strand primer binding sites appropriate to the retrovirus used (if these are not already present).

The "tissue-specific promoter" refers to transcriptional promoter/enhancer or locus defining elements, or other elements which control gene expression as discussed above, which are preferentially active in a limited number of tissue types or cell types. Representative examples of such tissue-specific promoters include the PEPCK promoter, HER2/neu promoter, casein promoter, IgG promoter, chorionic embryonic antigen promoter, elastase promoter, porphobilinogen deaminase promoter, insulin promoter, growth hormone factor promoter, tyrosine hydroxylase promoter, albumin promoter, α-fetoprotein promoter, acetyl-choline receptor promoter, alcohol dehydrogenase promoter, α- or β-globin promoters, T-cell receptor promoter, osteocalcin promoter, etc.
The "event-specific promoter" refers to transcriptional promoter/enhancer or locus defining elements, or other elements which control gene expression as discussed above, whose transcriptional activity is altered upon response to cellular stimuli. Representative examples of such event-specific promoters include thymidine kinase or thymidilate synthase promoters, α- or β-interferon promoters, promoters that respond to the presence of hormones (natural, synthetic or from other nonhost organisms, e.g., insect hormones), etc.

The term "fusion protein" or "fusion polypeptide" refers to proteins or polypeptides obtainable by the recombinant expression of more than one heterologous coding sequence in a vector or contiguous connection such that expression of the polypeptide in the vector results in expression of one polypeptide that includes more than one protein or portion of more than one protein. Most optimally, the fusion protein retains the biological activity of at least one of the polypeptide units from which it is built. Preferably, the fusion protein generates a synergistic improved bioactivity by combining the portion of the separate proteins to form a single polypeptide. The produced fusion protein can also be created with a polypeptide that has function and a peptide or polypeptide that has no function when expressed, but which serves a purpose for the expression of the polypeptide with activity. Examples of fusion proteins useful for the invention include any modified galectin 8 protein (Gal-8 mutein) fusion polypeptide genetically engineered to some advantage for the therapy, detection or assay, and further analysis or isolation/purification.

The term "chimera" or "chimeric protein" means an equivalent to fusion protein or fusion polypeptide. The "chimeric molecule" can be a fusion polypeptide, or a polynucleotide fusion molecule encoding a fusion polypeptide. The chimera can be constructed from ligated DNA coding sequences and expressed in a cell system, or administered in a vector for expression in vivo in an animal. For example, a chimera or fusion protein including a modified galectin 8 protein (Gal-8 mutein) can be administered in a gene therapy protocol in vivo or ex vivo.

The "patient" can be any treatable living organism, including but not limited to a eukaryote or a prokaryote. The patient eukaryote can be, for example, a vertebrate or an invertebrate. Thus, for example, the patient can be a fish, a bird, a worm, an insect, a mammal, a reptile, an amphibian, a fungus, or a plant, preferably a mammal. The mammal can be, for example a human.

Described below are general methods of making and using a modified galectin 8 protein (Gal-8 mutein) therapeutic agents and/or diagnostic agents. In one aspect, the present invention provides a technique for treating a disorder, disease, or abnormal condition occurred due to the deficiency or absence of physiological or biological activity retained by galectin 8. Said treating technique includes for example a step of providing a modified galectin 8 protein (Gal-8 mutein) therapeutic agent, and/or a step of administering an effective amount of the modified galectin 8 protein (Gal-8 mutein) therapeutic agent to a mammal bearing said disorder, etc. Modified galectin 8 proteins (Gal-8 muteins) are active in hemagglutination or induction of cell adhesion such as induction of neutrophil adhesion, capable of binding to integrin α_{M} or proMMP-9, active in promotion of active form MMP-9 production or acceleration of superoxide production, cytotoxic on malignant tumor cells, active in induction of apoptosis in malignant cells, antitumor (anticancer or antineoplastic) on malignant tumor cells, immunomodulatory (immunoregulatory), anti-inflammatory, antiallergic, and suppressive in LPS-induced inflammation, LPS-induced TNF-α, IL-12, and/or IFN-γ production or endotoxin shock. Therefore, modified galectin 8 proteins (Gal-8 muteins) can be expected to serve as anti-tumor agents (anti-neoplastic agents), anti-allergy agents, immunoregulators (immunomodulators), therapeutic agents for autoimmune diseases, anti-inflammatory agents, hormone alternatives, and endotoxin shock suppressants. Said treating technique includes a method for treating abnormal condition-manifesting activated T-cells. The "autoimmune disease", "autoimmune" and "autoimmunity" all refer to a disorder characterized by autoimmunity in the mammal which is the response of an immune system against self components. An autoimmune response can develop into a condition manifesting clinical symptoms. Although strictly speaking transplantation rejection is not an autoimmune reaction, where patient condition prescribes surgery to replace or graft cells, tissue or an organ, the body receiving the allograft can react immunologically against the foreign graft. "Transplantation rejection" occurs when during an allograft of cells, tissue, or an organ, from one member of a species to another, an immune response in the recipient results, sufficient to reject the transplanted cells, tissue or organ.

The inventive methods and therapeutic agents (drugs) can be applied to "tumors". Examples of such tumors may include malignant tumors. For example, a tumor that may metastasize to several sites is a malignant neoplasm, and the term "malignant neoplasm" is generally referred to as being epithelial or non-epithelial and may be distinguished as being cancer, sarcoma, or leukemia, etc. Among the general public, when the neoplasm or tumor is simply called "cancer", it refers to a malignant neoplasm or tumor. As used herein, the term "cancer" is employed in the broadest sense and should not be interpreted as being just an epithelial malignant neoplasm. The term "cancer" used herein may cover epithelial malignant tumors and non-epithelial malignant tumors (including those that are tumorigenic and non-tumorigenic), such as skin cancers (which may include melanomas), breast cancers, ovarian cancers, uterine cancers, malignant testicular tumors, prostatic cancers, urinary bladder cancers, renal cancers, thyroid cancers, pharyngeal/larynx cancers, tongue cancers, maxillary cancers, esophageal cancers, stomach cancers, colon/rectal cancers, lung/bronchial cancers, liver cancers (including liver cell cancers and intrahepatic bile duct cancers), extrahepatic bile duct/gall bladder cancers, pancreatic cancers, leukemia, malignant lymphoma, plasmacytoma, osteosarcoma, chondrosarcoma, leiomyosarcoma, rhabdomyosarcoma, liposarcoma, fibrosarcoma, malignant hemangioma, malignant hemangioendothelioma, brain tumors (including meningioma, glioma, astrocytoma), etc., but is not restricted to these. It should be understood that they may encompass those wherein the application of the inventive modified Gal-8 variant will give bright prospects, and further those wherein some sort of physiological or biological responses will take place in association with said modified Gal-8 variant.

Examples of "autoimmune diseases" that can be treated by the method and therapeutic agent of the invention include multiple sclerosis, Hashimoto's thyroiditis, systemic lupus erythematosus (SLE), Goodpasture's syndrome, Pemphigus, receptor autoimmunity, autoimmune hemolytic anemia, autoimmune thrombocytopenic purpura, osteoarthritis, chronic rheumatoid arthritis, scleroderma with anticollagen antibodies, mixed connective tissue disease, polymyositis, pernicious anemia, idiopathic Addison's disease, spontaneous infertility, glomerulonephritis, bullous pemphigoid, adrenergic drug resistance, chronic active hepatitis, primary biliary cirrhosis, autoimmune-based endocrine gland failure, vitiligo, vasculitis, post-myocardial infarction, cardiotomy syndrome, urticaria, atopic dermatitis, autoimmune-based asthma, autoimmune-based inflammatory reactions, granulomatous disorders, alkylizing spondylitis, poststreptococcal glomerulonephritis, autoimmune hemolytic anemia, encephalitis, autoimmune reaction secondary to lymphoma, degenerative disorders, and atrophic disorders. Autoimmune diseases manifesting receptor autoimmunity include, for example, Grave's disease, myasthenia gravis, insulin resistance and others. Autoimmune diseases of adrenergic drug resistance include, for example, asthma, cystic fibrosis, and others.

Other autoimmune diseases appropriate for the invention include, for example those for which an animal model exists, including, for example, Sjögren's syndrome (autoimmune dacryodentis or immune-mediated sialadenitis), autoimmune myocarditis, primary biliary cirrhosis (PBC), inflammatory heart disease, mercury-induced renal autoimmunity, insulin dependent diabetes mellitus (type I diabetes or IDD), post-thymectomy autoimmunity, a central nervous system (CNS) demyelination disorder, CNS lupus, narcolepsy, an immune-mediated PNS disorder, osteoarthritis, rheumatoid arthritis, uveitis, medullary cystic fibrosis, autoimmune hemolytic disease, autoimmune vasculitis, ovarian autoimmune disease, human scleroderma, etc. An autoimmune disease characterized by a central nervous system (CNS) demyelination disorder can be, for example, multiple sclerosis (MS), etc. A peripheral nervous system (PNS) autoimmune disease can be, for example, Guillain-Barre syndrome (GBS).

The modified galectin 8 protein (Gal-8 mutein) therapeutic agent can include a polypeptide, a polynucleotide, a small organic compound, a peptide, a peptoid compound, a peptidic substance, or others. The modified galectin 8 protein (Gal-8 mutein) therapeutic agent can be a modified galectin 8 protein (Gal-8 mutein) polypeptide, a polynucleotide encoding a modified galectin 8 protein (Gal-8 mutein) polypeptide, a fusion polypeptide comprising a portion of the inventive modified galectin 8 protein (Gal-8 mutein) polypeptide, a polynucleotide encoding a fusion polypeptide comprising a portion of said modified galectin 8 protein (Gal-8 mutein) polypeptide, a biologically active peptide derivative of modified galectin 8 protein (Gal-8 mutein) polypeptide, a biologically active peptoid compound or peptidic substance derived from modified galectin 8 protein (Gal-8 mutein) polypeptide, or a small organic modified galectin 8 protein (Gal-8 mutein) mimic (including an agonist) of modified galectin 8 protein (Gal-8 mutein) activity. The modified galectin 8 protein (Gal-8 mutein) polypeptide can be a biologically active modified galectin 8 protein (Gal-8 mutein) polypeptide such as a modified galectin 8 protein (Gal-8 mutein) polypeptide variant, a modified galectin 8 protein (Gal-8 mutein) polypeptide derivative, a mutant polypeptide derived from the modified galectin 8 protein (Gal-8 mutein) polypeptide, or a truncated modified galectin 8 protein (Gal-8 mutein) polypeptide. The polynucleotide encoding a modified galectin 8 protein (Gal-8 mutein) polypeptide can be a polynucleotide sequence encoding modified galectin 8 protein (Gal-8 mutein) polypeptide with both full length N-terminal CRD and full length C-terminal CRD of wild type galectin 8, a sequence encoding a biologically active portion of modified galectin 8 protein (Gal-8 mutein) polypeptide, a sequence encoding a biologically active peptide derived from modified galectin 8 protein (Gal-8 mutein) polypeptide, a sequence encoding a soluble modified galectin 8 protein (Gal-8 mutein) polypeptide, etc. Another embodiment of the invention is a composition having a gene delivery vehicle capable of expressing a polynucleotide sequence encoding a modified galectin 8 protein (Gal-8 mutein) polypeptide.

In the present invention, utilization of "gene recombination techniques" allows not only construction, acquisition, isolation, and sequencing of targeted nucleic acid molecules (polynucleotides), proteins (peptides and fragments thereof), but also creation and production of recombinant constructs thereof. Gene recombination techniques (including recombinant DNA techniques) as can be used herein include those known in the art, and can be carried out by the methods described in, for example, J. Sambrook, E. F. Fritsch & T. Maniatis, "Molecular Cloning: A Laboratory Manual (2nd edition)", Cold Spring Harbor Laboratory Press, Cold Spring Harbor, New York (1989); D. M. Glover et al. ed., "DNA Cloning", 2nd ed., Vol. 1 to 4, (The Practical Approach Series), IRL Press, Oxford University Press (1995); The Japanese Biochemical Society (JBS) ed., "Zoku-Seikagaku Jikken Koza 1, Idenshi Kenkyu-Hou II", Tokyo Kagaku Dozin Co. Ltd., Japan, (1986); JBS ed., "Shin-Seikagaku Jikken Koza 2, Kakusan III (Recombinant DNA technique)", Tokyo Kagaku Dozin Co. Ltd., Japan, (1992); M. J. Gait (Ed), Oligonucleotide Synthesis, IRL Press (1984); B. D. Hames and S. J. Higgins (Ed), Nucleic Acid Hybridization, A Practical Approach, IRL Press Ltd., Oxford, UK (1985); B. D. Hames and S. J. Higgins (Ed), Transcription and Translation: A Practical Approach (Practical Approach Series), IRL Press Ltd., Oxford, UK (1984); B. Perbal, A Practical Guide to Molecular Cloning (2nd Edition), John Wiley & Sons, New York (1988); J. H. Miller and M. P. Calos (Ed), Gene Transfer Vectors for Mammalian Cells, Cold Spring Harbor Laboratory, Cold Spring Harbor, New York (1987); R. J. Mayer and J. H. Walker (Ed), Immunochemical Methods in Cell and Molecular Biology, Academic Press, (1987); R. K. Scopes et al. (Ed), Protein Purification: Principles and Practice (2nd Edition, 1987 & 3rd Edition, 1993), Springer-Verlag, N.Y.; D. M. Weir and C. C. Blackwell (Ed), Handbook of Experimental Immunology, Vol. 1, 2, 3 and 4, Blackwell Scientific Publications, Oxford, (1986); L. A. Herzenberg et al. (Ed), Weir's Handbook of Experimental Immunology, Vol. 1, 2, 3 and 4, Blackwell Science Ltd. (1997); R. W. Ellis (Ed), Vaccines - new approaches to immunological problems, Butterworth-Heinemann, London (1992); R. Wu ed., "Methods in Enzymology", Vol. 68 (Recombinant DNA), Academic Press, New York (1980); R. Wu et al. ed., "Methods in Enzymology", Vol. 100 (Recombinant DNA, Part B) & 101 (Recombinant DNA, Part C), Academic Press, New York (1983); R. Wu et al. ed., "Methods in Enzymology", Vol. 153 (Recombinant DNA, Part D), 154 (Recombinant DNA, Part E) & 155 (Recombinant DNA, Part F), Academic Press, New York (1987); J. H. Miller ed., "Methods in Enzymology", Vol. 204, Academic Press, New York (1991); R. Wu et al. ed., "Methods in Enzymology", Vol. 218, Academic Press, New York (1993); S. Weissman (ed.), "Methods in Enzymology", Vol. 303, Academic Press, New York (1999); J. C. Glorioso et al. (ed.), "Methods in Enzymology", Vol. 306, Academic Press, New York (1999), etc., or by methods described in the references quoted therein or substantially equivalent methods thereto or modified methods thereof, the disclosures of which are incorporated herein by reference (hereinafter, all such techniques or methods shall be referred to as "gene recombination techniques").

As used herein, the term "homology" or "homologous" means the quantity (or number), in terms of identity, which can be obtained by determining that corresponding amino acid residues or corresponding nucleotide bases are matched each other between two chains in polypeptide sequences (or amino acid sequences) or polynucleotide sequences (or nucleotide sequences) when amino acid residues or nucleotide bases constituting the chain are compared one another between the two chains and it also means the level of sequence correlation in terms of similarity between two polypeptide sequences or two polynucleotide sequences. The homology can be easily calculated. Various methods for measuring the homology between two polynucleotide sequences or polypeptide sequences have been known and the term "homology" (sometimes called "identity") has been well known to the persons skilled in the art (for example, Lesk, A. M. (Ed.), Computational Molecular Biology, Oxford University Press, New York, (1988); Smith, D. W. (Ed.), Biocomputing: Informatics and Genome Projects, Academic Press, New York, (1993); Grifin, A. M. & Grifin, H. G. (Ed.), Computer Analysis of Sequence Data: Part I, Human Press, New Jersey, (1994); von Heinje, G., Sequence Analysis in Molecular Biology, Academic Press, New York, (1987); Gribskov, M. & Devereux, J. (Ed.), Sequence Analysis Primer, M-Stockton Press, New York, (1991), etc.). Generic techniques for determining the homology between two strands include those disclosed in Martin, J. Bishop (Ed.), Guide to Huge Computers, Academic Press, San Diego, (1994); Carillo, H. & Lipman, D., SIAM J. Applied Math., 48: 1073 (1988), etc., but are not limited to. Preferred methods for measuring the homology include those which are designed so as to obtain the part of the highest fitting relation between the two sequences tested. An example of such methods is a technique which is constructed as a computer program. Preferred computer programming methods include a GCG program package (Devereux, J. et al., Nucleic Acids Research, 12(1): 387 (1984)), BLASTP, BLASTN, FASTA (Atschul, S. F. et al., J. Mol. Biol., 215: 403 (1990)), etc., but are not limited to. For such methods, those known in the art may be employed.

The term "polymerase chain reaction" or "PCR" used herein usually refers to techniques described in US Patent No. 4,683,195 and other documents. For example, the PCR is an in vitro method for the enzymatic amplification of desired specific nucleotide sequences. In general, the PCR includes repetitive series of cycles wherein a primer elongation synthesis is constructed using two oligonucleotide primers capable of preferentially hybridizing with a template nucleic acid. Typically, the primers used in PCR may include those which are complementary to the internal nucleotide sequence of interest in the template. For example, preferable primer pairs as used herein may be those which are complementary to both ends of said nucleotide sequence to be amplified, or flanking regions adjacent to said nucleotide sequence. It is preferable to select a 5'-terminal primer such that at least an initiation codon is contained or the amplification can be performed including the initiation codon, and to select a 3'-terminal primer such that at least a stop codon is contained or the amplification can be performed including the stop codon. The primers include oligonucleotides made up of preferably 5 or more nucleotide bases, more preferably 10 or more nucleotide bases, and still preferably 18 to 25 nucleotide bases.

The PCR reactions can be carried out by methods known in the art or substantially equivalent methods thereto and modified methods thereof. For example, the PCR can be performed according to methods described in R. Saiki, et al., Science, 230: 1350, 1985; R. Saiki, et al., Science, 239: 487, 1988 ; H. A. Erlich ed., PCR Technology, Stockton Press, 1989; D. M. Glover et al. ed., "DNA Cloning", 2nd ed., Vol. 1, (The Practical Approach Series), IRL Press, Oxford University Press (1995); M. A. Innis et al. ed., "PCR Protocols: a guide to methods and applications", Academic Press, New York (1990)) ; M. J. McPherson, P. Quirke and G. R. Taylor (Ed.), PCR: a practical approach, IRL Press, Oxford (1991); M. A. Frohman et al., Proc. Natl. Acad. Sci. USA, 85, 8998 to 9002 (1988) and other documents, and modified methods or variants thereof. The PCR methods can also be performed using commercially available kits suitable therefor, and can also be carried out according to protocols disclosed by manufacturers or distributors of the kits.

In a representative case, the PCR is performed for example, using a template (e.g., DNA synthesized using mRNA as a template; 1st strand DNA) and primers synthesized according to designs on said gene, in admixture with a 10X reaction buffer (contained in a Taq DNA polymerase kit), dNTPs (deoxyribonucleoside triphosphates; dATP, dGTP, dCTP and dTTP mix), Taq DNA polymerase and deionized distilled water. The mixture is subjected to 25 to 60 cycles of amplification using an automated thermal cycler such as GeneAmp^{®} 2400 PCR system (Perkin-Elmer/Cetus) under general PCR cycle conditions. The number of amplification cycles can be suitably set to an appropriate value depending on purposes. The PCR cycle includes, for example, denaturation at 90 to 95°C for 5 to 100 sec, annealing at 40 to 60°C for 5 to 150 sec and extension at 65 to 75°C for 30 to 300 sec, and preferably denaturation at 94°C for 15 sec, annealing at 58°C for 15 sec and extension at 72°C for 45 sec. For the annealing temperature and reaction time, an appropriate value is suitably selected by experimentation. For the denaturation and extension time, an appropriate value suitably varies according to the strand length of expected PCR products. In general, the annealing reaction time preferably varies depending on the Tm value of primer-template DNA hybrids. The time period of extension is usually set with the aim of getting about 1 min per 1000 bp in strand length, but it may be possible to select a shorter time period in some cases.

The term "oligonucleotide(s)" used herein refers to a relatively short single-stranded polynucleotide or double-stranded polynucleotides, or preferably polydeoxynucleotide(s). They can be chemically synthesized by known methods as described in Angew. Chem. Int. Ed. Engl., Vol. 28, pp.716 to 734 (1989), including phosphotriester, phosphodiester, phosphite, phosphoramidite, phosphonate methods, and the like. It has been typically known that the synthesis can be conveniently carried out on modified solid supports. For example, the synthesis can be carried out using an automated synthesizer and such a synthesizer is commercially available. The oligonucleotide may contain one or more modified nucleotide bases. For example, it may contain a nucleotide base which does not naturally occur, such as inosine, or a tritylated nucleotide base. In some cases, they may contain one or more nucleotide bases tagged with a marker.

The target nucleic acid molecules (polynucleotides) can be identified by adaptations of hybridization techniques. The hybridization may be carried out according to methods as described in documents disclosing the aforementioned "gene recombination techniques", or substantially equivalent methods and modifications thereof. For instance, the hybridization is achieved by transferring a sample containing a nucleic acid such as DNA onto a carrier including a membrane such as a nylon filter, as required, optionally followed by denaturation, fixation, washing, etc., and then reacting the transfers on the carrier (e.g., membrane), with labeled DNA probe fragments which are, as required, optionally denatured in a hybridization buffer.

The hybridization operations can be ordinarily conducted at about 35 to about 80°C, more preferably about 50 to about 65°C, for about 15 min to about 36 hours, more preferably about 1 to about 24 hours, but optimal hybridization conditions may be suitably selected. For example, the hybridization is carried out at about 55°C for about 18 hours. The hybridization buffers can be selected from those customarily used in the art. Examples of the hybridization buffers are Rapid hybridization buffer (Amersham), etc. The denaturation of carriers (e.g., membranes, etc.) with transfers includes techniques using an alkali denaturing solution. It is preferable to treat the carrier with a neutralizing solution and a buffer solution after the denaturation. The carrier fixation (e.g., membrane fixation) is usually achieved by baking at about 40 to about 100°C, more preferably about 70 to about 90°C, for about 15 min to about 24 hours, more preferably about 1 to about 4 hours, but desired fixation conditions may be suitably selected. For example, the fixation is carried out by baking at about 80°C for about 2 hours. The washing of carriers (e.g., membranes) with transfers can be performed with washing solutions customarily used in the art, such as 50mM Tris-HCl buffer, pH8.0, containing 1M NaCl, 1mM EDTA and 0.1% sodium dodecyl sulfate (SDS). The carriers including membranes can be selected from those customarily used in the art. Examples of such carriers include nylon filters.

The alkaline denaturing solution, neutralizing solution and buffer solution can be selected from those conventionally used in the art. The alkaline denaturing solution may include, for example, solutions containing 0.5M NaOH and 1.5M NaCl, etc. The neutralizing solution may include, for example, 0.5M Tris-HCl buffers, pH8.0, containing 1.5M NaCl, etc. The buffer solution may include, for example, 2×SSPE (0.36M NaCl, 20mM NaH₂PO₄ and 2mM EDTA), etc. As required, prior to hybridization, it is desirable that carriers (e.g., membranes) with transfers are optionally prehybridized for the prevention of non-specific hybridization. For the prehybridization, the sample is dipped, for example, in a solution for prehybridization [50% formamide, 5×Denhardt's solution (0.2% bovine serum albumin and 0.2% polyvinylpyrrolidone), 5×SSPE, 0.1% SDS, and 100 µg/ml thermally denatured salmon sperm DNA] and the like, and reacted at about 35°C to about 50°C, preferably about 42°C, for about 4 to about 24 hours, preferably about 6 to about 8 hours. These conditions can be determined by those of skill in the art with suitably repeated experiments and more preferred conditions would be selected. Labeled probe DNA fragments used in hybridization can be denatured, for example, under heating conditions at about 70 to 100°C, preferably about 100°C, for about 1 to 60 minutes, preferably about 5 minutes, etc. The hybridization is carried out by well known techniques per se in the art or according to methods analogous thereto. As used herein, the stringent conditions refer to, for example, those equivalent to hybridization in about 15 to 50 mM, preferably about 19 to 40 mM, and more preferably about 19 to 20 mM, with regard to Na ion concentration, at about 35 to 85°C, preferably about 50 to 70°C, and more preferably about 60 to 65°C with regard to temperature.

After the hybridization is completed, the carriers (such as filters) are washed extensively to remove labeled probes except the labeled probe DNA fragments which specifically hybridize. Thereafter, detections are done. The carrier (filter) washing process may be performed by a method suitably selected from techniques used in the art. For example, the washing is carried out in 0.5XSSC solution (XSSC = 0.15M NaCl, 15mM citric acid) containing 0.1% SDS. The hybridized nucleic acids can be detected representatively by autoradiography, but the detection may be performed by a method suitably selected from techniques used in the art. A nucleic acid band corresponding to the detected signal is suspended in a suitable buffer solution such as SM solution (50mM Tris-HCl buffer, pH7.5, containing 100mM NaCl and 10mM MgSO₄). After the nucleic acid suspension is diluted to a suitable level, target nucleic acids can be isolated and purified. Further, the nucleic acids can be subjected to amplification. The term "high homology" as used herein may refer to, though it depends on the sequence length of the targets, for example, 50% or higher, further 60% or higher, preferably 70% or higher, still preferably 80% or higher, in a particular case 95% or higher and most preferably 97% or higher homology. The "nucleotide sequence with the same efficacy" or "equivalently effective nucleotide sequence" includes, for example, those which hybridize with any of those containing the sequence of interest under stringent conditions. Examples of such nucleotide sequences are those which not only hybridize with a nucleotide sequence with 5 or more contiguous nucleotides, preferably 10 or more contiguous nucleotides, more preferably 15 or more contiguous nucleotides, or further preferably 20 or more contiguous nucleotides, selected from said nucleotide sequence, but also code for a substantially equivalent amino acid sequence to said polypeptide. The nucleic acid molecules may also be chemically synthesized. In such cases, fragments may be chemically synthesized and coupled together with enzymes.

Screening treatments can be repeated plural times with hybridization techniques for target nucleic acid molecules from nucleic acid samples including gene libraries, cDNA libraries, and others. Utilizable cDNA libraries are cloned human-derived ones including, for example, cDNA libraries of various human-derived tissues, cultured human cells, or human cell lines (in particular, human body parts, human tissues and cells such as kidney, brain, corpus peale, posterior pituitary gland, nerve cells, retina, retinal blood vessel cells, retinal nerve cells, thymus, blood vessel, endothelial cells, vascular smooth muscle cells, blood cells, macrophages, lymphocytes, testis, ovary, uterus, intestine, heart, liver, pancreas, small intestine, large intestine (including colon and rectum), gingiva-related cells, skin-related cells, glomerular cells, renal tubular cells, and connective tissue cells; various tumor tissues, and cancer cells; and other sources). Further, the cDNA library used as a template may be directly selected from commercially available cDNA libraries derived from a variety of tissues. Examples of the commercially available cDNA libraries are those commercially distributed or delivered by Stratagene (US), Invitrogen (US), Clontech (US), and other distributors. In typical embodiments, the utilizable products include gene libraries generated from human tissues and cells, such as human P1 artificial chromosome genomic libraries (Human Genome Mapping Resource Center, US), and human tissue cDNA libraries (e.g., available from Clontech, US). The screening with probes can be done using human genomic DNA libraries or human-derived cDNA libraries constructed from various human tissues or culture cell lines and other resources. The probe, etc. may be labeled, with a radioactive isotope, using a commercially available labeling kit, such as the Random Prime DNA Labeling Kit (Boehringer Mannheim), etc. For example, a random priming kit (Pharmacia LKB, Uppsala), etc. may be used to label the probe DNA with [α-³²P]dCTP (Amersham), etc. and thus provide a probe with radioactivity.

Phage particles, recombinant plasmids, recombinant vectors and others, containing the target nucleic acid molecules, can be isolated and purified by customary techniques used in the art. For instance, they are obtained by glycerol gradient ultracentrifugation (Molecular Cloning, a laboratory manual, ed. T. Maniatis, Cold Spring Harbor Laboratory, 2nd ed. 78, 1989), electrophoresis and other isolation/purification techniques. DNA can be isolated and purified from phage particles and the like by a member selected from customary techniques used in the art. For instance, the resulting phages are suspended in TM solution (50mM Tris-HCl buffer, pH7.8, containing 10mM MgSO₄), etc., and treated with DNase I and RNase A, etc., followed by addition of a Proteinase K mixture solution (20mM EDTA, 50 µg/ml Proteinase K and 0.5% SDS). The resultant mixture is incubated at about 65°C for 1 hr., subjected to phenol extraction and then to diethyl ether extraction, followed by precipitation with ethanol to form DNA pellets. Next, the resultant DNA is washed with 70% ethanol, dried and dissolved in TE solution (10mM Tris-HC1 buffer, pH8.0, containing 10mM EDTA). A large amount of target DNA can be obtained by subcloning, etc. For example, the subcloning can be performed with plasmid vectors, etc. in host E. coli, etc. The DNA thus subcloned can also be isolated and purified by techniques including phenol extraction, ethanol precipitation, etc. in the same manner as aforementioned.

The resultant nucleic acid molecules (including DNA) such as PCR products are typically herein subjected to electrophoresis on 1 to 2% agarose gels. Specific bands are cut out from the gel, and DNA is extracted with a commercially available kit, e.g., Gene clean kit (Bio 101) and the like. The extracted DNA is cleaved with appropriate restriction enzymes and purified if necessary. Further, the 5'-end is, if necessary, phosphorylated with T4 polynucleotide kinase, etc. and subsequently the DNA is ligated into an appropriate plasmid vector including a pUC vector system such as pUC18, and transformed into suitable competent cells. The cloned PCR products are sequenced and analyzed. Commercially available plasmid vectors such as p-Direct (Clontech), pCR-Script^{®} SK(+) (Stratagene), pGEM-T (Promega), and pAmp^{®} (Gibco-BRL) are useful for cloning of the PCR products. Transformation (transfection) of host cells can be carried out by methods known in the art such as the calcium method, the rubidium/calcium method, the calcium/manganese method, the TFB high efficiency method, the FSB frozen competent cell method, the rapid colony method, electroporation and a member selected from methods known in the art and substantial equivalents thereto (D. Hanahan, J. Mol. Biol., 166: 557, 1983, etc.). Reverse transcription PCR (polymerase chain reaction coupled reverse transcription; RT-PCR) and RACE (rapid amplification of cDNA ends) can be applied to isolate the target DNA. RACE can be carried out according to the methods, for example, described in M. A. Innis et al. ed., "PCR Protocols" (M. A. Frohman, "a guide to methods and applications"), pp.28 to 38, Academic Press, New York (1990), etc.

DNA of interest can be cloned depending on necessity. Suitable vectors for cloning DNA include plasmids, λ phages, cosmids, P1 phage, F element, YAC and others, and are preferably vectors derived from λ phages, such as Charon 4A, Charon 21A, λgt10, λgt11, λDASHII, λ FIXII, λEMBL3, and λZAPII^{®} (Stratagene). The resultant DNA can be incorporated into an appropriate vector such as plasmid pEX, pMAMneo, and pKG5, as described in detail below, and can be expressed in appropriate host cells, e.g., E. coli, yeast, CHO cells, COS cells and others as described in detail below. The DNA fragments can be introduced into animal cells as intact molecules or appropriate control sequence-added DNA fragments or after incorporated into an appropriate vector. Thus, transgenic animals which express the given gene can be produced. The animals include mammalian animals, and include, for example, mice, rats, rabbits, guinea pigs, cattle etc. Preferably, the transgenic animal can be produced by introducing the DNA fragments into fertilized eggs of an animal such as a mouse. Targeted gene products are verified using suitable animal cells, such as 293T cells and COS-1 cells, transfected with said foreign gene.

The methods for transferring foreign genes into mammal animal cells may be practicable ones known in the art or substantially similar techniques thereto, The method may include, for example, the calcium phosphate method (e.g., F. L. Graham et al., Virology, 52: 456, 1973, etc.), the DEAE-dextran method (e.g., D. Warden et al., J. Gen. Virol., 3: 371, 1968, etc.), electroporation (e.g., E. Neumann et al., EMBO J, 1: 841, 1982, etc.), microinjection, the liposome method, virus infection, the phage particle method and others. The gene products produced in the animal cells transfected with the given gene in such ways can also be analyzed.

Any plasmid into which the target gene and others (DNA obtainable in the present invention and the like) are incorporated may be used as long as said DNA can be expressed in host cells conventionally used in genetic engineering techniques (such as prokaryotic host cells including Escherichia coli, Bacillus subtilis, etc. and eukaryotic host cells including yeast cells, CHO cells, COS cells, and insect host cells such as Sf21. It goes without saying that it is possible to use those selected from attachments and reagents in commercially available kits. In such plasmid sequences, it is possible, for example, to contain modified codons suitable for expressing the cloned DNA in selected host cells or to construct restriction enzyme sites. It is also possible to contain control sequences, enhancer sequences, and other sequences for facilitating the expression of the target gene; linkers, adaptors and others, useful for ligating the target gene; effective sequences useful in controlling resistance to antibiotics or in controlling metabolism or in selection (including those coding for hybrid proteins and fusion proteins); and the like. Preferably, suitable promoters may be used. For example, such promoters may include tryptophan promoter (trp), lactose promoter (lac), tryptophan-lactose promoter (tac), lipoprotein promoter (lpp), λ phage P_{L} promoter, etc. in the case of plasmids where hosts are E. coli; SV40 late promoter, MMTV LTR promoter, RSV LTR promoter, CMV promoter, SRα promoter, etc. in the case of plasmids where hosts are animal cells; and GAL1, GAL10 promoters, etc. in the case of plasmids where hosts are yeast cells. It is also possible to use regulation systems such as CYC1, HIS3, ADH1, PGK, PHO5, GAPDH, ADC1, TRP1, URA3, LEU2, EN0, TP1, and AOX1.

An enhancer can be inserted into the vector to facilitate the transcription of DNA encoding the desired polypeptide. Such enhancers include elements of approximately 10 to 100 bp, acting on the promoter to facilitate the transcription and typically having a cis action. A great number of enhancers have been known in mammalian genes such as globin, elastase, albumin, α-fetoprotein, insulin genes and others. Preferably useful representatives of the enhancers are those obtained from eukaryotic infectious viruses, including, for example, an SV40 enhancer (100 to 270 bp) located at the late region of the replication origin, a cytomegalovirus enhancer for the early promoter, a polyoma enhancer located at the late region of the replication origin, an adenovirus enhancer and the like. A signal sequence fitting for the host can be added if necessary. Such signal sequences which can be used herein are well known by those skilled in the art.

The plasmids for E. coli hosts include, for example, pBR322, pUC18, pUC19, pUC118, pUC119, pSP64, pSP65, pTZ-18R/-18U, pTZ-19R/-19U, pGEM-3, pGEM-4, pGEM-3Z, pGEM-4Z, pGEM-5Zf(-), pBluescript KS^{®} (Stratagene 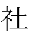 ), etc. The plasmid vectors suitable for the expression in E. coli also include, for example, pAS, pKK223 (Pharmacia), pMC1403, pMC931, pKC30, pRSET-B (Invitrogen), etc. The plasmids for animal host cells include the SV40 vector, polyoma viral vector, vaccinia viral vector, retroviral vector, etc. Examples of such plasmids are pcD, pcD-SRα, CDM8, pCEV4, pME18S, pBC12BI, pSG5 (Stratagene), etc. The plasmids for yeast host cells include YIp, YEp, YRp, YCp type vectors and others. Examples of such plasmids are pGPD-2, etc. The E. coli host cells include those derived from the E. coli K12 strain or the E. coli B834 strain. Examples of the E. coli host cells are NM533, XL1-Blue, C600, DH1, DH5, DH11S, DH12S, DH5 α, DH10B, HB101, MC1061, JM109, STBL2, etc. for the E. coli K12 strain, and BL21(DE3)/pLYsS, etc. for the E. coli B834 strain. Examples of bacterial expression systems can be seen in the following documents: Chang et al., Nature (1978) 275: 615; Goeddel et al., Nature (1979) 281: 544; Goeddel et al., Nucleic Acid Res., (1980) 8: 4057; EP 36,776, U.S. Patent No. 4,551,433; deBoer et al., Proc. Natl. Acad. Sci. USA (1983) 80: 21 to 25; Siebenlist et al., Cell (1980) 20: 269, etc. The yeast host cells include, for example, Saccharomyces cerevisiae, Schizosaccharomyces prombe, Pichia pastoris, Kluyveromyces cells, Candida, Trichoderma reesia and the other yeast cells. Examples of yeast expression systems can be seen in the following documents: Hinnen et al., Proc. Natl. Acad. Sci. USA (1978) 75: 1929; Ito et al., J. Bacteriol. (1983) 153: 163; Kurtz et al., Mol. Cell. Biol. (1986) 6: 142; Kunze et al., J. Basic Microbiol. (1985) 25: 141; Gleeson et al., J. Gen. Microbiol. (1986) 132: 3459; Roggenkamp et al., Mol. Gen. Genet (1986) 202: 302; Das et al., J. Bacteriol. (1984) 158: 1165; De Louvencourt et al., J. Bacteriol. (1983) 154: 737; Van den Berg et al., Bio/Technology (1990) 8: 135; Kunze et al., J. Basic Micr Biol. (1985) 25: 141; Cregg et al., Mol. Cell. Biol. (1985) 5: 3376; U.S. Patent Nos. 4,837,148 & 4,929,555; Beach and Nurse, Nature (1981) 300: 706; Davidow et al., Curr. Genet. (1985) 10: 380; Gaillardin et al., Curr. Genet. (1985) 10: 49; Ballance et al., Biochem. Biophys. Res. Commun. (1983) 112: 284 to 289; Tilburn et al., Gene (1983) 26: 205 to 221; Yalton et al., Proc. Natl. Acad. Sci. USA (1984) 81: 1470 to 1474; Kelly and Hynes, EMBO J., (1985) 4: 475479; EP 244,234; WO 91/00357, etc.

The host cells which are animal cells include, for example, African grivet fibroblast-derived COS-7 cells, COS-1 cells, CV-1 cells, human renal cell-derived 293 cells, human epidermal cell-derived A431 cells, human colon cell-derived 205 cells, murine fibroblast-derived COP cells, MOP cells, WOP cells, Chinese hamster cell-derived CHO cells, CHO DHFR cells, human HeLa cells, murine cell-derived C127 cells, murine cell-derived NIH 3T3 cells, murine L cells, 9BHK, HL60, U937, HaK, Jurkat cells, other transformed cell lines, normal diploid cells, cell lines induced from in vitro primary cultured tissue, etc. Techniques for expressing exogenous DNA in mammalian host cells can be seen in the following documents: Dijkema et al., EMBO J. (1985) 4: 761; Gorman et al., Proc. Natl. Acad. Sci. USA (1982b) 79: 6777; Boshart et al., Cell (1985) 41: 521; U.S. Patent No. 4,399,216; Ham and Wallace, Methods in Enzymology (1979) 58: 44; Barnes and Sato, Anal. Biochem. (1980) 102: 255; U.S. Patent Nos. 4,767,704; 4,657,866; 4,927,762; 4,560,655; WO 90/103430; WO 87/00195; U.S. Patent No. RE 30,985, etc. Insect cells used include Spodoptera frugiperda (caterpillar), Aedes aegypti (mosquito), Aedes albopictus (mosquito), Drosophila melanogaster (fruitfly), silk worm larva or cultured cells (e.g., BM-N cells), in combination with vectors, silk worm (Bombyx mori) nuclear polyhedrosis virus, those derived therefrom or other suitable ones (for example, Luckow et al., Bio/Technology, 6, 47 to 55 (1988); Setlow, J. K. et al. (eds.), Genetic Engineering, Vol. 8, pp.277 to 279, Plenum Publishing, 1986; Maeda et al., Nature, 315, pp.592 to 594 (1985)). Methods of expressing exogenous DNA in insects can be seen in the following documents: U.S. Patent No. 4,745,051; Friesen et al. (1986), "The Regulation of Baculovirus Gene Expression", The Molecular Biology of Baculoviruses (W. Doerfler (Ed)); EP 127,839; EP 155,476; Vlak et al., J. Gen. Virol., (1988) 69: 765 to 776; Miller et al., Ann. Rev. Microbiol. (1988) 42: 177; Carbonell et al., Gene (1988) 73: 409; Maeda et al., Nature, (1985) 315: 592 to 594; Lebacq-Verheyden et al., Mol. Cell. Biol. (1988) 8: 3129; Smith et al., Proc. Natl. Acad. Sci. USA, (1985) 82: 8404; Miyajima et al., Gene (1987) 58: 273; Martin et al., DNA (1988) 7: 99, etc. Numerous baculoviral strains and variants and corresponding permissive insect host cells from hosts are described in Luckow et al., Bio/Technology (1988) 6: 47 to 55; Miller et al., Generic Engineering (Setlow, J. K. et al. (Ed)) Vol. 8 (Plenum Publishing, 1986) pp. 277 to 279; Maeda et al., Nature (1985) 315: 592 to 594, etc.

With utilizing Agrobacterium tumefaciens etc., it is possible to use plant cells as the host cells, which have been widely known along with vectors suitable therefor in the art. In the gene engineering techniques of the present invention, it is possible to use restriction enzymes, reverse transcriptases known and widely used in the art, DNA-modifying enzymes, DNase, DNA polymerases, terminal nucleotidyltransferases, DNA ligases and the like to modify or convert DNA into a structure suitable for cloning the DNA fragment. For example, restriction enzymes include those described in, for example, R. J. Roberts, Nucleic Acids Res., 13: r165, 1985; S. Linn et al. ed. Nucleases, p. 109, Cold Spring Harbor Lab., Cold Spring Harbor, New York, 1982; R. J. Roberts, D. Macelis, Nucleic Acids Res., 19: Suppl. 2077, 1991, etc.

In accordance with the present invention, if necessary, appropriate selection markers are used to select host cells transformed or transfected with the expression vector containing the target polypeptide (protein)-coding polynucleotide. Cloning can be repeated to obtain stable cell clones with high expression levels. For instance, when a dhfr gene is utilized as a selection marker in the transformed or transfected animal host cells (transformants or transfectants), cell clones with higher expression levels can be obtained by culturing with a gradual increase in methotrexate (MTX) concentration to amplify the target polypeptide-coding DNA and selecting resistant cells. The transformants or transfectants can be cultured, under conditions wherein the target polypeptide-coding nucleic acid molecules are expressible, to produce and accumulate target products. The transformants (transfectants) can be cultured in a member selected from media conventionally used in the art. For example, the transformant (transfectant) in which the host is a prokaryotic cell such as Escherichia coli and Bacillus subtilis, yeast or the like can be cultivated suitably in a liquid culture medium. The culture medium may contain carbon sources, nitrogen sources, minerals, and others, necessary for growing the transformant. The carbon source may include glucose, dextrin, soluble starch, sucrose, etc. The nitrogen source may include organic or inorganic substances such as ammonium salts, nitrates, corn steep liquor, peptone, casein, meat extracts, malt extracts, bean-cakes, potato extracts, etc. Examples of the minerals may include calcium chloride, sodium dihydrogen phosphate, magnesium chloride, calcium carbonate, etc. It may also be supplemented with yeast extracts, vitamins, casamino acids, growth-promoting factors, etc. Depending on necessity, the medium may be supplemented with drugs such as 3β-indolyl acrylic acid in order to improve efficiency of the promoter. It is desirable that the pH for culture medium is from about 5 to about 8.

In the case of the Escherichia hosts for example, the cultivation is carried out usually at about 15 to 45°C for about 3 to 75 hours. As required, aeration and stirring may be applied. In case of the transformants in which the hosts are animal cells, the culture medium used may include MEM medium, RPMI 1640 medium, DMEM medium, and others, which are containing, for example, fetal calf serum at about 5 to 20%. It is preferable that the pH is from about 6 to about 8. The cultivation is usually carried out at about 30 to 40°C for about 15 to 72 hours. As required, aeration and stirring may be optionally applied. Although target gene product-expressing transformants can be used without any isolation/purification, they may be utilized in the form of cell homogenates. The target gene products may be isolated for use. To extract the products from the cultured microorganisms or cells, the microorganisms or cells are collected by known methods after the cultivation, next suspended in a suitable buffer solution, disrupted by sonication, lysozyme digestion and/or freeze-thawing, and other treatments, followed by centrifugation or filtration. Thus, crude extracts are obtained. Other conventional extraction or isolation methods can be applied. The buffer solution may contain a protein-denaturing agent such as urea or guanidine hydrochloride or a detergent such as Triton X-100 (trade name) and Tween-80 (trade name). In the case where the target products are secreted into culture media, supernatants are separated from the microorganisms or cells with widely known methods after the cultivation is finished and the resulting supernatants are collected.

The culture supernatants thus obtained and target products contained in extracts can be purified by suitable combinations of widely known per se techniques for separation, isolation and purification. Such widely known techniques are, for example, salting out such as ammonium sulfate precipitation, etc.; gel filtration on Sephadex^{®}, etc.; ion exchange chromatography using carriers having, for example, a diethylaminoethyl or carboxymethyl group, etc.; hydrophobic chromatography using carriers having, for example, a hydrophobic group such as butyl, octyl, or phenyl, etc.; dye-ligand (or chromophore-linked) gel chromatography; electrophoresis; dialysis; ultrafiltration; affinity chromatography; high performance liquid chromatography (HPLC); etc. Preferably, the target products can be isolated, separated and purified by polyacrylamide gel electrophoresis (PAGE), affinity chromatography in which ligands are immobilized. Said ligand may comprise antibodies including monoclonal antibodies, or fragments thereof, capable of recognizing specific targets, lectins, saccharides, one member of a binding pair, and others. Examples of such techniques also include immunoaffinity chromatography, gelatin-agarose affinity chromatography, heparin-agarose chromatography, etc.

In the polypeptides (proteins) of the present invention, amino acid residues contained therein can be modified by chemical techniques. Also, they can be modified and partially degraded to make derivatives thereof using enzymes such as peptidases, e.g., pepsin, chymotrypsin, papain, bromelain, endopeptidase, exopeptidase, etc. In the polypeptides of the present invention, the C-terminal end is typically a carboxyl group (-COOH) or a carboxylate (-COO⁻), but the C-terminal end may be an amide form (-CONH₂) or an ester form (-COOR). For said ester, R includes C₁ to C₆ alkyl groups such as methyl, ethyl, n-propyl, isopropyl and n-butyl, C₃ to C₈ cycloalkyl groups such as cyclopentyl and cyclohexyl, C₆ to C₁₂ aryl groups such as phenyl and α-naphthyl, phenyl-C₁ to C₂ alkyl groups such as benzyl and phenethyl, C₇ to C₁₄ aralkyl groups including α-naphthyl-C₁ to C₂ alkyl groups such as α-naphthylmethyl, as well as a pivaloyloxymethyl group widely used as an oral ester. When the proteins of the present invention have a carboxyl group (or carboxylate) at a site other than the C-terminal end, amidated or esterified carboxyl groups are included in the proteins of the present invention. As the ester in this case, for example, the C-terminal ester and the like described above are used.

The polypeptides (proteins) of the present invention may be those having an N-terminal methionine residue in the above proteins, and further include those in which an amino group of the methionine residue is protected with a protecting group (for example, C₁ to C₆ acyl groups including C₁ to C₅ alkyl-carbonyl groups such as formyl and acetyl), those in which the N-terminus is cleaved in vivo and the resultant glutamyl group is pyroglutamylated, those in which substituents (for example, -OH, -COOH, amino, imidazole, indole, guanidino groups and the like) on side chains of the intramolecular amino acids are protected with appropriate protecting groups (for example, C₁ to C₆ acyl groups such as formyl and acetyl groups), or conjugated proteins (such as so-called glycoproteins) in which saccharide chains are linked.

Further, by relying on the gene nucleotide sequences associated with the present invention, equivalent polypeptides or derivatives thereof wherein each amino acid sequence of the target polypeptides is altered may be produced with conventional genetic engineering techniques. Such alterations include substitution (replacement), deletion, insertion, transfer or addition of one or more amino acid residues, etc. For example, such mutations, conversions and modifications are those described in The Japanese Biochemical Society (JBS) ed., "Zoku-Seikagaku Jikken Koza 1, Idenshi Kenkyu-Hou II", p. 105 (Susumu Hirose), Tokyo Kagaku Dozin Co. Ltd., Japan, (1986); JBS ed., "Shin-Seikagaku Jikken Koza 2, Kakusan III (Recombinant DNA technique)", p. 233 (Susumu Hirose), Tokyo Kagaku Dozin Co. Ltd., Japan, (1992); R. Wu, L. Grossman, ed., "Methods in Enzymology", Vol. 154, p. 350 & p. 367, Academic Press, New York (1987); R. Wu, L. Grossman, ed., "Methods in Enzymology", Vol. 100, p. 457 & p. 468, Academic Press, New York (1983); J. A. Wells et al., Gene, 34: 315, 1985; T. Grundstroem et al., Nucleic Acids Res., 13: 3305, 1985; J. Taylor et al., Nucleic Acids Res., 13: 8765, 1985; R. Wu ed., "Methods in Enzymology", Vol. 155, p. 568, Academic Press, New York (1987) ; A. R. Oliphant et al., Gene, 44: 177, 1986, etc. For example, included are methods such as the site-directed mutagenesis (site specific mutagenesis) utilizing synthetic oligonucleotides or others (Zoller et al., Nucl. Acids Res., 10: 6487, 1987; Carter et al., Nucl. Acids Res., 13: 4331, 1986), the cassette mutagenesis (Wells et al., Gene, 34: 315, 1985), restriction selection mutagenesis (Wells et al., Philos. Trans. R. Soc. London Ser A, 317: 415, 1986), the alanine scanning (Cunningham & Wells, Science, 244: 1081-1085, 1989), PCR mutagenesis, Kunkel method, dNTP[αS] method (Eckstein), the region directed mutagenesis using sulfurous acid and nitrous acid and other techniques.

The polypeptides (proteins) may be expressed as fusion polypeptides (fusion proteins) when produced by gene recombination techniques, and may be converted or processed into those having substantially equivalent biological activity as compared to those which naturally occur in vivo or in vitro. The fusion polypeptide expression system usually used in gene engineering can be applied. Such fusion polypeptides can be purified by an affinity chromatography and the like, taking advantage of their fusion moieties. Such fusion polypeptides include those fused to a histidine tag, or those fused to the amino acid sequence of β-galactosidase (β-gal), maltose-binding protein (MBP), glutathione S-transferase (GST), thioredoxin (TRX), or Cre Recombinase. Similarly, the polypeptide can be added with a tag of heterogeneous epitope, and can be isolated/purified by an immunoaffinity chromatography using an antibody specifically binding to the epitope. In more suitable embodiments, the representatives include poly histidine (poly-His) or polyhistidine-glycine (poly-His-Gly) tags, and epitope tags such as AU5, c-Myc, CruzTag 09, CruzTag 22, CruzTag 41, Glu-Glu, HA, Ha.11, KT3, FLAG (registered trademark, Sigma-Aldrich), Omni-probe, S-probe, T7, Lex A, V5, VP16, GAL4, and VSV-G (Field et al., Molecular and Cellular Biology, 8: pp.2159-2165 (1988); Evan et al., Molecular and Cellular Biology, 5: pp.3610-3616 (1985); Paborsky et al., Protein Engineering, 3(6): pp.547-553 (1990); Hopp et al., BioTechnology, 6: pp.1204-1210 (1988); Martin et al., Science, 255: pp.192-194 (1992); Skinner et al., J. Biol. Chem., 266: pp.15163-15166 (1991); Lutz-Freyermuth et al., Proc. Natl. Acad. Sci. USA, 87: pp.6393-6397 (1990), etc.). Yeast two-hybrid systems are also utilizable.

Besides, the fusion polypeptides can be those tagged with a marker such that they become detectable proteins. In more suitable embodiments, the detectable markers may be Biotin-Avi Tag which is a biotin/streptavidin system, and fluorescent substances. The fluorescent substances include green fluorescent proteins (GFP) derived from luminescent jelly fish such as Aequorea victorea and the like, modified variants thereof (GFP variants) such as EGFP (enhanced-humanized GFP) and rsGFP (red-shift GFP), yellow fluorescent proteins (YFP), green fluorescent proteins (GFP), cyan fluorescent proteins (CFP), blue fluorescent proteins (BFP), GFP derived from Renilla reniformis, and the like (Atsushi Miyawaki ed., Jikken Igaku (Experimental Medicine), Besatsu (suppl.), Postgenome Jidai no Jikken Kouza 3 (GFP and Bioimaging), Yodosha Co., Ltd., 2000). Detection can be carried out using antibodies (including monoclonal antibodies and fragments thereof) which specifically recognize the above fusion tag. The expression and purification of such fusion polypeptides can be performed using commercially available kits suitable for these techniques, and can also be carried out according to protocols as instructed by manufacturers or distributors of the kits.

The resultant proteins (which may include peptides and polypeptides) can be coupled with suitable carrier or solid phases by techniques known in the enzyme immunoassay and others to form solid phased products. Solid-phased proteins and solid-phased peptides are conveniently useful in binding assays and screenings for substances.

Modifications and alterations of the polypeptide or protein structures can be performed in reference to, for example, The Japanese Biochemical Society (JBS) ed., "Shin-Seikagaku Jikken Koza 1, Protein VII, Protein Engineering" Tokyo Kagaku Dozin Co. Ltd., Japan, 1993) using the methods described therein or the methods described in the references quoted therein, and, further, substantially equivalent methods thereto. Their biological activity as described herein below may include immunological activity, for example, antigenicity. The modification and alteration may be deamination, hydroxylation, carboxylation, phosphorylation, sulfation, alkylation such as methylation, acylation such as acetylation, esterification, amidation, ring-opening, cyclization, glycosylation, alteration of contained saccharide chains to different types, increasing or decreasing the number of contained saccharide chains, lipid-binding, substitution to D-amino acid residues, etc. Those methods are known in the art (for example, T. E. Creighton, Proteins: Structure and Molecular Properties, pp. 79 to 86 W.H. Freeman & Co., San Francisco, USA (1983), etc.).

When modified galectin 8 proteins (modified Gal-8 variants) according to the present invention are utilized, screening can be done for compounds, or salts thereof, which promote (agonists) or inhibit (antagonists) the interesting Gal 8-mediated functions such as biological actions (e.g., hemagglutinating actions, inducing actions on the induction of cell adhesion such as neutrophil adhesion, integrin α_{M}-binding actions, proMMP-9 binding actions, promoting actions on the production of active form MMP-9, promoting actions on the production of superoxides, cytotoxic actions, apoptosis-inducible actions, suppressive actions on LPS-induced inflammation, inhibitory actions on the LPS-induced production of TNF-α, IL-12, and/or IFN-γ, suppressive actions on endotoxin shock, malignant cell metastasis-inhibiting actions and the like). This means that screening kits and reagents are contemplated herein. Thus, the present invention provides methods for screening of either (1) a promoting compound (agonist), or a salt thereof, which promotes the predetermined functions exerted by any of galectin 8 proteins (including human galectin 8), peptide fragments thereof, and salts thereof, etc., wherein the function may include Gal 8-mediated biological actions as identified or disclosed herein, or (2) an inhibitory compound (antagonist), or a salt thereof, which inhibits the same functions, which comprises using a disclosed or identified action or activity mediated or owned by a member selected from the group consisting of said galectin 8 proteins (including human galectin 8), peptide fragments thereof, and salts thereof, in connection with a variety of substances.
For example, the screening comprises steps of:
(i) contacting a modified galectin-8 protein (or modified Gal-8 variant), a peptide fragment thereof, a salt thereof , or an equivalent thereof (including a transformant or transfectant which expresses said protein; hereinafter the same) with a suitable test sample, thereby obtaining a first assay;
(ii) incubating the protein of the present invention, a peptide fragment thereof, a salt thereof, or an equivalent thereof, without the test sample of interest, thereby obtaining a second assay; and
(iii) comparing said first assay and said second assay.

In an embodiment of the screening, said biological activities (e.g., activities associated with interactions between each galectin 8 protein and biological components, etc.) are measured and compared.

The screening systems may contain suitable detectable substrates for the convenience of assays. The substrates may be any as long as they are effectively utilizable in assays. For instance, they can be selected from those known to be conventional substrates and preferably include synthesized compounds and other materials. The substrate can be employed without any modification, or preferably after labeling with fluorochromes such as fluorescein, enzymes or radioactive substances.

The test samples include, for example, proteins, peptides, nonpeptidic compounds, synthetic compounds, fermented products, plant extracts, tissue extracts such as animal tissue extracts, cell extracts, etc. Examples of test compounds as used for the test samples may include preferably anti-galectin antibodies, enzyme inhibitors, cytokines, a variety of compounds having inhibitor activity, inter alia synthetic compounds, etc. These compounds can be novel or known to the public. The screening is conducted according to conventional techniques for measuring binding activities or enzyme activities, for example, by referring to known methods in the art. It can also be performed by using various labels, buffers and suitable other reagents, etc. and according to the operations, etc., as described herein for the assays. In the screening, it is possible to treat the peptides used and the like with activators, and to convert their precursors or latent forms into active forms thereof prior to the assay. The assay can usually be performed in buffer without any adverse effect on the reaction, including Tris-HCl buffer, phosphate buffer, etc., for example, at pH about 4 to 10, preferably at pH about 6 to 8. For each of these screenings, by giving technical consideration ordinarily owned by persons skilled in the art to customary conditions and operations for each method, suitable assay systems may be constructed in connection with each of the galectin 8 proteins and polypeptides or peptides having substantially equivalent activity thereto, according to the present invention. With details of those conventional techniques, a variety of reviews, reference books, etc. may be referred to (e.g., Methods in Enzymology, Academic Press, New York, USA).

The activity of the modified galectin 8 protein (Gal-8 mutein) may have the following meaning:

The modified galectin 8 protein (Gal-8 mutein) can be assayed for cytokine, cell proliferation (either inducing or inhibiting) or cell differentiation (either inducing or inhibiting) activity or the ability to induce production of other cytokines in certain cell populations. A number of routine factor dependent cell proliferation assays are known, including, for example, those for cell lines such as 32D, DA2, DA1G, T10, B9, B9/11, BaF3, MC9/G, M+ (preB M+), 2E8, RB5, DA1, 123, T1165, HT2, CTLL2, TF-1, Mo7e, and CMK. Assays for T-cell or thymocyte proliferation include, without limitation, those described in John E. Coligan, Ada M. Kruisbeek, David H. Margulies, Ethan M. Shevach, Warren Strober (Ed.), Current Protocols in Immunology (hereinafter referred to "J. E. Coligan et al. (Ed.), Current Protocols in Immunology, John Wiley & Sons, Inc.") (Chapter 3: "In Vitro assays for Mouse Lymphocyte Function (3.1-3.19)"; Chapter 7: "Immunologic studies in Humans"), John Wiley & Sons, Inc; Takai et al., J. Immunol., 137: 3494-3500 (1986); Bertagnolli et al., J. Immunol., 145: 1706-1712 (1990); Bertagnolli et al., Cellular Immunology, 133: 327-341 (1991); Bertagnolli et al., J. Immunol., 149: 3778-3783 (1992); Bowman et al., J. Immunol., 152: 1756-1761 (1994). Assays for cytokine production and/or proliferation of spleen cells, lymph node cells or thymocytes include, without limitation, those described in J. E. Coligan et al. (Ed.), Current Protocols in Immunology, John Wiley & Sons, Inc. (Chapter 3 "In Vitro assays for Mouse Lymphocyte Function--Proliferative Assays for T Cell Function" and Chapter 6 "Cytokines and Their Cellular Receptors--Measurement of mouse and human Interferon γ"). Assays for proliferation and differentiation of hematopoietic and lymphopoietic cells include, without limitation, those described in J. E. Coligan et al. (Ed.), Current Protocols in Immunology, John Wiley & Sons, Inc. (Chapter 6 "Cytokines and Their Cellular Receptors-Measurement of Human and Murine Interleukin 2 and Interleukin 4; --Measurement of mouse and human interleukin 6; --Measurement of human Interleukin 11; --Measurement of mouse and human Interleukin 9"); deVries et al., J. Exp. Med., 173: 1205-1211 (1991); Moreau et al., Nature, 336: 690-692 (1988); Greenberger et al., Proc. Natl. Acad. Sci. U.S.A., 80: 2931-2938 (1983); and Smith et al., Proc. Natl. Acad. Sci. U.S.A., 83: 1857-1861 (1986). Assays for T-cell clone responses to antigens (which will identify, among others, proteins that affect APC-T cell interactions as well as direct T-cell effects by measuring proliferation and cytokine production) include, without limitation, those described in J. E. Coligan et al. (Ed.), Current Protocols in Immunology, John Wiley & Sons, Inc. (Chapter 3 "In Vitro assays for Mouse Lymphocyte Function"; Chapter 6 "Cytokines and Their Cellular Receptors"; Chapter 7 "Immunologic studies in Humans"); Weinberger et al., Proc. Natl. Acad. Sci. U.S.A., 77: 6091-6095 (1980); Weinberger et al., Eur. J. Immun., 11: 405-411 (1981); Takai et al., J. Immunol., 137: 3494-3500 (1986); and Takai et al., J. Immunol., 140: 508-512 (1988).

The modified galectin 8 protein (Gal-8 mutein) can also be assayed for immune stimulating or immune suppressing activity. Assays can be conducted for whether the modified galectin 8 protein (Gal-8 mutein) is active in the treatment of various immune deficiencies and disorders (including severe combined immunodeficiency (SCID)), e.g., in regulating (up or down) growth and proliferation of T and/or B lymphocytes, as well as effecting the cytolytic activity of NK cells and other cell populations. These immune deficiencies may be genetic or be caused by viral (e.g., HIV) as well as bacterial or fungal infections, or may result from autoimmune disorders. More specifically, they may comprise infectious diseases causes by viral, bacterial, fungal or other infection, including infections by HIV, hepatitis viruses, herpes viruses, mycobacteria, leshmania, malaria and various fungal infections such as candida. Also, cases where a boost to the immune system generally would be indicated, i.e., the treatment of cancer, may be included. The autoimmune disorder includes, for example, connective tissue disease, multiple sclerosis, systemic lupus erythematosus, rheumatoid arthritis, autoimmune pulmonary inflammation, Guillain-Barre syndrome, autoimmune thyroiditis, insulin dependent diabetes mellitus, myasthenia gravis, graft-versus-host disease (GVHD) and autoimmune inflammatory eye disease. The activity of modified galectin 8 protein (Gal-8 mutein) may be measured in the treatment of allergic reactions and conditions, such as asthma or other respiratory problems. Assays may be carried out for other conditions, in which immune suppression is desired (including, for example, asthma and related respiratory conditions).

The modified galectin 8 protein (Gal-8 mutein)-mediated induction of immune responses can be determined in a number of ways. Down regulation may be in the form of inhibiting or blocking an immune response already in progress or may involve preventing the induction of an immune response. The functions of activated T cells may be inhibited by suppressing T cell responses or by inducing specific tolerance in T cells, or both. Immunosuppression of T cell responses is generally an active, non-antigen-specific, process which requires continuous exposure of the T cells to the suppressive agent. Tolerance, which involves inducing non-responsiveness or anergy in T cells, is distinguishable from immunosuppression in that it is generally antigen-specific and persists after exposure to the tolerizing agent has ceased. Operationally, tolerance can be demonstrated by the lack of a T cell response upon reexposure to specific antigen in the absence of the tolerizing agent. Down regulating or preventing one or more antigen functions (including, without limitation, B lymphocyte antigen functions such as B7), e.g., preventing high level lymphokine synthesis by activated T cells, will be useful in situations of tissue, skin and organ transplantation and in graft-versus-host disease (GVHD). For example, blockage of T cell function should result in reduced tissue destruction in tissue transplantation. Typically, in tissue transplants, rejection of the transplant is initiated through its recognition as foreign by T cells, followed by an immune reaction that destroys the transplant. The administration of a molecule which inhibits or blocks interaction of a B7 lymphocyte antigen with its natural ligand(s) on immune cells (such as a soluble, monomeric form of a peptide having B7-2 activity alone or in conjunction with a monomeric form of a peptide having an activity of another B lymphocyte antigen (e.g., B7-1, 37-3) or blocking antibody), prior to transplantation can lead to the binding of the molecule to the natural ligand(s) on the immune cells without transmitting the corresponding costimulatory signal. Blocking B lymphocyte antigen function in this matter prevents cytokine synthesis by immune cells, such as T cells, and thus acts as an immunosuppressant. Moreover, the lack of costimulation may also be sufficient to anergize the T cells, thereby inducing tolerance in a subject. Induction of long-term tolerance by B lymphocyte antigen-blocking reagents may avoid the necessity of repeated administration of these blocking reagents. To achieve sufficient immunosuppression or tolerance in a subject, it may also be necessary to block the function of a combination of B lymphocyte antigens.

The following can be determined in a number of ways: how much does the modified galectin 8 protein (Gal-8 mutein) have one or more of activities or effects, such as hemagglutination, induction of cell adhesion including neutrophil adhesion, integrin α_{M}-binding, proMMP-9 binding, promotion of active form MMP-9 production, promotion of superoxide production, suppression of LPS-induced inflammation, inhibition of LPS-induced TNF-α, IL-12, and/or IFN-γ production, and suppression of endotoxin shock? The modified galectin 8 protein (Gal-8 mutein)-mediated induction of one or more of such activities or effects can also be determined similarly. For apoptosis assays, it is possible to refer to Sei-ichi Tamuma (Ed.), "Saiboukagaku Bessatsu: Jikken Protocol Series, Apoptosis Jikken Protocol" (1st Edition, 2nd Print), Shujunsha Co., Ltd., January 20, 1995 and others, and to use commercially available assay kits.

The efficacy of particular blocking reagents in preventing organ transplant rejection or GVHD can be assessed using animal models that are predictive of efficacy in humans. Examples of appropriate systems which can be used include allogeneic cardiac grafts in rats and xenogeneic pancreatic islet cell grafts in mice, both of which have been used to examine the immunosuppressive effects of CTLA4Ig fusion proteins in vivo as described in Lenschow et al., Science, 257: 789-792 (1992) and Turka et al., Proc. Natl. Acad. Sci. U.S.A., 89: 11102-11105 (1992). In addition, murine models of GVHD (see Paul ed., Fundamental Immunology, Raven Press, New York, pp.846-847 (1989)) can be used to determine the effect of blocking B lymphocyte antigen function in vivo on the development of that disease. Blocking antigen function may also be therapeutically useful for treating autoimmune diseases. Many autoimmune disorders are the result of inappropriate activation of T cells that are reactive against self tissue and which promote the production of cytokines and autoantibodies involved in the pathology of the diseases. Preventing the activation of autoreactive T cells may reduce or eliminate disease symptoms. Administration of reagents which block costimulation of T cells by disrupting receptor:ligand interactions of B lymphocyte antigens can be used to inhibit T cell activation and prevent production of autoantibodies or T cell-derived cytokines which may be involved in the disease process. Additionally, blocking reagents may induce antigen-specific tolerance of autoreactive T cells which could lead to long-term relief from the disease. The efficacy of blocking reagents in preventing or alleviating autoimmune disorders can be determined using a number of well-characterized animal models of human autoimmune diseases. Examples include murine experimental autoimmune encephalitis, systemic lupus erythmatosis in MRL/lpr/lpr mice or NZB hybrid mice, murine autoimmune collagen arthritis, diabetes mellitus in NOD mice and BB rats, and murine experimental myasthenia gravis (see Paul (Ed.), Fundamental Immunology, Raven Press, New York, 840-856 (1989)). Upregulation of an antigen function (preferably a B lymphocyte antigen function), as a means of up regulating immune responses, may also be useful in therapy. Upregulation of immune responses may be in the form of enhancing an existing immune response or eliciting an initial immune response. For example, enhancing an immune response through stimulating B lymphocyte antigen function may be useful in cases of viral infection. In addition, systemic viral diseases such as influenza, the common cold, and encephalitis might be alleviated by the administration of stimulatory forms of B lymphocyte antigens systemically. Upregulation or enhancement of an antigen function (preferably a B lymphocyte antigen function) may also be useful in inducing tumor immunity. For example, after administration of tumor cells (such as, for example, sarcoma, melanoma, lymphoma, leukemia, neuroblastoma and cancer) transfected with a nucleic acid molecule encoding at least one of modified galectin 8 proteins (Gal-8 muteins) to a subject, it is possible to determine whether tumor-specific tolerance is overcome in the subject.

The modified galectin 8 protein (Gal-8 mutein) can be assayed for regulation of hematopoiesis, and additionally examined for whether it is useful in the treatment of myeloid or lymphoid cell deficiencies. Suitable assays for proliferation and differentiation of various hematopoietic lines are widely known in the art and can be employed herein. Assays for embryonic stem cell differentiation (which will identify, among others, proteins that influence embryonic differentiation hematopoiesis) include, without limitation, those described in Johansson et al., Cellular Biology, 15: 141-151 (1995); Keller et al., Molecular and Cellular Biology, 13: 473-486 (1993); McClanahan et al., Blood 81: 2903-2915 (1993). Assays for stem cell survival and differentiation (which will identify, among others, proteins that regulate lympho-hematopoiesis) include, without limitation, those described in Methylcellulose colony forming assays, Freshney, M.G. in "Culture of Hematopoietic Cells", R.I. Freshney et al. eds., pp. 265-268, Wiley-Liss, Inc., New York, NY. 1994; Hirayama et al., Proc. Natl. Acad. Sci. USA 89: 5907-5911, 1992; Primitive hematopoietic colony forming cells with high proliferative potential, McNiece, I.K. and Briddell, R.A. in "Culture of Hematopoietic Cells", R.I. Freshney et al. eds., pp. 23-39, Wiley-Liss, Inc., New York, NY. 1994; Neben et al., Experimental Hematology 22: 353-359, 1994; Cobblestone area forming cell assay, Ploemacher, R.E. in "Culture of Hematopoietic Cells", R.I. Freshney et al. eds., pp. 1-21, Wiley-Liss, Inc., New York, NY. 1994; Long term bone marrow cultures in the presence of stromal cells, Spooncer, E., Dexter, M. and Allen, T. in "Culture of Hematopoietic Cells", R.I. Freshney et al. eds., pp. 163-179, Wiley-Liss, Inc., New York, NY. 1994; and Long term culture initiating cell assay, Sutherland, H.J. in "Culture of Hematopoietic Cells", R.I. Freshney et al. eds., pp. 139-162, Wiley-Liss, Inc., New York, NY. 1994.

The utility of modified galectin 8 proteins (Gal-8 muteins) can be assessed in compositions used for bone, cartilage, tendon, ligament and/or nerve tissue growth or regeneration, as well as for wound healing and tissue repair and replacement, and in the treatment of burns, incisions and ulcers. The ability to induce cartilage and/or bone growth in circumstances where bone is not normally formed, is useful in the healing of bone fractures and cartilage damage or defects in humans and other animals. The modified galectin 8 proteins (Gal-8 muteins) can be assayed for their activity in the treatment of periodontal disease, and in other tooth repair processes. The ability to attract bone-forming cells, stimulate growth of bone-forming cells or induce differentiation of progenitors of bone-forming cells, can also be examined. The modified galectin 8 protein (Gal-8 mutein) may be active in the treatment of osteoporosis or osteoarthritis, such as through stimulation of bone and/or cartilage repair or by blocking inflammation or processes of tissue destruction (collagenase activity, osteoclast activity, etc.) mediated by inflammatory processes. Additionally, it can be assayed for its activity in the healing of tendon or ligament tears, deformities and other tendon or ligament defects in humans and other animals. The modified galectin 8 proteins (Gal-8 muteins) can be assayed to determine whether they are active in proliferation of neural cells and in regeneration of nerve and brain tissue, i.e. in the treatment of central and peripheral nervous system diseases and neuropathies, as well as mechanical and traumatic disorders, which involve degeneration, death or trauma to neural cells or nerve tissue. More specifically, the activity against diseases of the peripheral nervous system, such as peripheral nerve injuries, peripheral neuropathy and localized neuropathies, and central nervous system diseases, such as Alzheimer's disease, Parkinson's disease, Huntington's disease, amyotrophic lateral sclerosis, and Shy-Drager syndrome, will be examined.

The modified galectin 8 proteins (Gal-8 muteins) can be assayed for their integrin-related activity, chemotactic or chemokinetic activity (including the adhesion property) for mammalian cells, including, for example, monocytes, fibroblasts, neutrophils, T-cells, mast cells, eosinophils, epithelial and/or endothelial cells, hemostatic or thrombolytic activity, activity as receptors, receptor ligands or inhibitors or agonists of receptor/ligand interactions, anti-inflammatory activity (including activity of providing a stimulus to cells involved in the inflammatory response), LPS-induced inflammation-suppressing activity, LPS-induced TNF-α, IL-12, and/or IFN-γ production-suppressing activity, endotoxin shock-inhibiting activity, tumor-related activity including anti-tumor activity, one or more of the following additional activities or effects: inhibiting the growth, infection or function of, or killing, infectious factors, including, without limitation, bacteria, viruses, fungi and other parasites; effecting (suppressing or enhancing) bodily characteristics, including, without limitation, height, weight, hair color, eye color, skin, fat to lean ratio or other tissue pigmentation, or organ or body part size or shape (such as, for example, breast augmentation or diminution, change in bone form or shape); effecting biorhythms or caricadic cycles or rhythms; effecting the fertility of male or female subjects; effecting the metabolism, catabolism, anabolism, processing, utilization, storage or elimination of dietary fat, lipid, protein, carbohydrate, vitamins, minerals, co-factors or other nutritional factors or component(s); effecting behavioral characteristics, including, without limitation, appetite, libido, stress, cognition (including cognitive disorders), depression (including depressive disorders) and violent behaviors; providing analgesic effects or other pain reducing effects; promoting differentiation and growth of embryonic stem cells in lineages other than hematopoietic lineages; hormonal or endocrine activity; in the case of enzymes, correcting deficiencies of the enzyme and treating deficiency-related diseases; treatment of hyperproliferative disorders (such as psoriasis); immunoglobulin-like activity (such as the ability to bind antigens or complement); and the ability to act as an antigen in a vaccine composition to raise an immune response against such protein or another material or entity which is cross-reactive with such protein.

The compounds or salts thereof identified or obtained by the screening method or kit according to the present invention are those selected from the aforementioned test compounds, including peptides, proteins, nonpeptidic compounds, synthetic compounds, fermented products, cell extracts, plant extracts, animal tissue extracts, etc. Such compounds are those which enhance (or promote) or inhibit (or suppress) the functions of the proteins and other species according to the present invention. Salts of said compounds are, for example, pharmaceutically acceptable salts thereof, etc. Examples of such salts are those with inorganic bases, with organic bases, with inorganic acids, with organic acids, with basic or acidic amino acids, etc. Preferred examples of the inorganic base salts are alkaline metal salts such as sodium salts, and potassium salts; alkaline earth metal salts such as calcium salts and magnesium salts; aluminum salts, ammonium salts; etc. Preferred examples of the organic base salts are salts with trimethylamine, triethylamine, pyridine, picoline, 2,6-lutidine, ethanolamine, diethanolamine, triethanolamine, cyclohexylamine, dicyclohexylamine, N,N-dibenzylethylenediamine, etc. Preferred examples of the inorganic acid salts are salts with hydrochloric acid, hydrobromic acid, sulfuric acid, phosphoric acid, etc. Preferred examples of the organic acid salts are salts with formic acid, acetic acid, propionic acid, fumaric acid, oxalic acid, tartaric acid, maleic acid, citric acid, succinic acid, malic acid, methanesulfonic acid, benzenesulfonic acid, benzoic acid, etc. Preferred examples of the basic amino acid salts are those of arginine, lysine, ornithine, etc. Preferred examples of the acidic amino acid salts are those of aspartic acid, glutamic acid, etc.

The active components of the present invention [e.g., (a) the modified Gal-8 polypeptides (modified Gal-8 variants), peptide fragments thereof, salts thereof, their related peptides, etc.; (b) the modified Gal-8 variant-coding or its related peptide-coding nucleic acid molecules (including DNA and others), etc.; (c) the compounds, or salts thereof, that control or regulate said interesting activities (or functions) exerted by Gal-8 (the compounds that promote or suppress/inhibit Gal-8 biological activities, including phenomena that they promote or suppress/inhibit Gal-8 protein-dependent hemagglutinating actions, inducing actions on the induction of cell adhesion such as neutrophil adhesion, integrin α_{M}-binding actions, proMMP-9 binding actions, promoting actions on the production of active form MMP-9, promoting actions on the production of superoxides, cytotoxic actions, apoptosis-inducing actions, suppressive actions on LPS-induced inflammation, inhibitory actions on the LPS-induced production of TNF-α, IL-12, and/or IFN-γ, suppressive actions on endotoxin shock, or Gal-8 abilities of exerting desirable efficacies without any adverse effect on normal cells, etc. and degeneration, overproduction, or degradation of tissues or proteins); the compounds, or their salts, that control or regulate said protein production; (d) the compounds identified or characterized by means of the present invention; etc.] can be employed as pharmaceutical agents. The active components can be administered alone or in the form of a pharmaceutical composition or preparation in admixture with any of various pharmaceutically acceptable aids. Preferably, it may be administered in the form of a convenient pharmaceutical composition or formulation suitable for oral, topical, parenteral application, or the like. Any of dosage forms (including those for inhalation and rectal administration) may be selected depending on purpose.

The active components of the present invention can be used in combination with any of various drugs, including antitumor drugs (antineoplastic drugs), tumor metastasis-inhibitors, inhibitors for thrombogenesis, therapeutic drugs for joint destruction, analgesics, anti-inflammatory drugs, immunoregulators (or immunomodulators) and/or immunosuppressants, which can be employed as not being restricted to particular species as long as they serve effectively or advantageously. For instance, they can be optionally selected from those known in the art.

The parenteral administration includes topical, percutaneous, intravenous, intramuscular, subcutaneous, intracutaneous, and intraperitoneal routes. It is also possible to apply the drug directly to affected sites, and, in a certain case, the direct application is suitable. Preferably mammal animals including human can receive the drug orally or parenterally (e.g., intracellularly, intratissularly, intravenously, intramuscularly, subcutaneously, intracutaneously, intraperitoneally, intrapleurally, intraspinally, by instillation, enterally, per rectum, by instillation into the ear, eye, or nose by swabbing or application on the teeth, skin or mucosa, etc.). Specific dosage forms of the pharmaceutical preparations and formulations include pharmaceutical solutions, pharmaceutical dispersions, semisolid preparations, particulate preparations, shaped preparations, extractives, etc. Examples of the dosage forms are tablets, coated tablets, sugar coated tablets, pills, troches, hard capsules, soft capsules, microcapsules, implants, powders, pulvis, granules, fine granules, injections, liquids and solutions, elixirs, emulsions, irrigations, syrups, mixtures, suspensions, liniments, lotions, aerosols, sprays, inhalations, nebula, ointments, plasters, patches, pastes, cataplasms, creams, oleates, suppositories (e.g., rectal suppositories), tinctures, dermatologic waters, ophthalmic solutions, collunariums, auristillae, paints, transfusions, powders for injection solutions, lyophilized preparations, conditioned gels, etc.

The pharmaceutical compositions can be formulated in accordance with conventional techniques. For example, the pharmaceutical composition or formulation may comprise at least one of said compounds (active components including proteins) of the present invention or a salt alone or in admixture with physiologically allowable carriers, pharmaceutically acceptable carriers, adjuvants, vehicles, excipients, diluents, etc. The compound (active component or protein) of the present invention or a salt thereof is usually admixed with a single member selected from the group consisting of physiologically allowable carriers, pharmaceutically acceptable carriers, adjuvants, vehicles, excipients, diluents, flavoring agents, perfuming agents, sweetening agents, expanders, antiseptics, stabilizers, binders, pH regulators, buffering agents, detergents (surfactants), bases, solvents, fillers, bulking agents, solution adjuvants, solubilizers, tonicity agents, emulsifiers, suspending agents, dispersers, viscosity-increasing agents, thickening agents, gelling agents, stiffening agents, absorbents, adhesives, elastomers, plasticizers, disintegrants, aerosol propellants, preservatives, antioxidants, opacifying agents, humectants, emollients, charge protectors, soothing agents, etc., or suitably in a combination thereof, depending on necessity, to give a unit dose form which is required for generally approved pharmaceutical practices.

Formulations suitable for parenteral routes include aseptic solutions or suspensions containing at least one active component in admixture with water or other pharmaceutically acceptable media. Examples of such parenteral formulations are injections. Preferred liquid carriers for injection generally include water, saline, dextrose solution, other related saccharide solutions, ethanol, glycols such as propylene glycol and polyethylene glycol, etc. For the preparation of injections, the active component is usually admixed with any of carriers such as distilled water, Ringer's solution, physiological saline, suitable dispersing agents, moistening agents, suspending agents, and other materials to form injectable formulations including solutions, suspensions, emulsions, etc. by known techniques in the art.

Examples of aqueous liquids for the injection are a physiological saline and isotonic solutions containing glucose and other aids (e.g. D-sorbitol, D-mannitol, sodium chloride, etc.) where they may be used in combination with a suitable pharmaceutically acceptable auxiliary solubilizer such as alcohol (e.g. ethanol, etc.), polyalcohol (e.g. propylene glycol, polyethylene glycol, etc.), nonionic surface-active agent (e.g. Polysorbate 80^{™}, HCO-50, etc.), etc. The injectable oily liquids may include sesame oil, soybean oil, etc. where they may be used in combination with benzyl benzoate, benzyl alcohol, and other materials as auxiliary solubilizers. In addition, buffers (e.g. phosphate buffer, sodium acetate buffer, etc.) or agents for osmoregulation, analgesic agents (e.g. benzalkonium chloride, procaine hydrochloride, etc.), stabilizers (e.g. human serum albumin, polyethylene glycol, etc.), preservatives (e.g. benzyl alcohol, phenol, etc.), antioxidants such as ascorbic acid, absorbefacients, etc. may be admixed therewith too. The prepared injection solution is usually filled in suitable ampoules.

For parenteral administration, solution or suspension unit dosage forms are prepared in pharmaceutically acceptable sterile fluids such as water, ethanol, and oils, in admixture with or without detergent and other pharmaceutically acceptable aids. The oily vehicle and solvent used in the parenteral formulation may include natural, synthetic or semi-synthetic mono-, di-, or triglycerides; natural, semi-synthetic or synthetic fats and oils; and fatty acids. Examples of such oily vehicles and solvents are plant oils such as peanut oil, corn oil, soybean oil, and sesame oil. For example, this injection can usually be prepared to form unit doses each containing approximately from 0.1 to 10 parts of the compound of the present invention per 100 parts by weight of the dose composition.

The formulation suitable for topical use, such as buccal or rectal application, includes mouthwashes and gargles, dentifrices, sprays for buccal cavity, inhalants, ointments (salves), dental fillers, dental coating agents, dental pastes, suppositories, etc. The mouthwashes and other dental agents are prepared by conventional techniques, using pharmaceutically acceptable carriers. For the sprays for buccal cavity and inhalants, the compound of the present invention can be applied to teeth or other sites after dissolving alone or together with pharmaceutically acceptable inert carriers, in an aerosol or solution for nebulizers, or in the form of powders for inhalation. The ointments (salves) are prepared by conventional techniques, in admixture with conventionally employed pharmaceutical bases such as ointment bases (white petrolatum, paraffin, olive oil, macrogol 400, macrogol ointment, etc.).

The pharmaceutical drugs for topical application (including painting) to teeth and skin can be prepared in the form of a solution or suspension utilizing suitably sterilized water or non-aqueous vehicles. The additives used include buffering agents such as sodium bisulfite and disodium edetate; preservatives including antiseptic, antimicrobial and antifungal agents such as acetic acid, phenylmercuric nitrate, benzalkonium chloride and chlorhexidine; and thickeners such as hypromellose.

The suppositories can be prepared by conventional techniques utilizing carriers well known in the art, preferably suitable non-irritative excipients. Examples of the excipients are those which are solid at room temperature but liquid at rectal temperature wherein such substances melt in the rectum to deliver a drug, such as polyethylene glycols, lanolin, cacao butter, and fatty acid triglycerides. In the suppositories, the compounds of the present invention are applied in the form of compositions containing approximately from 0.1 to 95 percent (weight per volume). The compound, depending on the vehicle and concentration used, can be either suspended or dissolved in the vehicle. Adjuvants such as a local anesthetic, preservative and buffering agent can be dissolved in the vehicle. The formulations suitable for oral application include solid compositions such as tablets, pills, capsules, powders, granules, and troches; fluid compositions such as solutions, syrups, and suspensions; etc. In preparing oral formulations, pharmaceutical adjuvants known in the art are employed. The tablets and pills can be prepared further by enteric coating. When the unit dosage form is a capsule, fluid carriers such as fats and oils can be contained in addition to the aforementioned materials.

When the active components are proteins or polypeptides, conjugation to polyethylene glycol (PEG) is particularly useful, because its toxicity is extremely low in mammals. Further, the conjugation with PEG can sometimes reduce the immunogenicity and antigenicity of a heterologous compound effectively. The compound may be given after being put in a microcapsule device. A polymer such as PEG can be easily attached to an α-amino group of amino-terminal amino acids, an ε-amino group of lysine side chains, a carboxyl group of aspartic acid or glutamic acid side chains, an α-carboxyl group of carboxyl-terminal amino acids, or an activated derivative of glycosyl chains attached to certain asparagine, serine or threonine residues.

Various activated forms of PEG suitable for direct reaction with proteins are known. PEG reagents useful for reaction with amino groups of a protein include active esters of carboxylic acids and carbonate derivatives, particularly those having N-hydroxysuccinimide, p-nitrophenol, imidazole, or 1-hydroxy-2-nitrobenzene-4-sufonate as a leaving group. Similarly, PEG reagents having an aminohydrazine or hydrazide group are useful for reaction with aldehydes produced by periodate oxidation of proteins.

Practice of the invention may begin by diagnosing the mammal as is appropriate for the particular disorder/disease such as autoimmunity, tumor including malignant tumor such as cancer, allergic disease, inflammation, LPS-induced inflammation, LPS-induced elevation in TNF-α, IL-12, and/or IFN-γ production, LPS-induced endotoxin shock that they may be exhibiting. The diagnosis may also continue during treatment, as a therametric procedure, to monitor the progress of treatment, and to direct modification of such parameters as the dosage or frequency in continued treatments, for example. Additional diagnosis that might aid in determining appropriateness for administration of a modified galectin 8 protein (Gal-8 mutein) therapeutic agent include an analysis of expression levels of galectin 8 in the mammal, and a comparison of these levels between cells such as lymphocytes distal from the site of autoimmunity, and those proximal to the site or autoimmunity. The disorder/disease such as autoimmunity, tumor including malignant tumor such as cancer, allergic disease, and inflammation autoimmune disease in the mammal being treated can be monitored by detecting galectin 8 antigen on a cell surface. This monitoring can include contacting a sample derived from the mammal with a galectin 8-specific antibody, and detecting binding of the antibody to the sample.

Gene therapy vehicles include those for delivery of constructs including a coding sequence of a therapeutic of the invention, to be delivered to the mammal for expression in the mammal, for example, a modified galectin 8 protein (Gal-8 mutein) coding sequence, or also including a nucleic acid sequence of all or a portion of modified Gal-8 protein (Gal-8 mutein) for delivery, which can be administered either locally or systemically. These constructs can utilize viral or non-viral vector approaches in in vivo or ex vivo modality. Expression of such coding sequence can be induced using endogenous mammalian or heterologous promoters. Expression of the coding sequence in vivo can be either constitutive or regulated. Where the modified Gal-8 protein (Gal-8 mutein) is expressed in the mammal, it can be expressed as soluble modified Gal-8 mutein, or as a precursor form modified Gal-8 mutein, both or either including, for example, all of the modified Gal-8 mutein, or a biologically active portion, variant, derivative or fusion of modified Gal-8 mutein.

The invention includes gene delivery vehicles capable of expressing the contemplated modified Gal-8 mutein nucleic acid sequences. The gene delivery vehicle is preferably a viral vector. A more preferable gene delivery vehicle includes viral vectors such as retroviral, adenoviral, adeno-associated viral (AAV), herpes viral, or alphavirus vectors. The viral vector can also be an astrovirus, coronavirus, orthomyxovirus, papovavirus, paramyxovirus, parvovirus, picornavirus, poxvirus, togavirus viral vector. The gene delivery vehicles are disclosed generally in D. Jolly, Cancer Gene Therapy, 1(1): 51 to 64 (1994); O. Kimura et al., Human Gene Therapy, 5: 845 to 852 (1994); S. Connelly et al., Human Gene Therapy, 6: 185 to 193 (1995); M.G. Kaplitt et al., Nature Genetics, 8: 148 to 153 (1994), etc.

Retroviral vectors are well known in the art and may include any retroviral gene therapy vector employable in the invention, such as B, C and D type retroviruses, and xenotropic retroviruses (for example, NZB-X1, NZB-X2, NZB9.1 :see, R. R. O'Neill, J. Virol., 53: 100 to 106 (1985), etc.), polytropic retroviruses (for example, MCF, MCF-MLV : see, M. Kelly, J. Virol., 45: 291 to 298 (1983), etc.), spumaviruses and lentiviruses and others (see, R. L. Weiss et al. (Eds.), RNA Tumor Viruses, Second Edition, Cold Spring Harbor Laboratory, Cold Spring Harbor, 1985).

Portions of the retroviral gene therapy vector may be derived from different retroviruses. For example, retrovector LTRs may be derived from a murine sarcoma virus, a tRNA binding site from a Rous sarcoma virus, a packaging signal from a murine leukemia virus, and an origin of second strand synthesis from an avian leukosis virus. These recombinant retroviral vectors may be used to generate transduction competent retroviral vector particles by introducing them into appropriate packaging cell lines (see, U.S. Patent No. 5,591,624). Retrovirus vectors can be constructed for site-specific integration into host cell DNA by incorporation of chimeric integrase (an enzyme that enables the target DNA to be integrated into the DNA of the host cell) into the retroviral particle. It is preferable that the recombinant viral vector is a replication defective recombinant virus.

Packaging cell lines suitable for use with the aforementioned retrovirus vectors are well known in the art, are readily prepared (see, U.S. Patent No. 6,013,517, WO 92/05266). Said packaging cell lines can be used to create producer cell lines (vector cell lines or "VCLs") for the production of recombinant vector particles. Preferably, the packaging cell lines are made from human parent cells (e.g., HT1080 cells) or mink parent cell lines, which eliminates inactivation in human serum.

Preferred retroviruses for the construction of retroviral gene therapy vectors include avian leukosis virus, bovine leukemia virus, murine leukemia virus, mink-cell focus-inducing virus, murine sarcoma virus, reticuloendotheliosis virus and Rous sarcoma virus. Particularly preferred murine leukemia viruses include for example, 4070A and 1504A (Hartley & Rowe, J. Virol., 19: 19-25 (1976)), Abelson (ATCC No. VR-999), Friend (ATCC No. VR-245), Graffi, Gross (ATCC No. VR-590), Kirsten, Harvey sarcoma virus and Rauscher (ATCC No. VR-998), and moloney murine leukemia virus (ATCC No. VR-190), etc.

Such retroviruses may be obtained from depositories or collections such as the American Type Culture Collection ("ATCC") in Rockville, Maryland, USA or isolated from known sources using commonly available techniques.

Exemplary known retroviral gene therapy vectors employable in this invention include those described in GB 2200651, EP 0415731, EP 0345242, WO 89/02468, WO 89/05349, WO 89/09271, WO 90/02806, WO 90/07936, WO 94/03662, WO 93/25698, WO 93/25234, WO 93/11230, WO 93/10218, WO 93/10218, WO 91/02805, U.S. Patent Nos. 5,219,740, 4,405,712, 4,861,719, 4,980,289, 4,777,127, 5,591,624, Vile, Cancer Res, 53: 3860-3864 (1993), Vile, Cancer Res, 53: 962-967 (1993), Ra, Cancer Res, 53: 83-88 (1993), Takamiya, J Neurosci Res, 33: 493-503 (1992), Baba, J Neurosurg, 79: 729-735 (1993), Mann, Cell 33: 153 (1983), Cane, Proc Natl Acad Sci USA, 81: 6349 (1984), Miller, Human Gene Therapy, 1: 5-14 (1990), etc.

Human adenoviral gene therapy vectors are also known in the art and employable in this invention. Such vectors are disclosed in, for example, Berkne, Biotechniques, 6: 616 (1988); Rosenfeld, Science, 252: 431 (1991); WO 93/07283; WO 93/06223; WO 93/07282, etc.

Exemplary known adenoviral gene therapy vectors employable in this invention include those described in the above referenced documents and in WO 94/12649; WO 93/03769; WO 93/19191; WO 94/28938; WO 95/11984; WO 95/00655; WO 95/27071; WO 95/29993; WO 95/34671; WO 96/05320; WO 94/08026; WO 94/11506; WO 93/06223; WO 94/24299; WO 95/14102; WO 95/24297; WO 95/02697; WO 94/28152; WO 94/24299; WO 95/09241; WO 95/25807; WO 95/05835; WO 94/18922; WO 95/09654; etc. Alternatively, administration of DNA linked to killed adenovirus as described in Curiel, Human Gene Therapy, 3: 147-154 (1992) may be employed.

The gene delivery vehicles of the invention also include adenovirus associated virus (AAV) vectors. Leading and preferred examples of such vectors for use in this invention are the AAV-2 based vectors disclosed in WO 93/09239. Most preferred AAV vectors comprise the two AAV inverted terminal repeats in which the native D-sequences are modified by substitution of nucleotides, such that at least 5 native nucleotides and up to 18 native nucleotides, preferably at least 10 native nucleotides up to 18 native nucleotides, most preferably 10 native nucleotides are retained and the remaining nucleotides of the D-sequence are deleted or replaced with non-native nucleotides. The native D-sequences of the AAV inverted terminal repeats are sequences of 20 consecutive nucleotides in each AAV inverted terminal repeat (i.e., there is one sequence at each end) which are not involved in HP formation. The non-native replacement nucleotide may be any nucleotide other than the nucleotide found in the native D-sequence in the same position. Other employable exemplary AAV vectors are pWP-19, pWN-1, and others (Nahreini, Gene, 124: 257-262 (1993)). Another example of such an AAV vector is psub201, and others (Samulski, J. Virol., 61: 3096 (1987)). Another exemplary AAV vector is the Double-D ITR vector, etc. Methods for construction of Double-D ITR are disclosed in U.S. Patent No. 5,478,745. Still other AAV vectors are those disclosed in U.S. Patent Nos. 4,797,368, 5,139,941, 5,474,935, WO 94/288157, etc. Yet a further example of an AAV vector employable in this invention is SSV9AFABTKneo, which contains the AFP enhancer and albumin promoter and directs expression predominantly in the liver. Its structure and construction are disclosed in Su, Human Gene Therapy, 7: 463-470 (1996). Additional AAV gene therapy vectors are described in U.S. Patent Nos. 5,354,678, 5,173,414, 5,139,941, 5,252,479, etc.

The gene therapy vectors of the invention also include herpes vectors. Leading and preferred examples are herpes simplex virus vectors containing a sequence encoding a thymidine kinase polypeptide such as those disclosed in U.S. Patent No. 5,288,641 and EP 0176170. Additional exemplary herpes simplex virus vectors include HFEM/ICP6-LacZ disclosed in WO 95/04139, pHSVlac described in Geller, Science, 241: 1667 to 1669 (1988), WO 90/09441, WO 92/07945, etc., HSV Us3: :pgC-lacZ described in Fink, Human Gene Therapy, 3: 11 to 19 (1992), HSV7134, 2RH 105 and GAL4 described in EP 0453242 A, those deposited with the ATCC as accession numbers ATCC VR-977 and ATCC VR-260, and others.

Alpha virus gene therapy vectors may be employed in this invention. Preferred alpha virus vectors are Sindbis viruses vectors, togavirus, Semliki Forest virus (ATCC VR-67; ATCC VR-1247), Middleberg virus (ATCC VR-370), Ross River virus (ATCC VR-373; ATCC VR-1246), Venezuelan equine encephalitis virus (ATCC VR923; ATCC VR-1250; ATCC VR-1249; ATCC VR-532), those described in U.S. Patent Nos. 5,091,309, 5,217,879, and WO 92/10578, and others. Alpha virus vectors employable herein are those disclosed in U.S. Patent Nos. 5,091,309, 5,217,879, 5,843,723, 6,376,236, WO 94/21792, WO 92/10578, WO 95/07994 and other documents. Such alpha viruses may be obtained from depositories or collections such as the ATCC (Rockville, Maryland, USA), or isolated from known sources using commonly available techniques. Preferably, alphavirus vectors with reduced cytotoxicity are used (see U.S. Patent No. 6,391,632).

DNA vector systems such as eukaryotic layered expression systems are also useful for expressing the modified galectin 8 mutein nucleic acids of the invention. Details of eukaryotic layered expression systems are disclosed in WO 95/07994. Preferably, the eukaryotic layered expression systems of the invention are derived from alphavirus vectors and most preferably from Sindbis viral vectors.

Other viral vectors suitable for use in the present invention include those derived from poliovirus, for example ATCC VR-58 and those described in Evans, Nature, 339: 385 (1989), Sabin, J. Biol. Standardization, 1: 115 (1973), etc.; rhinovirus, for example ATCC VR-1110, and those described in Arnold, J. Cell Biochem, L401 to 405 (1990), etc.; pox viruses such as canary pox virus or vaccinia virus, for example ATCC VR-111 and ATCC VR-2010 and those described in Fisher-Hoch, Proc Natl Acad Sci USA, 86: 317 (1989), Flexner, Ann NY Acad Sci, 569: 86 (1989), Flexner, Vaccine, 8: 17 (1990), U.S. Patent Nos. 4,603,112 & 4,769,330, and WO 89/01973, etc.; SV40 virus, for example ATCC VR-305 and those described in Mulligan, Nature, 277: 108 (1979) and Madzak, J. Gen. Vir, 73: 1533 (1992), etc.; influenza virus (for example ATCC VR-797, etc.) and recombinant influenza viruses made employing reverse genetics techniques as described in U.S. Patent No. 5,166,057, Enami, Proc Natl Acad Sci USA, 87: 3802-3805(1990), Enami & Palese, J Virol, 65: 2711 to 2713 (1991), Luytjes, Cell, 59: 110 (1989), McMicheal., N E J Med, 309: 13 (1983), Yap, Nature, 273: 238 (1978), Nature, 277: 108(1979), and other documents; human immunodeficiency virus as described in EP 0386882, Ruchschacher, J. Vir., 66: 2731 (1992), etc.; measles virus (for example ATCC VR-67, VR-1247) and those described in EP 0440219; Aura virus (for example ATCC VR-368, etc.); Bebaru virus (for example ATCC VR-600, ATCC VR-1240, etc.); Cabassou virus (for example ATCC VR-922, etc.); Chikungunya virus (for example ATCC VR-64, ATCC VR-1241, etc.); Fort Morgan virus (for example ATCC VR-924, etc.); Getah virus (for example ATCC VR-369, ATCC VR-1243, etc.); Kyzylagach virus (for example, ATCC VR-927, etc.); Mayaro virus (for example ATCCVR-66, etc.); Mucambo virus (for example ATCC VR-580, ATCC VR-1244, etc.) ; Ndumu virus (for example ATCC VR-371, etc.); Pixuna virus (for example ATCC VR-372, ATCC VR-1245, etc.); Tonate virus (for example ATCC VR-925, etc.); Triniti virus (for example ATCC VR-469, etc.); Una virus (for example ATCC VR-374, etc.); Whataroa virus (for example ATCC VR-926, etc.); Y-62-33 virus (for example ATCC VR-375, etc.) ; O'Nyong virus, Eastern encephalitis virus (for example ATCC VR-65, ATCC VR-1242, etc.); Western encephalitis virus (for example ATCC VR-70, ATCC VR-125L, ATCC VR-622, ATCC VR-1252, etc.); coronavirus (for example ATCC VR-740, and those described in Hamre, Proc Soc Exp Biol Med, 121: 190 (1966); etc.

Delivery of the compositions of this invention into cells is not limited to the aforementioned viral vectors. Other delivery methods and media may be employed such as, for example, nucleic acid expression vectors, polycationic condensed DNA linked or unlinked to killed adenovirus alone (for example see Curiel, Hum Gene Ther, 3: 147-154 (1992)), ligand linked DNA (for example see Wu, J Biol Chem, 64: 16985 to 16987 (1989)), eucaryotic cell delivery vehicles cells (see, e.g., U.S. Patent Nos. 6,013,517, 6,015,686, etc.), deposition of photopolymerized hydrogel materials, portable gene transfer particle gun, as described in U.S. Patent No. 5,149,655, ionizing radiation as described in WO 92/11033, nucleic charge neutralization or fusion with cell membranes. Additional approaches are described in Philip, Mol Cell Biol, 14: 2411 to 2418 (1994), Woffendin, Proc Natl Acad Sci USA, 91: 1581 to 585 (1994), etc.

Particle mediated gene transfer may be employed. The sequence can be inserted into conventional vectors that contain conventional control sequences for high level expression, and then be incubated with synthetic gene transfer molecules (for example polymeric DNA-binding cations like polylysine, protamine, and albumin, linked to cell targeting ligands such as asialoorosomucoid, as described in Wu et al., J. Biol. Chem., 262: 4429 to 4432 (1987), insulin as described in Hucked, Biochem Pharmacol, 40: 253 to 263 (1990), galactose as described in Plank, Bioconjugate Chem, 3: 533 to 539 (1992), lactose, or transferrin).

Naked DNA may also be employed. Exemplary naked DNA introduction methods are described in WO 90/11092 and U.S. Patent No. 5,580,859. Uptake efficiency may be improved using biodegradable latex beads. DNA coated latex beads are efficiently transported into cells after endocytosis initiation by the beads. The method may be improved further by treatment of the beads to increase hydrophobicity and thereby facilitate disruption of the endosome and release of the DNA into the cytoplasm.

Liposomes that can act as gene delivery vehicles are described in U.S. Patent No. 5,422,120, WO 95/13796, WO 94/23697, WO 91/144445 and EP 524,968. On non-viral delivery, the nucleic acid sequences encoding a modified galectin 8 protein (Gal-8 mutein) polypeptide can be inserted into conventional vectors that contain conventional control sequences for high level expression, and then be incubated with synthetic gene transfer molecules, as described in U.S. Patent Appln. Serial No. 60/023,867. The synthetic gene transfer molecule includes polymeric DNA-binding cations linked to cell targeting ligands such as asialoorosomucoid, insulin, galactose, lactose, or transferrin. The polymeric DNA-binding cation includes for example polylysine, protamine, albumin, etc. Other delivery systems include the use of liposomes to encapsulate DNA comprising the gene under the control of a variety of tissue-specific or ubiquitously-active promoters. Further non-viral delivery suitable for use includes mechanical delivery systems such as the approach described in Woffendin et al., Proc. Natl. Acad. Sci. USA, 91(24): 11581 to 11585 (1994).

Moreover, the coding sequence and the product of expression of such can be delivered through deposition of photopolymerized hydrogel materials. Other conventional methods for gene delivery that can be used for delivery of the coding sequence include, for example, use of portable gene transfer particle gun, as described in U.S. Patent No. 5,149,655; use of ionizing radiation for activating transferred gene, as described in WO 92/11033. Examples of liposome and polycationic gene delivery vehicles are those described in U.S. Patent Nos. 5,422,120 and 4,762,915, WO 95/13796, WO 94/23697, WO 91/14445, EP 0524968, Stryer, Biochemistry, 236 to 240 (1975), W. H. Freeman et al., Biochem Biophys Acta, 600: 1 (1980), Bayer, Biochem Biophys Acta, 550: 464 (1979), Rivnay, Meth Enzymol, 149: 119 (1987), Wang, Proc Natl Acad Sci USA, 84: 7851 (1987), Plant, Anal Biochem, 176: 420 (1989), etc.

The invention discloses a method of treating mammals afflicted with a disorder or disease selected from the group consisting of tumors including malignant ones such as cancer, allergic diseases, inflammations, conditions with immunological abnormality, and autoimmune diseases associated with neutrophils and activated lymphocytes (inter alia, activated T-cells; may include activated B-cells), by administration of a modified galectin 8 mutein or modified galectin 8 mutein-derived therapeutic agent (for example, composition comprising, as a therapeutic agent, either a modified galectin 8 mutein polypeptide or a polynucleotide encoding a modified galectin 8 mutein polypeptide for expression in the mammal). Autoimmune diseases that can be treated by the method and compositions of the invention include any autoimmune disease, or transplantation rejection, including, but not limited to, for example, those autoimmune diseases listed herein.

Modified galectin 8 protein (Gal-8 mutein) can be administered, for example, as a recombinantly expressed polypeptide, or as a variant, derivative, or fusion protein of modified galectin 8 mutein polypeptide, delivered either locally or systemically to the mammal. The nucleic acid molecule (e.g., DNA, RNA, etc.) encoding modified galectin 8 mutein, or a derivative or variant of modified galectin 8 mutein, or a modified galectin 8 mutein fusion, can be administered in a gene therapy protocol, as naked plasmid DNA including regulatory regions for expression in the mammal, or in a viral vector for expression in the mammal. Delivery of modified galectin 8 mutein polypeptide for expression can be accomplished with a pharmaceutically acceptable carrier capable of facilitating the delivery. Treatment of a mammal having an autoimmune disease with a modified galectin 8 mutein-derived therapeutic agent can result in amelioration or remission or the autoimmune disease, or in absence of clinical symptoms attributable to the autoimmunity.

The invention is not limited to theories of how the invention as disclosed herein works. By expressing modified galectin 8 mutein or causing modified galectin 8 mutein to be expressed, or by administering a modified galectin 8 mutein derived therapeutic agent, the activated lymphocytes of concern are preferentially targeted for apoptosis by receiving an action of the modified galectin 8 mutein moiety made available. The modified galectin 8 mutein polypeptide or modified galectin 8 mutein derived therapeutic agent can be administered in the region exhibiting the autoimmunity (for example, in the localized region that characterized the particular autoimmune disease being treated). This optimizes the contact between the administered modified galectin 8 mutein or other therapeutic agents and the target expressing activated T-cells, or other cells, which are specific for the targets expressed on the cells of that region. The cells of the region are thus also good candidates for expressing, by aid of a gene delivery vehicle, a polynucleotide encoding a modified galectin 8 mutein polypeptide administered to the region. Thus, in various permutations and applications of the invention, the expression of the modified galectin 8 mutein polypeptide can be recombinantly engineered to facilitate expression in cells that are under attack by the activated T-cells and other cells. Proposed in the case of transplantation rejection is a modified galectin 8 mutein polypeptide fusion with a binding portion of a molecule capable of binding a protein ubiquitously expressed on the cell surfaces of many cell types. This binding portion can be, for example, heparin, and the molecule on the cell surface to which it binds can be a glycosaminoglycan. Alternatively, the binding portion may be a single chain antibody binding domain, specific for any selected cell surface antigen.

Where the inventive agents and therapeutic techniques are applied in order to obtain cytotoxic actions on tumor cells including malignant tumor cells such as cancers, antiallergic actions, anti-inflammatory actions, normalization of immunological abnormality, hemagglutination, induction of cell adhesion such as neutrophil adhesion, integrin α_{M}-binding activity, proMMP-9 binding activity, promoting actions on active form MMP-9 production, promoting actions on superoxide production, suppressive actions on LPS-induced inflammation, suppressive actions on LPS-induced TNF-α, IL-12, and/or IFN-γ production, suppressive actions on endotoxin shock and other active actions, as well as apoptosis inducing actions on activated lymphocytes (may include inter alia activated T-cells), the invention should be interpreted in the same fashion as in the aforementioned autoimmune case.

The term "administration" or "administering" as used herein refers to the process of delivering, to a mammal, a therapeutic agent, or a combination of therapeutic agents. The process of administration can be varied, depending on the therapeutic agent, or agents, and the desired effect. Administration can be accomplished by any means appropriate for the therapeutic agent, for example, by parenteral or oral delivery. The parenteral delivery can be, for example, subcutaneous, intravenous, intramuscular, intra-arterial, injection into the tissue of an organ, mucosal, pulmonary, topical, or catheter-based. Oral means is by mouth, including pills or other gastroenteric delivery means, including a drinkable liquid. Mucosal delivery can include, for example, intranasal delivery. Pulmonary delivery can include inhalation of the agent. Administration generally also includes delivery with a pharmaceutically acceptable carrier (for example, a buffer, a polypeptide, a peptide, a polysaccharide conjugate, a liposome, a lipid, etc.). A gene therapy protocol is considered to include an administration in which the therapeutic agent is a polynucleotide capable of accomplishing a therapeutic goal when expressed as a transcript or a polypeptide in the mammal, and can be applied to both parenteral and oral delivery means. Such administration means are selected as appropriate for the disease being treated. For example, where the disease is organ-based, delivery may be local, and for example, where the disease is systemic, the delivery may be systemic.

The "co-administration" refers to administration of one or more therapeutic agents in course of a given treatment of a patient. The agents may be administered with the same pharmaceutical carrier, or different carriers. They may be administered by the same or different administration means. The agents may be the same type of agent or different types of agents, for example, different types can include polynucleotides, polypeptide, or small molecules. The time of administration may be exactly the same time, or one therapeutic agent may be administered before or after another agent. Thus, co-administration can be simultaneous, or consecutive. The exact protocol for a given combination of therapeutic agents is determined considering the agents and the condition being treated, among other considerations.

The term "in vivo administration" refers to administration to a patient (for example a mammal), of a polynucleotide encoding a polypeptide for expression in the mammal. In particular, direct in vivo administration involves transfecting a mammalian cell with a coding sequence without removing the cell from the mammal. Thus, direct in vivo administration may include direct injection of the DNA encoding the polypeptide of interest in the region afflicted by the autoimmune disease, resulting in expression in the patient's cells.

The term "ex vivo administration" refers to transfecting a cell (for example, a cell from a population of cells that are under autoimmune attack) after the cell is removed from the patient (for example a mammal). After transfection the cell is then replaced in the mammal. Ex vivo administration can be accomplished by removing cells from a mammal, optionally selecting for cells to be transformed (i.e., cells under attack by an autoimmune mechanism), rendering the selected cells incapable of replication, transforming the selected cells with a polynucleotide encoding a gene for expression (i.e., modified galectin 8 mutein), including also a regulatory region for facilitating the expression, and placing the transformed cells back into the patient for expression of the modified galectin 8 mutein. The "therapeutically effective amount" is that amount that generates the desired therapeutic outcome. For example, if the therapeutic effect desired is a remission from autoimmunity, the therapeutically effective amount is that amount that facilitates the remission. The therapeutically effective amount can be an amount administered in a dosage protocol that includes days or weeks of administration, for example. Where the therapeutic effect is a reduction of the effects of an autoimmune response in the mammal, for example, during the manifestations of symptoms of an autoimmune disease, the effective amount of an agent to accomplish this in the mammal is that amount that results in reduction of the symptoms of autoimmunity.

The term "pharmaceutically acceptable carrier" refers to a carrier for administration of a therapeutic agent (for example, a polypeptide, polynucleotide, small molecule, peptoid, peptide, etc.). It refers to any pharmaceutically acceptable carrier that does not itself induce the production of antibodies harmful to the individual receiving the composition, and which may be administered without undue toxicity. Within another aspect of the invention, pharmaceutical compositions are provided, comprising a recombinant viral vector as described above, in combination with a pharmaceutically acceptable carrier or diluent. Such pharmaceutical compositions may be prepared either as a liquid solution, or as a solid form (e.g., lyophilized) which is suspended in a solution prior to administration. In addition, the composition may be prepared with suitable carriers or diluents for either surface administration, injection, oral, or rectal administration. Pharmaceutically acceptable carriers or diluents are nontoxic to recipients at the dosages and concentrations employed. Representative examples of carriers or diluents for injectable solutions include water, isotonic saline solutions which are preferably buffered at a physiological pH (such as phosphate-buffered saline or Tris-buffered saline), mannitol, dextrose, glycerol, and ethanol, as well as polypeptides or proteins such as human serum albumin. A particularly preferred composition comprises a vector or recombinant virus in 10 mg/ml mannitol, 1 mg/ml HSA, 20 mM Tris, pH 7.2, and 150 mM NaCl. In this case, since the recombinant vector represents approximately 1 mg of material, it may be less than 1% of high molecular weight material, and less than 1/100,000 of the total material (including water). This composition is stable at 20°C for at least six months.

The pharmaceutical compositions of the present invention may also additionally include factors which stimulate cell division, and hence, uptake and incorporation of a recombinant retroviral vector. Preserving recombinant viruses is described in U.S. Patent No. 5,792,643.

All of the therapeutic agents that make up the proposed therapy of the invention can be incorporated into an appropriate pharmaceutical composition that includes a pharmaceutically acceptable carrier for the agent. The pharmaceutical carrier for the agents may be the same or different for each agent. Suitable carriers may be large, slowly metabolized macromolecules such as proteins, polysaccharides, polylactic acids, polyglycolic acids, polymeric amino acids, amino acid copolymers, and inactive viruses in particles. Such carriers are well known to those of ordinary skill in the art.

Pharmaceutically acceptable salts which can be used therein, include for example inorganic acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington's Pharmaceutical Sciences (Mack Pub. Co., N.J., USA, 1991). Pharmaceutically acceptable carriers in therapeutic compositions may contain liquids such as water, saline, glycerol and ethanol. Auxiliary substances may include wetting or emulsifying agents, etc. Additionally, pH buffering substances, and the like, may be present in such vehicles. Typically, the therapeutic compositions are prepared as injectables, either as liquid solutions or suspensions; solid forms suitable for solution in, or suspension in, liquid vehicles prior to injection may also be prepared. Liposomes are included within the definition of a pharmaceutically acceptable carrier.

Provided are pharmaceutical compositions comprising a recombinant retrovirus or virus carrying one of the above-described vector constructs, in combination with a pharmaceutically acceptable carrier or diluent. The composition may be prepared either as a liquid solution, or as a solid form (e.g., lyophilized) which is suspended in a solution prior to administration. In addition, the composition may be prepared with suitable carriers or diluents for either surface administration, injection, oral, or rectal administration.

Pharmaceutically acceptable carriers or diluents are nontoxic to recipients at the dosages and concentrations employed. Representative examples of carriers or diluents for injectable solutions include water, isotonic saline solutions which are preferably buffered at a physiological pH (such as phosphate-buffered saline or Tris-buffered saline), mannitol, dextrose, glycerol, and ethanol, as well as polypeptides or proteins such as human serum albumin. A vector or recombinant virus can be delivered in a pharmaceutical composition in 10 mg/ml mannitol, 1 mg/ml HSA, 20 mM Tris, pH 7.2, and 150 mM NaCl. In this case, since the recombinant vector represents approximately 1 g of material, it may be less than 1% of high molecular weight material, and less than 1/100,000 of the total material (including water). This composition is stable at 20°C for at least six months.

The pharmaceutically acceptable carrier or diluent may be combined with the gene delivery vehicles to provide a composition either as a liquid solution, or as a solid form (e.g., lyophilized) which can be resuspended in a solution prior to administration. The two or more gene delivery vehicles are typically administered via traditional direct routes, such as buccal/sublingual, rectal, oral, nasal, topical, (such as transdermal and ophthalmic), vaginal, pulmonary, intraarterial, intramuscular, intraperitoneal, subcutaneous, intraocular, intranasal or intravenous, or indirectly.

The therapeutic drug of the present invention may include optionally, for example, polynucleotides for expression in the mammal. Said therapeutic drugs can be formulated into an enteric coated tablet or gel capsule according to known methods in the art. These are described in the following patent documents: U.S. Patent No. 4,853,230, EP 225,189, AU 9,224,296, AU 9,230,801, WO 92144,52, and others. Such a capsule is administered orally to be targeted to the intestinum. At 1 to 4 days following oral administration, expression of the polypeptide, or inhibition of expression by, for example a ribozyme or an antisense oligonucleotide, is measured in the plasma and blood, for example by antibodies to the expressed or non-expressed proteins.

The gene delivery vehicle can be introduced into a mammal, for example, by injection, particle gun, topical administration, parental administration, inhalation, or iontophoretic delivery, as described in U.S. Patent Nos. 4,411,648, 5,222,936, 5,286,254, WO 94/05369, etc.

The therapeutic composition or therapeutic agent can be administered with other therapeutic agents capable of combating tumors including malignant tumors such as cancers, or ameliorating allergy, inflammation, immunological abnormality, or the autoimmune disease, or capable of enhancing the therapeutic benefits of administration of a modified galectin 8 mutein therapeutic agent. For example, administration for treatment of an allergic reaction can be by aerosol administration of modified galectin 8 mutein polynucleotide for expression in the cells present in tissue such as mucosal, nasal, bronchial or lung tissue, and may be most favorably administered in repeat administrations, for example by nasal or aerosol spray several times daily for a period of time until the allergic reaction subsides.

The gene delivery vehicle may be administered at single or multiple sites to a mammal directly, for example by direct injection, or alternatively, through the use of target cells transduced ex vivo. The present invention also provides pharmaceutical compositions (including, for example, various excipients) suitable for administering the gene delivery vehicles.

A vector construct which directs the expression of a modified galectin 8 mutein polypeptide, variant, derivative, analogue, mutant, or chimera thereof can be directly administered to a tumor site containing malignant tumor such as cancer, or a site exhibiting allergy, inflammation, immunological abnormality, or autoimmunity, for example the pancreas, kidney, liver, joints, brain, the spinal fluid, skin, or other region or organ of the body. Various methods may be used within the context of the present invention in order to directly administer the vector construct. For example, arteries which serve the region may be identified, and the vector injected into such an artery, in order to deliver the vector directly into the site. Similarly, the vector construct may be directly administered to the skin surface, for example, by application of a topical pharmaceutical composition containing the vector construct.

In a direct administration, combination therapeutic agents including a modified galectin 8 mutein therapeutic agent and other anti-autoimmune agents can be administered together. The co-administration can be simultaneous, achieved for example by placing polynucleotides encoding the agents in the same vector, or by putting the agents, whether polynucleotide, polypeptide, or other drug, in the same pharmaceutical composition, or by administering the agents in different pharmaceutical compositions injected at about the same time, and perhaps in the same location. If the co-administration is not simultaneous (for example, in the case of administration of the prodrug after administration of the prodrug activator), the second agent can be administered by direct injection as appropriate for the goals of the therapy. Thus, for example, in the case of an administration of a prodrug, the prodrug is administered at the same location as the prodrug activator. The co-administration protocol can include a combination of administrations to achieve the goal of the therapy. Further, the co-administration can include subsequent administrations as is necessary, for example, repeat in vivo direct injection administrations of a modified galectin 8 mutein.

Within the context of the present invention, it should be understood that the removed cells may be returned to the same animal, or to another allogenic animal or mammal. In such a case it is generally preferable to have histocompatibility matched animals (although not always, see, e.g., Yamamoto et al., AIDS Research and Human Retroviruses, 7: 911-922 (1991); Yamamoto et al., Journal of Virology, 67: 601-605 (1993)).

Cells may be removed from a variety of locations in the patient. In addition, within other embodiments of the invention, a vector construct may be inserted into, for example, cells from the skin (dermal fibroblasts, etc.), or from the blood (e.g., peripheral blood leukocytes, etc.). If desired particular fractions of cells such as a T cell subset or stem cells may also be specifically removed from the blood (see, for example, WO 91/16116). Vector constructs may then be contacted with the removed cells utilizing any of the above-described techniques, followed by the return of the cells to the warm-blooded animal, preferably to or within the vicinity of the region exhibiting autoimmunity.

Once the patient, for example a mammal, has been diagnosed, practice of the invention includes providing a modified galectin 8 mutein therapeutic agent, and administering it to the mammal in a manner and dose appropriate for the particular disease being treated (for example tumors, allergic or autoimmune diseases, etc.), and monitoring the mammal for determining the need for continued or modified administrations of the therapeutic agent. Practice of the invention is accomplished by identifying the disease to be treated, and determining the probable cell-type or region of the body to which a targeted gene therapy can be applied. The modified galectin 8 mutein polynucleotide is constructed, including either a plasmid with regulatory regions for expression in the mammal, or a viral vector for the expression. Some of the mammalian cells can be removed, transfected with the polynucleotide encoding modified galectin 8 mutein, and replaced into the mammal for expression of modified galectin 8 mutein. Alternatively the polynucleotide can be administered to the mammal, for example in the region where the disease is manifest, for expression in the mammalian cells in that region.

Thus, for example, in the case of malignant tumor cells, the tumor cells in diseased tissue or organ can be transfected in vivo or ex vivo with modified galectin 8 mutein. Further, for example, in the case of rheumatoid arthritis, the synovial cells can be transfected ex vivo with modified galectin 8 mutein.

For example, in treatment of multiple sclerosis, modified galectin 8 mutein can be injected into the region of the brain being effected to facilitate expression of modified galectin 8 mutein in the cells that are under attack by the activated T-cells in an autoimmune type of reaction. Also, by example, in the case of multiple sclerosis, modified galectin 8 mutein DNA can be locally injected into the mammal's brain, or cells from the spinal fluid can be removed, transfected with modified galectin 8 mutein DNA, and returned to the region of the spinal cord. Further by example, for treating a mammal having Sjögren's syndrome, the organ targeted by the disease is selected for administration of modified galectin 8 mutein polypeptide by injection. Also, by example, for mammal's suffering from Sjögren's syndrome, the affected organ can be identified, for example the kidney, and modified galectin 8 mutein DNA administered to the organ directly, or cells from the organ removed, transfected, and replaced in the body for expression of modified galectin 8 mutein in those cells in the mammal.

For example, in the case of preventing transplantation rejection, the animal to receive the transplant can receive localized or systemic administration of a modified galectin 8 mutein therapeutic agent in order to kill any activated patient cells which attacks the foreign cells, tissue or organ, or a modified galectin 8 mutein polypeptide can be expressed in cells on the external surface of the organ just prior to the transplant, in order to protect the organ once inside the patient's body. Continued administration of the modified galectin 8 mutein therapeutic agent may be necessary while the recipient's immune system adjusts to the foreign cells, tissue or organ.

The modified galectin 8 mutein therapeutic agent is expected to act analogously to native galectin 8 (wild type galectin 8). Accordingly, it will be used to cause an apoptotic reaction in the cells. Thus, stoichiometrically, the clinician would be able to be aware of the amount of modified galectin 8 mutein that needs to be expressed or otherwise administered to the mammal for achieving apoptosis. Within other aspects of the present invention, the vector constructs described herein may also direct the expression of additional non-vector derived genes. For example, a prodrug system applied in conjunction with administration of modified galectin 8 mutein can act as a safety mechanism for the gene therapy, or can act as a combination therapeutic agent.

As a safety mechanism, the prodrug activator is expressed in a vector along with the modified galectin 8 mutein. When it is determined that the system should be arrested, the prodrug is administered and the prodrug activator is activated. This allows the clinician a measure of control over the gene therapy. The prodrug activator/prodrug system may be useful for inactivating the transfected cells in the mammal, where, for example, the autoimmunity is exacerbated by the modified galectin 8 mutein expression. The prodrug activator/prodrug system can also be administered as combination therapeutic agent, in a combination therapy protocol, for achieving cell killing using the prodrug activation provided by the prodrug activator/prodrug system.

The therapy including administration of a polynucleotide encoding a modified galectin 8 mutein polypeptide, in conjunction with a prodrug activator and prodrug, can also be immunomodulatory. The "immunomodulatory" refers to use of factors which, when manufactured by one or more of the cells involved in an immune response, or, which, when added exogenously to the cells, causes the immune response to be different in quality or potency from that which would have occurred in the absence of the factor. The quality or potency of a response may be measured by a variety of assays known to one of skill in the art including, for example, in vitro assays which measure cellular proliferation (e.g., ³H thymidine uptake), and in vitro cytotoxic assays (e.g., which measure ⁵¹Cr release) (see, Warner et al., AIDS Res. and Human Retroviruses, 7: 645-655 (1991)). Immunomodulatory factors may be active both in vivo and ex vivo. Representative examples of such factors include cytokines, such as interleukins 2, 4, 6, 12 and 15 (among others), α-interferons, β-interferons, γ-interferons, GM-CSF, G-CSF, and tumor necrosis factors (TNFs). Other immunomodulatory factors include, for example, CD3, ICAM-1, ICAM-2, LFA-1, LFA-3, MHC class I molecules, MHC class II molecules, β₂-microglobulin, chaperones, or analogs thereof. If the gene delivery vehicle, however, does not express an immunomodulatory cofactor which is a cytokine, this cytokine may be included in the above-described compositions, or may be administered separately (concurrently or subsequently) with the above-described compositions. Briefly, within such an embodiment, the immunomodulatory cofactor is preferably administered according to standard protocols and dosages known in the art. For example, α-interferon may be administered at a dosage of 100 to 5000,000 units/day for 2 to 4 months, and IL-2 at a dosage of 10,000 to 100,000 units/kg of body weight, 1 to 3 times/day, for 2 to 12 weeks. γ-Interferon may be administered at dosages of 150,000 to 1,500,000 units 2 to 3 times/week for 2 to 12 weeks for example, for upregulating the expression of a gene concerned in activated T-cells for achieving more effective therapy with the administration of modified galectin 8 mutein.

As a combination therapeutic agent, the prodrug activator can be expressed from its own vector, or from the same vector as the modified galectin 8 mutein polypeptide. Either vector system (a single vector, or two vectors) can be administered by in vivo or ex vivo means. In an autoimmune therapy, for example, the addition of the prodrug activator facilitates further immunomodulatory effect supporting the effect achieved by modified galectin 8 mutein and in addition, addition of the prodrug can activate the killing of transfected cells.

A chaperon molecule can be administered before, contemporaneously with or after administration of the polynucleotide therapeutic, and the chaperon molecule can be, for example, a heat shock protein, such as, for example hsp70. Further, the polynucleotide being expressed in the mammal can be linked to an inducible promoter, for example a tissue specific promoter, for the purpose of, for example, ensuring expression of the polynucleotide only in the desired target cells. Additionally, for the purpose of effectively delivering the polynucleotide to a tissue, the polynucleotide can be flanked by nucleotide sequences suitable for integration into genome of the cells of that tissue.

For this and many other aspects of the invention, effectiveness of treating humans may first be tested in animal models for a given autoimmune disease. Such existing animal models include those for the following autoimmune disease: for example, Sjögren's syndrome (autoimmune dacryodentis or immune-mediated sialadenitis), autoimmune myocarditis, primary biliary cirrhosis (PBC), inflammatory heart disease, mercury-induced renal autoimmunity, insulin dependent diabetes mellitus (type I diabetes or IDD), post-thymectomy autoimmunity, a central nervous system (CNS) demyelination disorder, CNS lupus, narcolepsy, myasthenia gravis (MG), Grave's disease, a immune-mediated PNS disorder, osteoarthritis, rheumatoid arthritis, uveitis, medullary cystic fibrosis, autoimmune hemolytic disease, autoimmune vasculitis, ovarian autoimmune disease, human scleroderma, and other autoimmune-related diseases.

The multiple gene delivery vehicles may be administered to animals or plants. In preferred embodiments, the animal is a warm-blooded animal, further preferably selected from the group consisting of mice, chickens, cattle, pigs, pets such as cats and dogs, horses, and humans. For polypeptide therapeutics, for example, modified galectin 8 mutein or other cytokine, the dosage can be in the range of about 5 to 50 µg/kg of mammal body weight, also about 50 µg/kg to about 5 mg/kg, about 100 to 500 µg/kg of mammal body weight, and about 200 to about 250 µg/kg.

For polynucleotide therapeutics, for example a polynucleotide encoding a native or mutant modified galectin 8 mutein polypeptide, depending on the expression of the polynucleotide in the patient, for example a mammal, for tissue targeted administration, vectors containing expressible constructs of coding sequences, or non-coding sequences can be administered in a range of: about 100 ng to about 200 mg of DNA for local administration in a gene therapy protocol, also about 500 ng to about 50 mg, also about 1 µg to about 2 mg of DNA, about 5 µg of DNA to about 500 µg of DNA, and about 20 µg to about 100 µg during a local administration in a gene therapy protocol, and for example, a dosage of about 500 µg, per injection or administration. Where greater expression is desired, over a larger area of tissue, larger amounts of DNA or the same amounts readministered in a successive protocol of administrations, or several administrations to different adjacent or close tissue portions of for example, a tumor site, may be required to effect a positive therapeutic outcome.

For administration of small molecule therapeutics, depending on the potency of the small molecule, the dosage may vary. For a very potent inhibitor, dose levels per kilogram of mammal may be sufficient, for example, in the range of about 1µg/kg to about 500 mg/kg of mammal weight, and about 100µg/kg to about 5 mg/kg, and about 1*µ*g/kg to about 50µg/kg, and, for example, about 10*µ*g/kg. For administration of peptides and peptoids the potency also affects the dosage, and may be in the range of about 1*µ*g/kg to about 500 mg/kg of mammal weight, and about 100µg/kg to about 5 mg/kg, and about 1*µ*g/kg to about 50µg/kg, and a usual dose might be about 10µg/kg.

Dose levels of said active components may vary within a wide range. Specific dose levels and administration cycles for any particular patient will be employed depending upon a variety of factors including the activity of specific compounds employed, the sex, age, body weight, general health, diet, time of administration, route of administration, rate of excretion, drug combination, and the severity of the particular disease undergoing therapy.

For the manufacture of pharmaceutical compositions and preparations, the additives, other materials, preparation methods and the like can be suitably selected from those disclosed in Nippon Yakkyokuho Kaisetsusho Henshu Iinkai (Ed.), "14th Edition Nippon Yakkyokuho Kaisetsusho (Commentary on The Japanese Pharmacopoeia 14th Edition (JPXIV))", June 27, 2001, Hirokawa Pub. Co., Tokyo, Japan; Hisashi Ichibagade et al. (Ed.), "Iyakuhin no Kaihatsu (Pharmaceutical Research and Development, Ikuo Suzuki, chief editor), Volume 12 (Seizai Sozai I (Pharmaceutical Necessities 1))", October 15, 1990, Hirokawa Pub. Co., Tokyo, Japan; ibid., Volume 12 (Seizai Sozai II (Pharmaceutical Necessities 2)), October 28, 1990, Hirokawa Pub. Co., Tokyo, Japan; etc., depending on necessity, and can be adapted by referring to the disclosures therein.

The active substances or components according to the present invention include (a) modified galectin-8 variants and polypeptides having biological activity substantially equivalent to that of said modified Gal-8 variant, (b) polynucleotides encoding modified Gal-8 variants or polypeptides having biological activity substantially equivalent to that of the modified Gal-8 variant, (c) factors discovered by applications of modified galectin-8 variant techniques, and (d) vehicles for transfer of genes coding for modified Gal-8 variants or polypeptides having biological activity substantially equivalent to that of the modified Gal-8 variant, as described herein. These substances and components are useful for utilizing the following properties of human galectin-8: exerting hemagglutination, induction of cell adhesion such as neutrophil adhesion, integrin α_{M} binding activity, proMMP-9 binding activity, promotion of active form MMP-9 production, promotion of superoxide production, suppression of LPS-induced inflammation, inhibition of LPS-induced TNF-α, IL-12, and/or IFN-γ production, suppression of endotoxin shock, exerting cytotoxity toward tumor cells, but not toward normal cells; inducing apoptosis in tumor cells, but not in normal cells; inhibiting metastasis of malignant cells; and inducing apoptosis in activated immune cells, in particular, in activated CD4-positive T cells, but not in resting T cells, in particular, in CD4-positive T cells (helper T cells). Thus, the above-mentioned substances and components are promising to serve as drugs utilizing activities similar to those of anti-neoplastic agents, anti-allergy agents, immunoregulators (immunomodulators), therapeutic agents for autoimmune diseases, anti-inflammatory agents, depressants against endotoxin-induced inflammation, endotoxin shock suppressants, and adrenocortical steroid hormones.

### Examples

Details of the present invention are described by the following examples but such examples are provided only for illustrative purposes, and for referential embodiments of the present invention. These examples have been described herein for the purpose of illustrating specific embodiments of the present invention but should not be construed as in any sense limiting the scope of the invention disclosed herein. It should be understood in the present invention that various embodiments can be made or executed within the spirit, scope and concept disclosed herein. All the examples were carried out or can be carried out, unless otherwise disclosed herein specifically, by standard techniques which are well known and conventional to those skilled in the art.

Specific molecular biological operations, treatment conditions, etc. in examples as described herein below are conducted or selected according to customary techniques disclosed in standard experimental manuals: for DNA cloning, J. Sambrook, E. F. Fritsch & T. Maniatis, "Molecular Cloning", 2nd ed., Cold Spring Harbor Laboratory, Cold Spring Harbor, N. Y. (1989), and D. M. Glover et al. ed., "DNA Cloning", 2nd ed., Vol. 1 to 4, (The Practical Approach Series), IRL Press, Oxford University Press (1995); when PCR techniques are applied, H. A. Erlich ed., PCR Technology, Stockton Press, 1989 ; D. M. Glover et al. ed., "DNA Cloning", 2nd ed., Vol. 1, (The Practical Approach Series), IRL Press, Oxford University Press (1995) 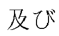 M. A. Innis et al. ed., "PCR Protocols", Academic Press, New York (1990) and others. When commercially available reagents and kits are used, protocols, agents, drugs, and the like attached thereto are employed herein.

### Example 1

### [Production of Modified Galectin 8 Protein (Gal-8 Mutein)]

### (A) Extraction of Total RNA from A432 Cell

A432 cells (human-derived cell lines, skin tumor, carcinoma, squanous cells) were obtained from American Type Culture Collection (ATCC CRL 1555). The cell line was maintained in FCS(10%)-added DME medium (DMEM; Sigma, St. Louis, USA) at 37°C under 5% CO₂/air.

Total RNA extraction from A432 cells was conducted as follows: Briefly, A432 cells were cultured in a 90-mm plate (in DMEM containing 10% FBS), and washed twice with PBS. To the washed cells was added ISOGEN (Trade Name: NIPPON GENE Co., Ltd., Japan) at 3 ml per plate, and total RNA was extracted according to the kit manual (NIPPON GENE Co., Ltd., Japan).

ISOGEN (NIPPON GENE Co., Ltd., Japan) is a homogeneous solution containing phenol and guanidinium thiocyanate, which is colored for the convenience of liquid phase separation. The following basic steps are carried out for RNA extraction with this ISOGEN (Trade Name: NIPPON GENE Co., Ltd., Japan). To a sample is added an aliquot of ISOGEN (Trade Name: NIPPON GENE Co., Ltd., Japan) and the resultant mixture is subjected to lysis or homogenization, followed by addition of chloroform. The mixture is then centrifuged. When centrifuged, the homogenate is separated to form three phases, interphase, aqueous and organic phases. Since RNA is extracted exclusively in the aqueous phase, this aqueous phase is collected, and admixed with isopropanol to precipitate RNA which is then washed to give total RNA.

### (B) Purification of Poly(A)⁺RNA from Total RNA and cDNA Synthesis

Poly(A)⁺RNA purification from total RNA and cDNA synthesis were conducted as follows: Briefly, A432 cell-derived total RNA was dissolved in DEPC-treated water to make the concentration 1 mg/ml. The purification of poly(A)⁺RNA from total RNA was carried out with PolyATtract^{®} mRNA Isolation System (Trade Name: Promega) according to the kit manual. The purified poly(A)⁺RNA was dissolved in DEPC-treated water to make the concentration 5 µg/20 µl.

The PolyATtract^{®} mRNA Isolation System (Trade Name: Promega) utilizes a biotinylated oligo(dT) probe and avidin coupled to paramagnetic particles. To RNase-free water is added an aliquot of total RNA sample, and annealing is conducted in the presence of biotinylated oligo(dT) probes and 2 XSSC. Next, the annealing mixture is admixed with Streptavidin MagneSphere^{®} Paramagnetic Particles (SA-PMPs). After incubation, the tube containing SA-PMPs is placed in a magnetic stand. The supernatant is carefully removed for collection of SA-PMP pellets. The collected SA-PMP pellet is resuspended in RNase-free water, and then placed in the magnetic stand until the SA-PMPs have collected, followed by acquisition of an aqueous solution containing eluted mRNA. When necessary, the resultant solution may be subjected to drying for removal of solvents, and/or to alcohol precipitation to give a concentrate.

The synthesis of cDNA from poly(A)⁺RNA (5 µg) was performed with First-Strand cDNA Synthesis Kit (Trade Name: Amersham Biosciences) according to the kit manual, wherein Not I-d(T)₁₈ was used as a primer. The aforementioned purified poly(A)⁺RNA was dissolved in DEPC-treated water to make the concentration a predetermined value, kept at 65°C for 10 min to denature templates, and then kept at 0°C for 2 min to give a poly(A)⁺RNA sample solution (RNA template). The First-Strand cDNA Synthesis Kit (Trade Name: Amersham Biosciences) is a set of cDNA synthesis primer: Not I-d(T)₁₈ Primer, First-Strand Reaction Mixes (Cloned, FPLCpure M-MuLV Reverse Transcriptase; RNA guard; RNase/DNase Free BSA; dATP, dCTP, dGTP & dTTP-containing Tris-HCl (pH8.0)), DTT Solution and RNase-Free Water. The Kit is optimized for incubating a mixture of RNA template, Reaction Mix, Primer, DTT Solution and RNase-Free Water at 37°C for 60 min in order to complete the synthesis of first-strand cDNA.
The resultant cDNA is applicable to PCR without any treatment.

### (C) Construction of Expression Vector for Modified Galectin 8 Protein (Gal-8 Mutein)

The expression vectors were constructed with the following:
(1) cDNA, prepared from a A432 poly (A)⁺RNA fraction
(2) pET-11a vector (STRATAGENE)
(3) primers for PCR:
   PEG8-1:
      CGTCCTCATATGATGTTGTCCTTAAACAACCTAC [SEQ ID NO: 11]
   PEG8NCRD2:
      CGACCGCATATGCGAGCTGAAGCTAAAACCAAT [SEQ ID NO: 12]
   PEG8CCRD1:
      CGTCCTCATATGAGGCTGCCATTCGCTGCAAGG [SEQ ID NO: 13]
   PEG8CCRD2:
      CGACCGGGATCCCTACCAGCTCCTTACTTCCAG [SEQ ID NO: 14]

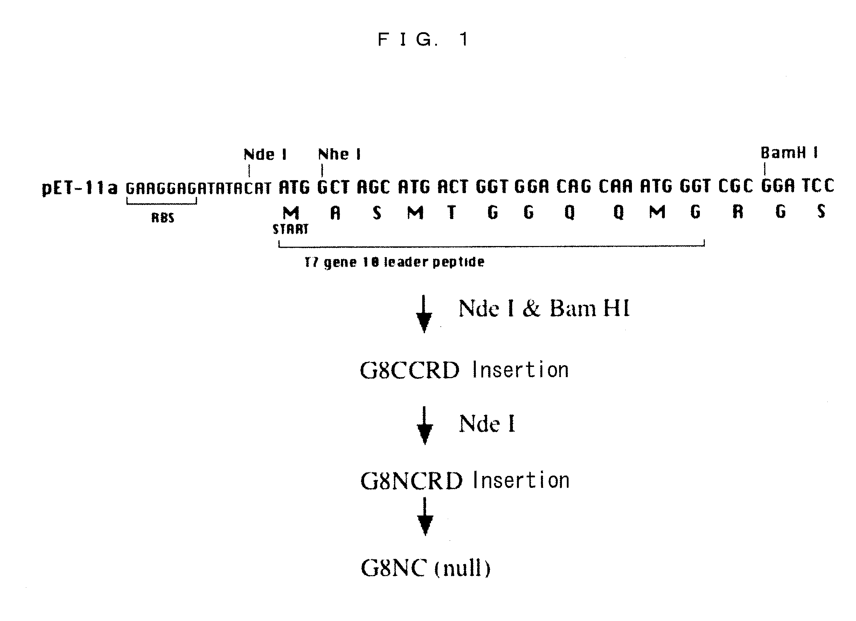

Into the NdeI-BamHI site of vector pET-11a was inserted the N-terminal carbohydrate recognition domain (NCRD) and C-terminal carbohydrate recognition domain (CCRD) of galectin 8 according to steps as shown in FIG. 1 to generate an expression vector for modified galectin 8 (G8NC(null)) wherein the linker peptide lacked.

First, (1) cDNA corresponding to the C-terminal CRD of human galectin 8 and (2) cDNA corresponding to the N-terminal CRD of human galectin 8, respectively, were obtained from galectin 8 cDNA. Briefly, cDNA corresponding to the C-terminal CRD of human galectin 8 (G8CCRD) was amplified from human galectin 8 cDNA with PCR primers:
PEG8CCRD1 + PEGBCCRD2. G8CCRD was digested with restriction enzymes (NdeI + BamHI), and inserted into vector pET-11a treated with the same restriction enzymes to create pET-G8CCRD. The PCR was performed with KOD DNA polymerase kit (TOYOBO Code No. KOD-101). A PCR Reaction mixture (dNTP mix, 25mM MgCl₂, 10×Buffer, KOD DNA polymerase (0.05u), primers and template cDNA) was reacted under the following PCR cycle conditions:
After treatment at 94°C for 2 min, a cycle consisting of 98°C for 15 sec, then 65°C for 2 sec, and next 74°C for 30 treatments was repeated 25 times, and finally the reaction was terminated at 4°C.
The insertion of the PCR-amplified fragment into the vector was carried out with Ligation high kit (TOYOBO Code No.LGK-101). For reaction, the PCR-amplified fragment was mixed with the vector at a molar ratio of insert: vector = about 5:1, and then admixed with the reagent "Ligation high" at a ratio of reagent/total DNA solution = 1/2 (volume/volume). The insertion was done by O/N reaction at 16°C for 16 hr.

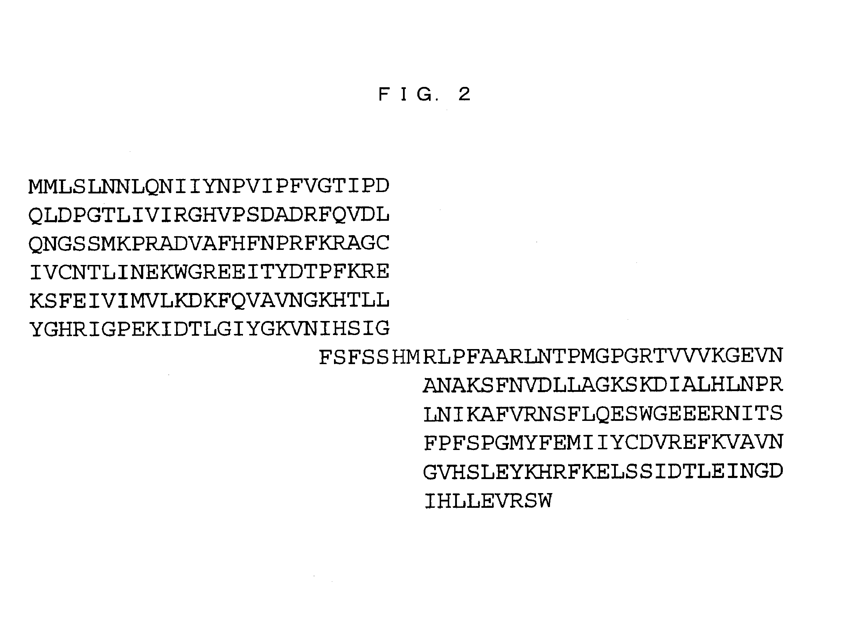

Secondly, cDNA corresponding to the N-terminal CRD of human galectin 8 (G8NCRD) was amplified from human galectin 8 cDNA with PCR primers: PEG8-1 + PEG8NCRD2. G8NCRD was digested with restriction enzyme NdeI, and the resultant fragment was inserted into a site derived from pET-G8CCRD by digestion with the same restriction enzyme (NdeI) followed by dephosphorylation to create pET-G8NC(null). The PCR amplification and incorporation into the vector were carried out in the same manner as aforementioned. In pET-G8NC(null) is encoded a polypeptide having a mutant amino acid sequence that differs from the amino acid sequence of human M-type galectin 8 (hGal-8M) by the amino acid replacement of a region ranging from Asp(D)¹⁵⁶ to Leu(L)¹⁸³ (28 amino acids) with the sequence: His-Met (HM). That is, the construct has a nucleotide sequence of SEQ ID NO: 1, which codes for a polypeptide with the amino acid sequence of SEQ ID NO: 2 (see FIG. 2).

### (D) Expression and Purification of Recombinant

### Modified Galectin 8 Protein (Gal-8 Mutein)

The expression plasmid vector pET-G8NC(null) obtained in the aforementioned step (C) was introduced into E. coli (BL21(DE3)). The introduction was done by electroporation (or electropermeabilization). Briefly, a mixture of competent BL21(DE3) and an aqueous plasmid vector solution was subjected to electroporation at a voltage of 1.8kV for transfection.

The expression of recombinant proteins were conducted as follows: E. coli was cultured in 2×YT medium containing 2% (w/v) glucose and 100 µg/ml ampicillin, and admixed with 0.1 M isopropyl-β-D-thiogalactopyranoside for induction of recombinant proteins at a point where an optical density at 600nm reached 0.7 (final concentration, 0.1 mM). After cultivation at 20°C for 18 hr, the cells were collected with a centrifuge, and then suspended in 10 mM Tris-HCl (pH 7.5), containing 0.5 M NaCl, 1 mM DTT, and 1 mM PMSF. The resultant suspension was sonicated for 10 min, then admixed with 10%(w/v) Triton X-100 (final concentration, 1%), and stirred at 4°C for 30 min. The mixture was centrifuged at 15,000Xg for 30 min, and the resulting supernatant was subjected to affinity chromatography on lactose agarose gels to isolate purified recombinant proteins.

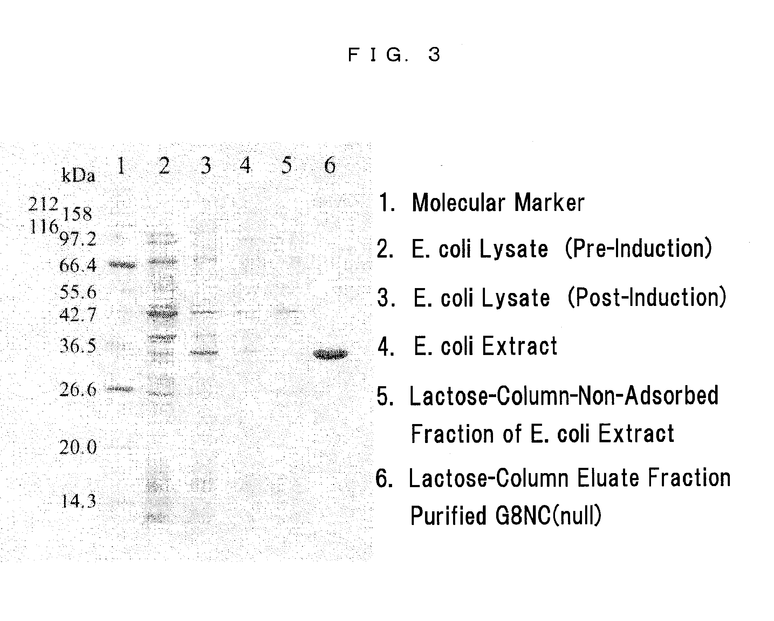

As a result, recombinant protein samples with high purity were obtained in comparatively good yields. The resultant electrophoretic patterns of recombinant protein products are shown in FIG. 3. SDS-PAGE conditions were as follows: Gel, Acrylamide-BIS (12% gel); buffer for electrophoresis, 25mM Tris-192mM glycine-0.1% SDS; electrical conditions, 180V, 45 min.; staining, CBB, 60°C/30 min. Samples for electrophoresis were adsorbed on Strata Clean^{™} Resin (Stratagene), treated with 1×sample buffer (62.5mM Tris-HCl, pH6.8, 2%(w/v) SDS, 5%(W/V) 2-ME, glycerol) to make the mixture 0.2 mg/ml, thermally treated at 98°C/3 min, and then subjected to electrophoresis at about 2µg (protein) per lane.

The purified modified galectin 8 protein, G8NC(null), was stably preservable at 4°C for at least 9 weeks while most of wild type galectin 8 (M-type, G8(M)) was decomposed within 1 week under the same storage conditions. This decomposition is thought to be caused by an action of E. coli-derived proteases contained in the purified galectin sample.

### Example 2

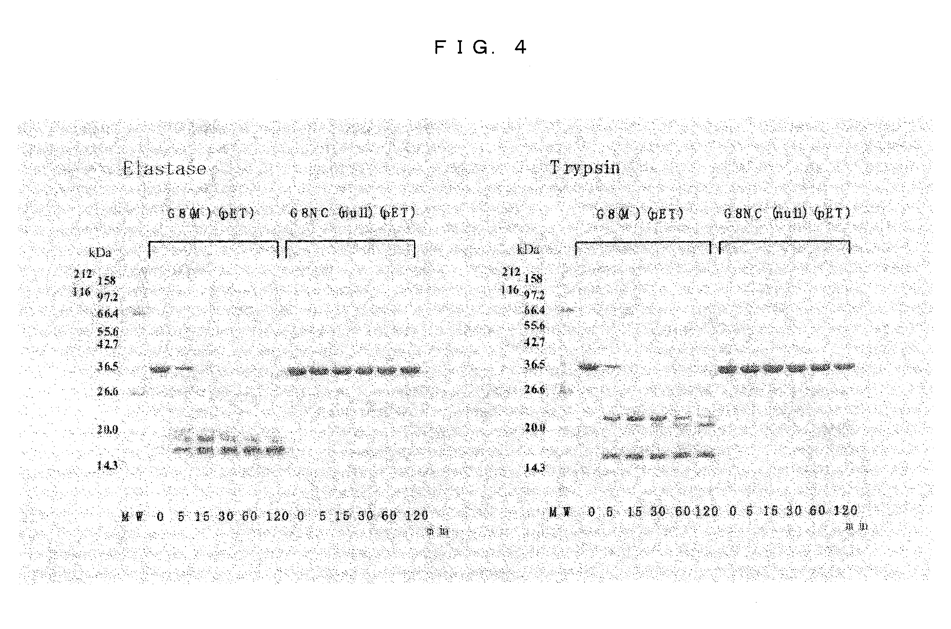

The susceptibility to proteases existing in human tissue was examined between wild type galectin 8 (M-type, G8(M); isoform with a short linker peptide) and G8NC(null) for comparison. To the galectins dissolved in PBS was added elastase or trypsin at 1/100 (weight ratio), and the mixture was incubated at 37°C. Most of G8(M) was decomposed within 15 min in either case while G8NC(null) was scarcely degraded even after the passage of 2 hr (see FIG. 4).

### Example 3

In order to examine how incorporation of the mutation into wild type galectin 8 affects galectin 8 bioactivity, assays were done for effects on peripheral blood neutrophil adhesion and also for effects on superoxide production in neutrophils.

### (1) In Vitro Cell Adhesion Assay

A peripheral blood leukocyte suspension from healthy volunteers was subjected to discontinuous Percoll (Amersham Pharmacia Biotech) density-gradient separation to isolate neutrophils. Contaminant erythrocytes were lysed with water for 20 sec, and the neutrophil suspension admixed with 2XPBS (neutrophil suspension/2XPBS = 1/1 volume/volume) and RPMI-1640 containing 10%FBS (neutrophil suspension/10%FBS-added RPMI-1640 = 1/4 volume/volume). After centrifugation, a cell suspension was reconstructed in RPMI-1640 containing 10%FBS. The separated cells were dispensed into three 24-well tissue culture plates (2.5×10⁵ cells/0.45ml medium/well). After addition of an aliquot (50 µl) of assay sample, cell adhesion was performed at 37°C for 60 min. At the end of the incubation period, insecurely bound cells were removed by vigorous pipetting, followed by washing with PBS. Bound cells were treated with 0.25% trypsin/0.5mM EDTA at 37°C for 10 min, then collected, and sonicated in 50mM sodium phosphate buffer (pH7.4) containing 2M NaCl and 2mM EDTA. Sonicated samples and standards (calf thymus DNA) were analyzed for their DNA content. DNA concentration was assessed via the method described by Labarca and Paigen (Anal. Biochem., 102, 344-352 (1980)).

### (2) Quantitation of Superoxide Generation

O₂⁻ generation was determined by the method described by Yamaoka et al. (J. Immunol., 154, 3479-3487 (1995)). Purified neutrophils were suspended in PBS containing 0.5mM MgCl₂, 0.8mM CaCl₂ and 7.5mM glucose, and an aliquot of the resultant neutrophil suspension (2.5× 10⁶/2ml) was placed in a cuvette. The cuvette also contained horse heart cytochrome c (75µM). The reaction was performed in the presence or absence of cytochalasin B (5 µg/ml). In comparative experiments, superoxide dismutase (SOD, 50 µg/ml) was added. After pre-incubation at 37°C for 5 min, cells were stimulated and absorbance was monitored at 550 nm. The activity of O₂⁻ production was calculated using a molar absorbance coefficient of 20.5 × 10³M⁻¹ cm⁻¹.

### (3) Results

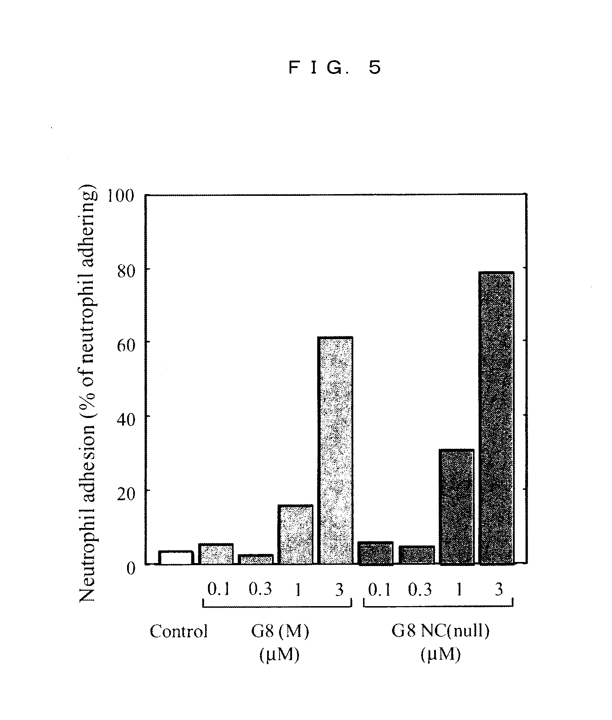

Attachment of neutrophils to culture dishes was examined in the presence of 10% serum. As a result, G8NC(null) exerted higher activity than G8(M) (see FIG. 5).
In addition, G8NC(null) nearly promoted neutrophil superoxide production as much as G8(M) did (see Table 1).

**Table 1**

| Additives | Superoxide production (O₂⁻ nmoles/1 x 10⁶ cells/min) | |
|---|---|---|
| | - cytochalasin B | + cytochalasin B |
| None | 0.00 | 0.00 |
| fMLP, 0.2 µM | 2.14 | 8.43 |
| G8(M), 0.3 µM | - | 2.60 |
| G8(M), 1 µM | 1.83 | 5.40 |
| G8NC(null), 0.3 µM | - | 2.72 |
| GBNC(nulll), 1 µM | 2.04 | 5.88 |

### Example 4

For the purpose of studying galectin 8 (Gal-8) bioactivities on endotoxin and endotoxin-related bioresponse phenomena, modified galectin 8 (stabilized galectin 8) was used to examine its exerted actions and effects.

BALB/c mice (♀, 6-week-old) received an intraperitoneal (i.p.) injection of lipopolysaccharide (LPS) wherein LPS is derived from E. coli 0111:B4, Sigma-Aldrich) at a dose of 4µg. To study the effect of modified galectin 8 protein (Gal-8 mutein), h-G89NC(null), mice received i.p. injections of recombinant human Gal-8 (modified galectin 8 protein: GBNC(null); in the drawing and table, written as "G8" or "Galectin-8"; 80µg) in conjunction with LPS (4µg).

Blood was collected diachronically from the orbital venous plexus of the mouse and serum samples were prepared. Thereafter, the samples were subjected to ELISA (Pierce Endogen) for cytokine assay. The level of serum TNF-α was assayed 1 hr, the level of serum IL-12(p70) 3 hr, and the level of serum IFN-γ 6 and 12 hr, respectively, after challenge with LPS and contemporaneous administration of LPS and Gal-8 (G8NC(null)).

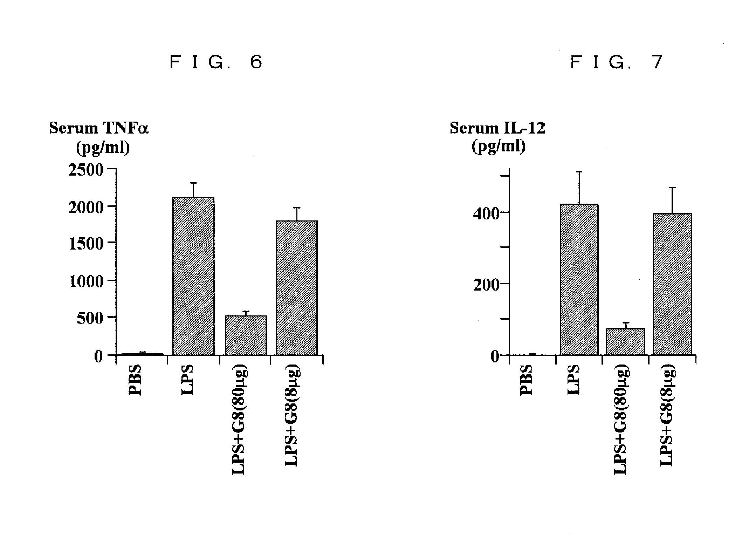

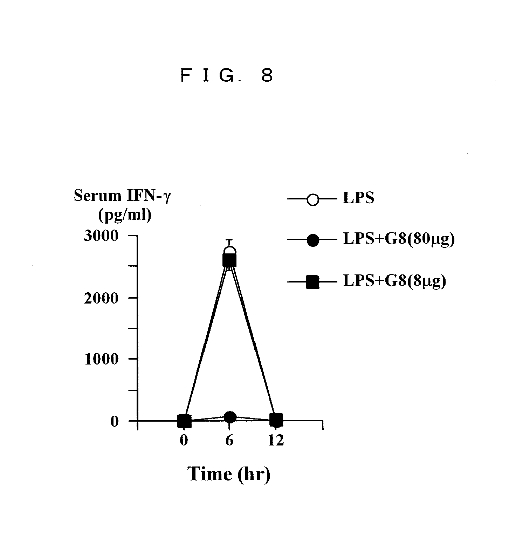

The assay results are shown in FIGs. 6 to 8. The data indicate that co-administration of LPS and Gal-8 (G8NC(null)) resulted in inhibition of TNF-α, IL-12(p70), and IFN-γ production, in comparison with administration of LPS. Since these suggested that Gal-8 might inhibit LPS-induced inflammation, the generalized Shwartzman reaction, a model for LPS-induced endotoxin shock, was used to examine Gal-8 mediated suppressive effects on endotoxin shock.

In this model a lethal shock response is elicited by sequential priming with a small amount of LPS (5µ g/mouse) i.p. followed by challenge with LPS (65*µ*g/mouse) within 24 hr after the first priming. When Gal-8 (G8NC(null)) was administered at 80 µg/mouse contemporaneously with the first priming with LPS, contemporaneously Gal-8 (G8NC(null))-administered 5 mice had an escape from shock-induced death and survived among 6 animals while all animals administered with LPS alone died due to shock. The test results are shown in Table 2.

**Table 2 Effect of Galectin-8 on Shwartzman reaction**

| Administration | Sensitization (1^{st}) i.p. | Challenge (2n^{d}) i.v. | Survival |
|---|---|---|---|
| 1. PBS | PBS | LPS (65µg) | 6/6 |
| 2. PBS | LPS (5µg) | LPS (65µg) | 0/6 |
| 3. Galectin-8 (80µg) | LPS (5µg) | LPS (65µg) | 5/6 (83.3%) |

From the foregoing, it has been found that recombinant human Gal-8 (G8NC(null)) suppresses LPS-induced production of TNF-α, IL-12(p70), and IFN-γ, and has the efficacy of preventing endotoxin shock. That is, galectin 8 (Gal-8) suppresses LPS-induced production of TNF-α, IL-12(p70), and IFN-γ, and is active in prevention of endotoxin shock.

LPS is a component characteristic of Gram-negative bacteria, which is toxic against animals and called "endotoxin". Although LPS is ordinarily bonded firmly to the cell wall, it is known that LPS is released mainly when bacteria are lysed, and induces a variety of responses in the body. Endotoxin is strongly resistant against heat, dryness, and antiseptics, unlike a protein toxin, i.e., "exotoxin". It is considered that a lipid A moiety is mostly responsible for the active effects. However, since lipid A itself is water-insoluble, it is inactive alone. Therefore, it is considered that it is solubilized due to a high hydrophilicity of the polysaccharide moiety whereby a micelle will be formed in an aqueous solution and delivered to act. LPS causes complement activation via the second pathway, thereby leading to a release of cytokines and other immunomodulating substances from macrophages and other cells. These physiologically-active substances are thought to work not only in host defense mechanisms but also for removal or eradication of bacteria. On the other hand, when these cytokines and others become too abundant, they will cause toxicity to hosts, and lead to death due to shock, etc. The known biological activity of LPS includes (1) lethal activity, (2) endotoxin shock, (3) induction of fever, (4) adjuvant activity, (5) activation of macrophages, (6) activation of alternative complement pathways, (7) involvement in membrane permeation of materials, (8) anti-tumor action, (9) receptor for viruses, (10) circulatory organ damage, complement activation, intravascular coagulation, (11) reduction and multiplication of whole leukocytes, hypoglycemia, hypotension, main organ function deficiency and lethal shock, (12) shock induced by cytokines and other substances secreted from lymphocytes, blood endothelial cells and other cells in response to stimulation with LPS, (13) induction of cytokines and autacoids, (14) damage of central nervous cells and muscular cells, (15) activation of coagulation factors and secretion-promoting substances, blood vessel contraction and expansion, inhibition of drug metabolizing enzymes, (16) Shwartzman reaction (non-immunologically inflammatory response associated with hemorrhagic necrosis), (17) blood vessel system damage including serotonin release by platelet aggregation, (18) induction of interferons, (19) involvement in inhibition of migration and membrane permeation of substances, etc. The lethal activity of LPS may include phenomena such as circulatory organ damage caused by a drop in blood pressure and a subnormal cardiac output, and endotoxin shock (for example, when a large amount of antibiotics are administered, bacteria will be disintegrated at one time, and LPS will flow into a blood stream, thereby leading to a state of shock). The endotoxin shock may include, for example, conditions where LPS is released at once from bacteria in a large abscess, thereby resulting in the occurrence of shock, such as a lowering of the blood pressure and occasionally death.

### INDUSTRIAL APPLICABILITY

Modified galectin 8 proteins (Gal-8 muteins) are more resistant against enzymes than wild type Gal-8 proteins. Therefore, the modified galectin 9 proteins are quite useful in effectively utilizing and applying versatile actions and functions owned by wild type galectin 8. It is suggested that wild type galectin 8 induces activities including hemagglutination, induction of cell adhesion such as neutrophil adhesion, integrin α_{M}-binding, proMMP-9 binding, promotion of active form MMP-9 production, promotion of superoxide generation, activity of inducing apoptosis of particular cells, suppressive/inhibitory actions on the growth of tumor cells, and metastasis inhibition and regression of cancers. Accordingly, the modified galectin 8 proteins can be expected to act as advantageously active materials having equivalent galectin 8 activity. Further, when modified galectin 8 proteins are used, the activity of suppressing LPS-induced inflammation, and of suppressing endotoxin shock (including suppression of endotoxin lethality) has been observed. Accordingly, it is suggested that galectin 8 has such activities. Thus, the present invention is utilizable as a tool for not only the development of endotoxin shock suppressants and therapeutic drugs for inflammations and inflammatory diseases, cancers, refractory immune diseases (including autoimmune diseases), and allergic diseases, but also the revelation, research & development of galectin 8 functions.

While the present invention has been described specifically in detail with reference to certain embodiments and examples thereof, it would be apparent that it is possible to practice it in other forms. In light of the disclosure, it will be understood that various modifications and variations are within the spirit and scope of the appended claims.

### <Sequence Listing Free Text>

SEQ ID NO: 1, Description of Artificial Sequence: Polynucleotide for galectin-8 mutein, G8NC(null)
SEQ ID NO: 2, Description of Artificial Sequence: Polynucleotide for galectin-9 mutein
SEQ ID NO: 5, galectin-8 short isoform (hGalectin-8(M))
SEQ ID NO: 7, galectin-8 long isoform (hGalectin-8(L))
SEQ ID NO: 11, Description of Artificial Sequence: Oligonucleotide to act as a primer for PCR
SEQ ID NO: 12, Description of Artificial Sequence: Oligonucleotide to act as a primer for PCR
SEQ ID NO: 13, Description of Artificial Sequence: Oligonucleotide to act as a primer for PCR
SEQ ID NO: 14, Description of Artificial Sequence: Oligonucleotide to act as a primer for PCR

## Claims

1. A protein, or a salt thereof, comprising a functional mutant galectin 8 protein with an amino acid sequence that differs from an amino acid sequence of wild type galectin 8 or a protein with substantially equivalent galectin 8 activity wherein said functional mutant galectin 8 protein has a modified link peptide or a modified site or region in the vicinity of the galectin 8 link peptide.

2. The protein, or a salt thereof, according to claim 1, wherein said functional mutant galectin 8 protein has not only a modified sequence that differs from an amino acid sequence of wild type galectin 8 or a protein with substantially equivalent galectin 8 activity by the deletion, substitution or addition of at least one or more amino acid residues at a link peptide or a site or region in the vicinity of the galectin 8 link peptide but also altered susceptibility to degradation of said galectin 8 link peptide as compared to wild type galectin 8.

3. The protein, or a salt thereof, according to claim 1 or 2, wherein said protein with substantially equivalent galectin 8 activity is at least 70% or more homologous to wild type galectin 8 at an amino acid level.

4. The protein, or a salt thereof, according to any of claims 1 to 3, wherein
(1) the N-terminal carbohydrate recognition domain (NCRD) of wild type galectin 8 or a polypeptide with substantially equivalent galectin 8 NCRD activity is coupled with
(2) the C-terminal carbohydrate recognition domain (CCRD) of wild type galectin 8 or a polypeptide with substantially equivalent galectin 8 CCRD activity via
(3) a modified link peptide with an amino acid sequence that differs from an amino acid sequence of wild type galectin 8 link peptide by the deletion, substitution or addition of at least one or more amino acid residues at a galectin 8 link peptide region.

5. The protein, or a salt thereof, according to any of claims 1 to 4, wherein
(1) a member selected from the group consisting of a polypeptide having an amino acid sequence of SEQ ID NO: 3, a polypeptide having not only substantially equivalent SEQ ID NO: 3 polypeptide activity but also an amino acid sequence at least 70% homologous to SEQ ID NO: 3, and a polypeptide having a mutant amino acid sequence that differs from an amino acid sequence of SEQ ID NO: 3 by the deletion, substitution or addition of at least 1 to 8 amino acid residues on the SEQ ID NO: 3 amino acid sequence is coupled with
(2) a member selected from the group consisting of a polypeptide having an amino acid sequence of SEQ ID NO: 4, a polypeptide having not only substantially equivalent SEQ ID NO: 4 polypeptide activity but also an amino acid sequence at least 70% homologous to SEQ ID NO: 4, and a polypeptide having a mutant amino acid sequence that differs from an amino acid sequence of SEQ ID NO: 4 by the deletion, substitution or addition of at least 1 to 21 amino acid residues on the SEQ ID NO: 4 amino acid sequence via
(3) a modified link peptide with an amino acid sequence that differs from an amino acid sequence of a member selected from the group consisting of SEQ ID NOs 9 and 10 by the deletion, substitution or addition of at least one or more amino acid residues on any amino acid sequence of SEQ ID NOs 9 to 10, provided that the deletion of residues 29 to 70 on SEQ ID NO: 10 is excluded.

6. A nucleic acid molecule comprising a nucleotide sequence encoding the protein according to any of claims 1 to 5.

7. The nucleic acid molecule according to claim 6, wherein said molecule is a polynucleotide.

8. The nucleic acid molecule according to claim 6 or 7, wherein said molecule is DNA or RNA.

9. A recombinant vector comprising the nucleic acid molecule according to any of claims 6 to 8.

10. The recombinant vector according to claim 9 wherein said vector comprises a nucleotide sequence coding for a protein marker and/or a peptide marker in combination with the nucleic acid molecule according to any of claims 6 to 8.

11. A transformed or transfected cell carrying the nucleic acid molecule according to any of claims 6 to 8 or the recombinant vector according to claim 9 or 10.

12. The transformed or transfected cell according to claim 11, wherein said host cell is procaryotic or eucaryotic.

13. A pharmaceutical drug comprising an effective amount of at least one member selected from the group consisting of the protein according to any of claims 1 to 5, the nucleic acid molecule according to any of claims 6 to 8, the recombinant vector according to claim 9 or 10, and the transformed or transfected cell according to claim 11 or 12.

14. The pharmaceutical drug according to claim 13 which is an immunoregulator, immunomodulator, anti-inflammatory agent, or depressant against endotoxin shock.

15. The pharmaceutical drug according to claim 13 which is an antineoplastic, antitumor agent, or anti-metastatic agent.

16. The pharmaceutical drug according to claim 13, which is a therapeutic or prophylactic agent for a pathological condition, disease or disorder wherein said agent utilizes at least one Gal-8 mutein activity selected form the group consisting of (1) hemagglutination, (2) induction of apoptosis, (3) induction of cell adhesion, (4) integrin α_{M}-binding, (5) proMMP-9 binding, proMMP-9 activation, or promotion of active form MMP-9 production, (6) promotion of superoxide production, (7) suppression of LPS-induced inflammation, (8) suppression of LPS-induced TNF-α, IL-12, and/or IFN-γ production, and (9) inhibition of endotoxin shock.

17. An assay or test reagent comprising an effective amount of at least one member selected from the group consisting of the protein according to any of claims 1 to 5, the nucleic acid molecule according to any of claims 6 to 8, the recombinant vector according to claim 9 or 10, and the transformed or transfected cell according to claim 11 or 12.


SEQUENCE LISTING

<110>  GALPHARMA Co., Ltd.

<120>  Novel Galectin-8 Muteins and Use Thereof

<130>  GL-06PCT

<150>  JP 2004-175086
<151>  2004-06-14

<160>  14

<170>  PatentIn version 3.1

<210>  1
<211>  876
<212>  DNA
<213>  Artificial Sequence

<220>
<223>  Polynucleodide for galectin-8 mutein

<220>
<221>  CDS
<222>  (1)..(876)
<223>  G8NC(null)

<400>  1
atg atg ttg tcc tta aac aac cta cag aat atc atc tat aac ccg gta       48
Met Met Leu Ser Leu Asn Asn Leu Gln Asn Ile Ile Tyr Asn Pro Val
1               5                   10                  15

atc ccg ttt gtt ggc acc att cct gat cag ctg gat cct gga act ttg       96
Ile Pro Phe Val Gly Thr Ile Pro Asp Gln Leu Asp Pro Gly Thr Leu
            20                  25                  30

att gtg ata cgt ggg cat gtt cct agt gac gca gac aga ttc cag gtg      144
Ile Val Ile Arg Gly His Val Pro Ser Asp Ala Asp Arg Phe Gln Val
        35                  40                  45

gat ctg cag aat ggc agc agc atg aaa cct cga gcc gat gtg gcc ttt      192
Asp Leu Gln Asn Gly Ser Ser Met Lys Pro Arg Ala Asp Val Ala Phe
    50                  55                  60

cat ttc aat cct cgt ttc aaa agg gcc ggc tgc att gtt tgc aat act      240
His Phe Asn Pro Arg Phe Lys Arg Ala Gly Cys Ile Val Cys Asn Thr
65                  70                  75                  80

ttg ata aat gaa aaa tgg gga cgg gaa gag atc acc tat gac acg cct      288
Leu Ile Asn Glu Lys Trp Gly Arg Glu Glu Ile Thr Tyr Asp Thr Pro
                85                  90                  95

ttc aaa aga gaa aag tct ttt gag atc gtg att atg gtg ctg aag gac      336
Phe Lys Arg Glu Lys Ser Phe Glu Ile Val Ile Met Val Leu Lys Asp

            100                 105                 110

aaa ttc cag gtg gct gta aat gga aaa cat act ctg ctc tat ggc cac 384
Lys Phe Gln Val Ala Val Asn Gly Lys His Thr Leu Leu Tyr Gly His
        115                 120                 125

agg atc ggc cca gag aaa ata gac act ctg ggc att tat ggc aaa gtg 432
Arg Ile Gly Pro Glu Lys Ile Asp Thr Leu Gly Ile Tyr Gly Lys Val
    130                 135                 140

aat att cac tca att ggt ttt agc ttc agc tcg cat atg agg ctg cca 480
Asn Ile His Ser Ile Gly Phe Ser Phe Ser Ser His Met Arg Leu Pro
145                 150                 155                 160

ttc gct gca agg ttg aac acc ccc atg ggc cct gga cga act gtc gtc 528
Phe Ala Ala Arg Leu Asn Thr Pro Met Gly Pro Gly Arg Thr Val Val
                165                 170                 175

gtt aaa gga gaa gtg aat gca aat gcc aaa agc ttt aat gtt gac cta 576
Val Lys Gly Glu Val Asn Ala Asn Ala Lys Ser Phe Asn Val Asp Leu
            180                 185                 190

cta gca gga aaa tca aag gat att gct cta cac ttg aac cca cgc ctg 624
Leu Ala Gly Lys Ser Lys Asp Ile Ala Leu His Leu Asn Pro Arg Leu
        195                 200                 205

aat att aaa gca ttt gta aga aat tct ttt ctt cag gag tcc tgg gga 672
Asn Ile Lys Ala Phe Val Arg Asn Ser Phe Leu Gln Glu Ser Trp Gly
    210                 215                 220

gaa gaa gag aga aat att acc tct ttc cca ttt agt cct ggg atg tac 720
Glu Glu Glu Arg Asn Ile Thr Ser Phe Pro Phe Ser Pro Gly Met Tyr
225                 230                 235                 240

ttt gag atg ata att tat tgt gat gtt aga gaa ttc aag gtt gca gta 768
Phe Glu Met Ile Ile Tyr Cys Asp Val Arg Glu Phe Lys Val Ala Val
                245                 250                 255

aat ggc gta cac agc ctg gag tac aaa cac aga ttt aaa gag ctc agc 816
Asn Gly Val His Ser Leu Glu Tyr Lys His Arg Phe Lys Glu Leu Ser
            260                 265                 270

agt att gac acg ctg gaa att aat gga gac atc cac tta ctg gaa gta 864
Ser Ile Asp Thr Leu Glu Ile Asn Gly Asp Ile His Leu Leu Glu Val
        275                 280                 285

agg agc tgg tag 876
Arg Ser Trp
    290

<210> 2
<211> 291
<212> PRT
<213> Artificial Sequence

<220>
<223>  Polynucleodide for galectin-8 mutein

<400>  2

Met Met Leu Ser Leu Asn Asn Leu Gln Asn Ile Ile Tyr Asn Pro Val
1               5                   10                  15

Ile Pro Phe Val Gly Thr Ile Pro Asp Gln Leu Asp Pro Gly Thr Leu
            20                  25                  30

Ile Val Ile Arg Gly His Val Pro Ser Asp Ala Asp Arg Phe Gln Val
        35                  40                  45

Asp Leu Gln Asn Gly Ser Ser Met Lys Pro Arg Ala Asp Val Ala Phe
    50                  55                  60

His Phe Asn Pro Arg Phe Lys Arg Ala Gly Cys Ile Val Cys Asn Thr
65                  70                  75                  80

Leu Ile Asn Glu Lys Trp Gly Arg Glu Glu Ile Thr Tyr Asp Thr Pro
                85                  90                  95

Phe Lys Arg Glu Lys Ser Phe Glu Ile Val Ile Met Val Leu Lys Asp
            100                 105                 110

Lys Phe Gln Val Ala Val Asn Gly Lys His Thr Leu Leu Tyr Gly His
        115                 120                 125

Arg Ile Gly Pro Glu Lys Ile Asp Thr Leu Gly Ile Tyr Gly Lys Val
    130                 135                 140

Asn Ile His Ser Ile Gly Phe Ser Phe Ser Ser His Met Arg Leu Pro
145                 150                 155                 160

Phe Ala Ala Arg Leu Asn Thr Pro Met Gly Pro Gly Arg Thr Val Val
                165                 170                 175

Val Lys Gly Glu Val Asn Ala Asn Ala Lys Ser Phe Asn Val Asp Leu
            180                 185                 190

Leu Ala Gly Lys Ser Lys Asp Ile Ala Leu His Leu Asn Pro Arg Leu
        195                 200                 205

Asn Ile Lys Ala Phe Val Arg Asn Ser Phe Leu Gln Glu Ser Trp Gly
    210                 215                 220

Glu Glu Glu Arg Asn Ile Thr Ser Phe Pro Phe Ser Pro Gly Met Tyr
225                 230                 235                 240

Phe Glu Met Ile Ile Tyr Cys Asp Val Arg Glu Phe Lys Val Ala Val
                245                 250                 255

Asn Gly Val His Ser Leu Glu Tyr Lys His Arg Phe Lys Glu Leu Ser
            260                 265                 270

Ser Ile Asp Thr Leu Glu Ile Asn Gly Asp Ile His Leu Leu Glu Val
        275                 280                 285

Arg Ser Trp
    290

<210>  3
<211>  155
<212>  PRT
<213>  Homo sapiens

<400>  3

Met Met Leu Ser Leu Asn Asn Leu Gln Asn Ile Ile Tyr Asn Pro Val
1               5                   10                  15

Ile Pro Phe Val Gly Thr Ile Pro Asp Gln Leu Asp Pro Gly Thr Leu
            20                  25                  30

Ile Val Ile Arg Gly His Val Pro Ser Asp Ala Asp Arg Phe Gln Val
        35                  40                  45

Asp Leu Gln Asn Gly Ser Ser Met Lys Pro Arg Ala Asp Val Ala Phe
    50                  55                  60

His Phe Asn Pro Arg Phe Lys Arg Ala Gly Cys Ile Val Cys Asn Thr
65                  70                  75                  80

Leu Ile Asn Glu Lys Trp Gly Arg Glu Glu Ile Thr Tyr Asp Thr Pro
                85                  90                  95

Phe Lys Arg Glu Lys Ser Phe Glu Ile Val Ile Met Val Leu Lys Asp
            100                 105                 110

Lys Phe Gln Val Ala Val Asn Gly Lys His Thr Leu Leu Tyr Gly His
        115                 120                 125

Arg Ile Gly Pro Glu Lys Ile Asp Thr Leu Gly Ile Tyr Gly Lys Val
    130                 135                 140

Asn Ile His Ser Ile Gly Phe Ser Phe Ser Ser
145                 150                 155

<210>  4
<211>  134
<212>  PRT
<213>  Homo sapiens

<400>  4

Arg Leu Pro Phe Ala Ala Arg Leu Asn Thr Pro Met Gly Pro Gly Arg
1               5                   10                  15

Thr Val Val Val Lys Gly Glu Val Asn Ala Asn Ala Lys Ser Phe Asn
            20                  25                  30

Val Asp Leu Leu Ala Gly Lys Ser Lys Asp Ile Ala Leu His Leu Asn
        35                  40                  45

Pro Arg Leu Asn Ile Lys Ala Phe Val Arg Asn Ser Phe Leu Gln Glu
    50                  55                  60

Ser Trp Gly Glu Glu Glu Arg Asn Ile Thr Ser Phe Pro Phe Ser Pro
65                  70                  75                  80

Gly Met Tyr Phe Glu Met Ile Ile Tyr Cys Asp Val Arg Glu Phe Lys
                85                  90                  95

Val Ala Val Asn Gly Val His Ser Leu Glu Tyr Lys His Arg Phe Lys
            100                 105                 110

Glu Leu Ser Ser Ile Asp Thr Leu Glu Ile Asn Gly Asp Ile His Leu
        115                 120                 125

Leu Glu Val Arg Ser Trp

    130

<210> 5
<211> 954
<212> DNA
<213> Homo sapiens

<220>
<221> CDS
<222> (1)..(954)
<223> galectin-8 short isoform (hGalectin-8(M))

<400> 5
atg atg ttg tcc tta aac aac cta cag aat atc atc tat aac ccg gta 48
Met Met Leu Ser Leu Asn Asn Leu Gln Asn Ile Ile Tyr Asn Pro Val
1               5                   10                  15

atc ccg ttt gtt ggc acc att cct gat cag ctg gat cct gga act ttg 96
Ile Pro Phe Val Gly Thr Ile Pro Asp Gln Leu Asp Pro Gly Thr Leu
            20                  25                  30

att gtg ata cgt ggg cat gtt cct agt gac gca gac aga ttc cag gtg 144
Ile Val Ile Arg Gly His Val Pro Ser Asp Ala Asp Arg Phe Gln Val
        35                  40                  45

gat ctg cag aat ggc agc agc atg aaa cct cga gcc gat gtg gcc ttt 192
Asp Leu Gln Asn Gly Ser Ser Met Lys Pro Arg Ala Asp Val Ala Phe
    50                  55                  60

cat ttc aat cct cgt ttc aaa agg gcc ggc tgc att gtt tgc aat act 240
His Phe Asn Pro Arg Phe Lys Arg Ala Gly Cys Ile Val Cys Asn Thr
65                  70                  75                  80

ttg ata aat gaa aaa tgg gga cgg gaa gag atc acc tat gac acg cct 288
Leu Ile Asn Glu Lys Trp Gly Arg Glu Glu Ile Thr Tyr Asp Thr Pro
                85                  90                  95

ttc aaa aga gaa aag tct ttt gag atc gtg att atg gtg ctg aag gac 336
Phe Lys Arg Glu Lys Ser Phe Glu Ile Val Ile Met Val Leu Lys Asp
            100                 105                 110

aaa ttc cag gtg gct gta aat gga aaa cat act ctg ctc tat ggc cac 384
Lys Phe Gln Val Ala Val Asn Gly Lys His Thr Leu Leu Tyr Gly His
        115                 120                 125

agg atc ggc cca gag aaa ata gac act ctg ggc att tat ggc aaa gtg 432
Arg Ile Gly Pro Glu Lys Ile Asp Thr Leu Gly Ile Tyr Gly Lys Val
    130                 135                 140

aat att cac tca att ggt ttt agc ttc agc tcg gac tta caa agt acc 480
Asn Ile His Ser Ile Gly Phe Ser Phe Ser Ser Asp Leu Gln Ser Thr
145                 150                 155                 160

caa gca tct agt ctg gaa ctg aca gag ata agt aga gaa aat gtt cca 528

Gln Ala Ser Ser Leu Glu Leu Thr Glu Ile Ser Arg Glu Asn Val Pro
                165                 170                 175

aag tct ggc acg ccc cag ctt agg ctg cca ttc gct gca agg ttg aac      576
Lys Ser Gly Thr Pro Gln Leu Arg Leu Pro Phe Ala Ala Arg Leu Asn
            180                 185                 190

acc ccc atg ggc cct gga cga act gtc gtc gtt aaa gga gaa gtg aat      624
Thr Pro Met Gly Pro Gly Arg Thr Val Val Val Lys Gly Glu Val Asn
        195                 200                 205

gca aat gcc aaa agc ttt aat gtt gac cta cta gca gga aaa tca aag      672
Ala Asn Ala Lys Ser Phe Asn Val Asp Leu Leu Ala Gly Lys Ser Lys
    210                 215                 220

gat att gct cta cac ttg aac cca cgc ctg aat att aaa gca ttt gta      720
Asp Ile Ala Leu His Leu Asn Pro Arg Leu Asn Ile Lys Ala Phe Val
225                 230                 235                 240

aga aat tct ttt ctt cag gag tcc tgg gga gaa gaa gag aga aat att      768
Arg Asn Ser Phe Leu Gln Glu Ser Trp Gly Glu Glu Glu Arg Asn Ile
                245                 250                 255

acc tct ttc cca ttt agt cct ggg atg tac ttt gag atg ata att tat      816
Thr Ser Phe Pro Phe Ser Pro Gly Met Tyr Phe Glu Met Ile Ile Tyr
            260                 265                 270

tgt gat gtt aga gaa ttc aag gtt gca gta aat ggc gta cac agc ctg      864
Cys Asp Val Arg Glu Phe Lys Val Ala Val Asn Gly Val His Ser Leu
        275                 280                 285

gag tac aaa cac aga ttt aaa gag ctc agc agt att gac acg ctg gaa      912
Glu Tyr Lys His Arg Phe Lys Glu Leu Ser Ser Ile Asp Thr Leu Glu
    290                 295                 300

att aat gga gac atc cac tta ctg gaa gta agg agc tgg tag              954
Ile Asn Gly Asp Ile His Leu Leu Glu Val Arg Ser Trp
305                 310                 315


<210>  6
<211>  317
<212>  PRT
<213>  Homo sapiens

<400>  6

Met Met Leu Ser Leu Asn Asn Leu Gln Asn Ile Ile Tyr Asn Pro Val
1               5                   10                  15


Ile Pro Phe Val Gly Thr Ile Pro Asp Gln Leu Asp Pro Gly Thr Leu
            20                  25                  30


Ile Val Ile Arg Gly His Val Pro Ser Asp Ala Asp Arg Phe Gln Val

35                  40                  45

Asp Leu Gln Asn Gly Ser Ser Met Lys Pro Arg Ala Asp Val Ala Phe
    50                  55                  60

His Phe Asn Pro Arg Phe Lys Arg Ala Gly Cys Ile Val Cys Asn Thr
65                  70                  75                  80

Leu Ile Asn Glu Lys Trp Gly Arg Glu Glu Ile Thr Tyr Asp Thr Pro
                85                  90                  95

Phe Lys Arg Glu Lys Ser Phe Glu Ile Val Ile Met Val Leu Lys Asp
            100                 105                 110

Lys Phe Gln Val Ala Val Asn Gly Lys His Thr Leu Leu Tyr Gly His
        115                 120                 125

Arg Ile Gly Pro Glu Lys Ile Asp Thr Leu Gly Ile Tyr Gly Lys Val
    130                 135                 140

Asn Ile His Ser Ile Gly Phe Ser Phe Ser Ser Asp Leu Gln Ser Thr
145                 150                 155                 160

Gln Ala Ser Ser Leu Glu Leu Thr Glu Ile Ser Arg Glu Asn Val Pro
                165                 170                 175

Lys Ser Gly Thr Pro Gln Leu Arg Leu Pro Phe Ala Ala Arg Leu Asn
            180                 185                 190

Thr Pro Met Gly Pro Gly Arg Thr Val Val Val Lys Gly Glu Val Asn
        195                 200                 205

Ala Asn Ala Lys Ser Phe Asn Val Asp Leu Leu Ala Gly Lys Ser Lys
    210                 215                 220

Asp Ile Ala Leu His Leu Asn Pro Arg Leu Asn Ile Lys Ala Phe Val
225                 230                 235                 240

Arg Asn Ser Phe Leu Gln Glu Ser Trp Gly Glu Glu Glu Arg Asn Ile
                245                 250                 255

Thr Ser Phe Pro Phe Ser Pro Gly Met Tyr Phe Glu Met Ile Ile Tyr

260                 265                 270

Cys Asp Val Arg Glu Phe Lys Val Ala Val Asn Gly Val His Ser Leu
        275                 280                 285

Glu Tyr Lys His Arg Phe Lys Glu Leu Ser Ser Ile Asp Thr Leu Glu
    290                 295                 300

Ile Asn Gly Asp Ile His Leu Leu Glu Val Arg Ser Trp
305                 310                 315

<210>  7
<211>  1080
<212>  DNA
<213>  Homo sapiens

<220>
<221>  CDS
<222>  (1)..(1080)
<223>  galectin-8 long isoform (hGalectin-8(L))

<400>  7
atg atg ttg tcc tta aac aac cta cag aat atc atc tat aac ccg gta       48
Met Met Leu Ser Leu Asn Asn Leu Gln Asn Ile Ile Tyr Asn Pro Val
1               5                   10                  15

atc ccg ttt gtt ggc acc att cct gat cag ctg gat cct gga act ttg       96
Ile Pro Phe Val Gly Thr Ile Pro Asp Gln Leu Asp Pro Gly Thr Leu
            20                  25                  30

att gtg ata cgt ggg cat gtt cct agt gac gca gac aga ttc cag gtg      144
Ile Val Ile Arg Gly His Val Pro Ser Asp Ala Asp Arg Phe Gln Val
        35                  40                  45

gat ctg cag aat ggc agc agc atg aaa cct cga gcc gat gtg gcc ttt      192
Asp Leu Gln Asn Gly Ser Ser Met Lys Pro Arg Ala Asp Val Ala Phe
    50                  55                  60

cat ttc aat cct cgt ttc aaa agg gcc ggc tgc att gtt tgc aat act      240
His Phe Asn Pro Arg Phe Lys Arg Ala Gly Cys Ile Val Cys Asn Thr
65                  70                  75                  80

ttg ata aat gaa aaa tgg gga cgg gaa gag atc acc tat gac acg cct      288
Leu Ile Asn Glu Lys Trp Gly Arg Glu Glu Ile Thr Tyr Asp Thr Pro
                85                  90                  95

ttc aaa aga gaa aag tct ttt gag atc gtg att atg gtg ctg aag gac      336
Phe Lys Arg Glu Lys Ser Phe Glu Ile Val Ile Met Val Leu Lys Asp
            100                 105                 110

aaa ttc cag gtg gct gta aat gga aaa cat act ctg ctc tat ggc cac      384

Lys Phe Gln Val Ala Val Asn Gly Lys His Thr Leu Leu Tyr Gly His
        115                 120                 125

agg atc ggc cca gag aaa ata gac act ctg ggc att tat ggc aaa gtg 432
Arg Ile Gly Pro Glu Lys Ile Asp Thr Leu Gly Ile Tyr Gly Lys Val
    130                 135                 140

aat att cac tca att ggt ttt agc ttc agc tcg gac tta caa agt acc 480
Asn Ile His Ser Ile Gly Phe Ser Phe Ser Ser Asp Leu Gln Ser Thr
145                 150                 155                 160

caa gca tct agt ctg gaa ctg aca gag ata agt aga gaa aat gtt cca 528
Gln Ala Ser Ser Leu Glu Leu Thr Glu Ile Ser Arg Glu Asn Val Pro
                165                 170                 175

aag tct ggc acg ccc cag ctt cct agt aat aga gga gga gac att tct 576
Lys Ser Gly Thr Pro Gln Leu Pro Ser Asn Arg Gly Gly Asp Ile Ser
            180                 185                 190

aaa atc gca ccc aga act gtc tac acc aag agc aaa gat tcg act gtc 624
Lys Ile Ala Pro Arg Thr Val Tyr Thr Lys Ser Lys Asp Ser Thr Val
        195                 200                 205

aat cac act ttg act tgc acc aaa ata cca cct atg aac tat gtg tca 672
Asn His Thr Leu Thr Cys Thr Lys Ile Pro Pro Met Asn Tyr Val Ser
    210                 215                 220

aag agg ctg cca ttc gct gca agg ttg aac acc ccc atg ggc cct gga 720
Lys Arg Leu Pro Phe Ala Ala Arg Leu Asn Thr Pro Met Gly Pro Gly
225                 230                 235                 240

cga act gtc gtc gtt aaa gga gaa gtg aat gca aat gcc aaa agc ttt 768
Arg Thr Val Val Val Lys Gly Glu Val Asn Ala Asn Ala Lys Ser Phe
                245                 250                 255

aat gtt gac cta cta gca gga aaa tca aag gat att gct cta cac ttg 816
Asn Val Asp Leu Leu Ala Gly Lys Ser Lys Asp Ile Ala Leu His Leu
            260                 265                 270

aac cca cgc ctg aat att aaa gca ttt gta aga aat tct ttt ctt cag 864
Asn Pro Arg Leu Asn Ile Lys Ala Phe Val Arg Asn Ser Phe Leu Gln
        275                 280                 285

gag tcc tgg gga gaa gaa gag aga aat att acc tct ttc cca ttt agt 912
Glu Ser Trp Gly Glu Glu Glu Arg Asn Ile Thr Ser Phe Pro Phe Ser
    290                 295                 300

cct ggg atg tac ttt gag atg ata att tat tgt gat gtt aga gaa ttc 960
Pro Gly Met Tyr Phe Glu Met Ile Ile Tyr Cys Asp Val Arg Glu Phe
305                 310                 315                 320

aag gtt gca gta aat ggc gta cac agc ctg gag tac aaa cac aga ttt 1008
Lys Val Ala Val Asn Gly Val His Ser Leu Glu Tyr Lys His Arg Phe
                325                 330                 335

aaa gag ctc agc agt att gac acg ctg gaa att aat gga gac atc cac 1056

Lys Glu Leu Ser Ser Ile Asp Thr Leu Glu Ile Asn Gly Asp Ile His
            340                 345                 350

tta ctg gaa gta agg agc tgg tag                                   1080
Leu Leu Glu Val Arg Ser Trp
        355


<210>  8
<211>  359
<212>  PRT
<213>  Homo sapiens

<400>  8

Met Met Leu Ser Leu Asn Asn Leu Gln Asn Ile Ile Tyr Asn Pro Val
1               5                   10                  15


Ile Pro Phe Val Gly Thr Ile Pro Asp Gln Leu Asp Pro Gly Thr Leu
            20                  25                  30


Ile Val Ile Arg Gly His Val Pro Ser Asp Ala Asp Arg Phe Gln Val
        35                  40                  45


Asp Leu Gln Asn Gly Ser Ser Met Lys Pro Arg Ala Asp Val Ala Phe
    50                  55                  60


His Phe Asn Pro Arg Phe Lys Arg Ala Gly Cys Ile Val Cys Asn Thr
65                  70                  75                  80


Leu Ile Asn Glu Lys Trp Gly Arg Glu Glu Ile Thr Tyr Asp Thr Pro
                85                  90                  95


Phe Lys Arg Glu Lys Ser Phe Glu Ile Val Ile Met Val Leu Lys Asp
            100                 105                 110


Lys Phe Gln Val Ala Val Asn Gly Lys His Thr Leu Leu Tyr Gly His
        115                 120                 125


Arg Ile Gly Pro Glu Lys Ile Asp Thr Leu Gly Ile Tyr Gly Lys Val
    130                 135                 140


Asn Ile His Ser Ile Gly Phe Ser Phe Ser Ser Asp Leu Gln Ser Thr
145                 150                 155                 160


Gln Ala Ser Ser Leu Glu Leu Thr Glu Ile Ser Arg Glu Asn Val Pro

                165                 170                 175

Lys Ser Gly Thr Pro Gln Leu Pro Ser Asn Arg Gly Gly Asp Ile Ser
            180                 185                 190

Lys Ile Ala Pro Arg Thr Val Tyr Thr Lys Ser Lys Asp Ser Thr Val
        195                 200                 205

Asn His Thr Leu Thr Cys Thr Lys Ile Pro Pro Met Asn Tyr Val Ser
    210                 215                 220

Lys Arg Leu Pro Phe Ala Ala Arg Leu Asn Thr Pro Met Gly Pro Gly
225                 230                 235                 240

Arg Thr Val Val Val Lys Gly Glu Val Asn Ala Asn Ala Lys Ser Phe
                245                 250                 255

Asn Val Asp Leu Leu Ala Gly Lys Ser Lys Asp Ile Ala Leu His Leu
            260                 265                 270

Asn Pro Arg Leu Asn Ile Lys Ala Phe Val Arg Asn Ser Phe Leu Gln
        275                 280                 285

Glu Ser Trp Gly Glu Glu Glu Arg Asn Ile Thr Ser Phe Pro Phe Ser
    290                 295                 300

Pro Gly Met Tyr Phe Glu Met Ile Ile Tyr Cys Asp Val Arg Glu Phe
305                 310                 315                 320

Lys Val Ala Val Asn Gly Val His Ser Leu Glu Tyr Lys His Arg Phe
                325                 330                 335

Lys Glu Leu Ser Ser Ile Asp Thr Leu Glu Ile Asn Gly Asp Ile His
            340                 345                 350

Leu Leu Glu Val Arg Ser Trp
        355

<210> 9
<211> 28
<212> PRT
<213> Homo sapiens

<400>  9

Asp Leu Gln Ser Thr Gln Ala Ser Ser Leu Glu Leu Thr Glu Ile Ser
1               5                   10                  15

Arg Glu Asn Val Pro Lys Ser Gly Thr Pro Gln Leu
            20                  25

<210>  10
<211>  70
<212>  PRT
<213>  Homo sapiens

<400>  10

Asp Leu Gln Ser Thr Gln Ala Ser Ser Leu Glu Leu Thr Glu Ile Ser
1               5                   10                  15

Arg Glu Asn Val Pro Lys Ser Gly Thr Pro Gln Leu Pro Ser Asn Arg
            20                  25                  30

Gly Gly Asp Ile Ser Lys Ile Ala Pro Arg Thr Val Tyr Thr Lys Ser
        35                  40                  45

Lys Asp Ser Thr Val Asn His Thr Leu Thr Cys Thr Lys Ile Pro Pro
    50                  55                  60

Met Asn Tyr Val Ser Lys
65                  70

<210>  11
<211>  34
<212>  DNA
<213>  Artificial Sequence

<220>
<223>  Oligonucleotide to act as a primer for PCR

<400>  11
cgtcctcata tgatgttgtc cttaaacaac ctac                                34

<210>  12
<211>  33
<212>  DNA
<213>  Artificial Sequence

<220>
<223>  Oligonucleotide to act as a primer for PCR

<400>  12
cgaccgcata tgcgagctga agctaaaacc aat                                    33


<210>  13
<211>  33
<212>  DNA
<213>  Artificial Sequence

<220>
<223>  Oligonucleotide to act as a primer for PCR

<400>  13
cgtcctcata tgaggctgcc attcgctgca agg                                    33


<210>  14
<211>  33
<212>  DNA
<213>  Artificial Sequence

<220>
<223>  Oligonucleotide to act as a primer for PCR

<400>  14
cgaccgggat ccctaccagc tccttacttc cag                                    33